# EUROPEAN PATENT APPLICATION

(11) **EP 4 393 912 A1**
(43) Date of publication of application: **03.07.2024**
(21) Application number: 22875066.7
(22) Date of filing: 29.09.2022
(51) Int. Cl.: C07D 309/02, A61K 31/715, A61P 29/00

(54) **BACTERIAL CAPSULAR OLIGOSACCHARIDE DERIVATIVE, PREPARATION METHOD THEREFOR, PHARMACEUTICAL COMPOSITION AND USE THEREOF**

(30) Priority: 29.09.2021 CN 202111152832
(71) Applicant: Peking University, Beijing 100871 (CN)
(72) Inventor: LI, Zhongjun, Beijing 100191 (CN); LI, Zhongtang, Beijing 100191 (CN); SUN, Xiaoyu, Beijing 100191 (CN); WANG, Yuchao, Beijing 100191 (CN); MENG, Xiangbao, Beijing 100191 (CN); YU, Yao, Beijing 100191 (CN); SUN, Ao, Beijing 100191 (CN)
(74) Representative: HGF
(86) International application number: PCT/CN2022/122560
(87) International publication number: WO 2023/051684

(57) **Abstract**

A bacterial capsular oligosaccharide derivative, a preparation method therefor, a pharmaceutical composition and a use thereof. The derivative is as shown in formula (I), and the substituent is described in detail in the description. The derivative has anti-inflammatory activity and can be used for treating sepsis.

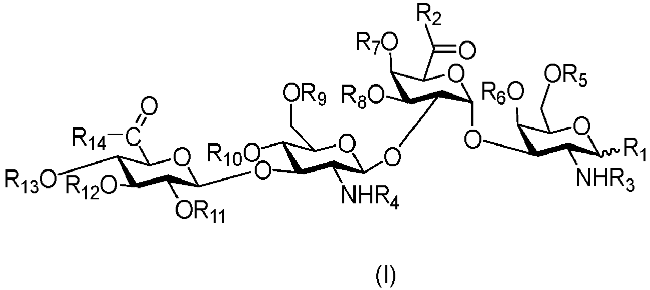

## Description

This application claims priority to Chinese invention patent application filed on September 29, 2021, titled "Bacterial Capsular Oligosaccharide Derivative, Preparation Method Therefor, Pharmaceutical Composition and Use Thereof", and with the application number 202111152832.1. The entire contents of which are incorporated herein by reference.

### Technical Field

The invention relates to biomedical technology, and in particular to a bacterial capsular oligosaccharide derivative, a preparation method thereof, a pharmaceutical composition and use thereof.

### Background

*Colwellia psychrerythraea* 34H bacterium is a psychrophilic bacterium isolated from Arctic marine sediments. Its capsular polysaccharide can simulate antifreeze protein and has good antifreeze activity, so it can adapt to low-temperature environment. The capsular polysaccharide structure of *Colwelliapsychrerythraea* 34H bacterium was reported in 2015. It is a glycosaminoglycan with amino acid modification, and its main chain consists of →4)-β-D-GlcA-(1→3)-β-D-GlcNAc-(1→2)-α-D-GalA-(1→3)-β-D-GalNAc-(1→tetrasaccharid e repeating units, and it has an L-threonine linked to the carboxyl group of galacturonic acid through an amide bond (Sara Carillo, et al., A Unique Capsular Polysaccharide Structure from the Psychrophilic Marine Bacterium Colwellia psychrerythraea 34H That Mimics Antifreeze (Glyco)proteins. J. Am. Chem. Soc. 2015, 137, 179). The disaccharide at the non-reducing end of the tetrasaccharide is hyaluronic acid disaccharide, while the disaccharide at the reducing end is a new structure that has never been reported before. However, there have been no reports of any other activity of this polysaccharide besides its antifreeze activity.

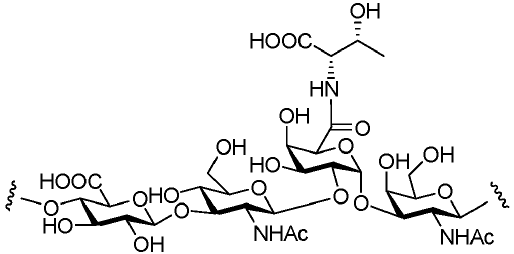

In 2019, the research group that discovered the polysaccharide structure reported the synthesis of its tetrasaccharide threonine unit. In the reported synthesis strategy thereof, the capsular polysaccharide oligosaccharide fragments were synthesized from four monosaccharide blocks by assembling oligosaccharides through protective groups and glycosylation, there are more than 40 reactions starting from monosaccharide blocks, and the total yield is less than 0.028% (Giulia Vessella et al., Synthesis of the tetrasaccharide repeating unit of the cryoprotectant capsular polysaccharide from Colwellia psychrerythraea 34H. Org. Biomol. Chem., 2019, 17, 3129). Therefore, it is of great significance to develop a new chemical synthesis method of capsular polysaccharide oligosaccharide fragments for facilitating its pharmaceutical chemistry research.

### Summary

The following is an overview of the subject matter described in detail herein. This summary is not intended to limit the protection scope of the claims.

In one aspect, the present application provides the use of a bacterial capsular oligosaccharide derivative or a pharmaceutically acceptable salt, solvate, prodrug thereof as an anti-inflammatory drug, the derivative is as shown in Formula I: 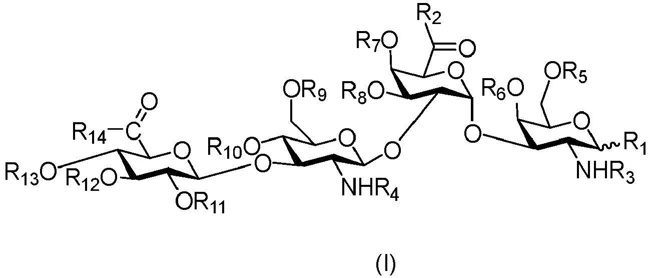
wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy;
R₂ is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue;
R₃ and R₄ are each independently unsubstituted or substituted C1-C6 alkanoyl;
R₅, R₆, R₇, R₈, R₉, R₁₀, R₁₁, R₁₂ and R₁₃ are each independently hydrogen, or unsubstituted or substituted C1-C6 alkanoyl, or unsubstituted or substituted C1-C6 alkyl;
R₁₄ is OH, unsubstituted or substituted C1-C6 alkoxy, or unsubstituted or substituted aryloxy.

In a second aspect, the present application provides a method of preventing or treating inflammation, the method comprises administering to an individual in need thereof a therapeutically effective amount of a bacterial capsular oligosaccharide derivative or a pharmaceutically acceptable salt, solvate, prodrug thereof, the derivative is as shown in Formula I: 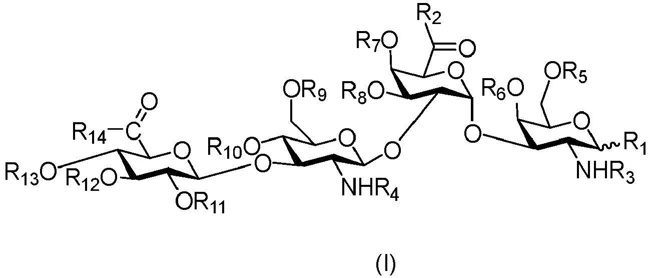
wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy;
R₂ is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue;
R₃ and R₄ are each independently unsubstituted or substituted C1-C6 alkanoyl;
R₅, R₆, R₇, R₈, R₉, R₁₀, R₁₁, R₁₂ and R₁₃ are each independently hydrogen, or unsubstituted or substituted C1-C6 alkanoyl, or unsubstituted or substituted C1-C6 alkyl;
R₁₄ is OH, unsubstituted or substituted C1-C6 alkoxy, or unsubstituted or substituted aryloxy.

In a third aspect, the present application provides a preparation method of the bacterial capsule oligosaccharide derivative as described above, the preparation method including the following steps:
reacting the compound of formula (I-13) with the compound of formula (I-18) to obtain the compound (I-19) 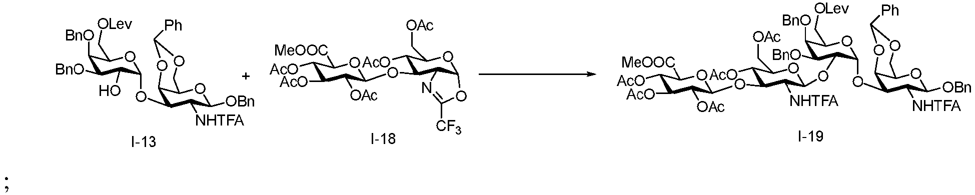
wherein in the compound of formula (I-13), formula (I-18), or formula (I-19), Ac is acetyl, Ph is phenyl, Bn is benzyl, Me is methyl, TFA is trifluoroacetyl, and Lev is acetylpropionyl.

In a fourth aspect, the present application provides a novel bacterial capsular oligosaccharide derivative represented by formula (I'), or a pharmaceutically acceptable salt, solvate, prodrug thereof: 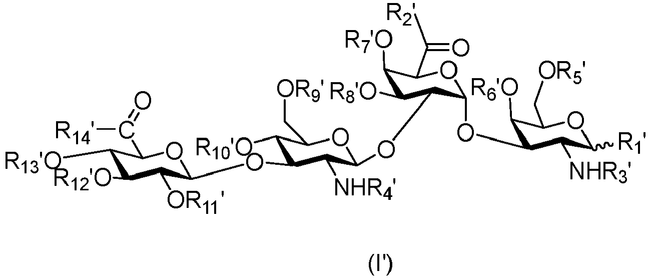
wherein R₁' in formula (I') is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy;
R₂' is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue;
R₃' and R₄' are each independently unsubstituted or substituted C1-C6 alkanoyl;
R₅', R₆', R₇', R₈', R₉', R₁₀', R₁₁', R₁₂' and R₁₃' are each independently hydrogen, unsubstituted or substituted C1-C6 alkanoyl, or unsubstituted or substituted C1-C6 alkyl;
R₁₄' is OH, unsubstituted or substituted C1-C6 alkoxy, or unsubstituted or substituted aryloxy;
and it is defined that R₁' in formula (I') is not n-propoxy or allyloxy.

In a fifth aspect, the present application provides a pharmaceutical composition comprising the above novel bacterial capsular oligosaccharide derivative.

In a sixth aspect, the present application provides the anti-inflammatory use of the novel bacterial capsular oligosaccharide derivative or pharmaceutical composition thereof.

In a seventh aspect, the present application provides the above novel bacterial capsular oligosaccharide derivative or pharmaceutical composition thereof for anti-inflammatory use.

In an eighth aspect, the present application provides a method of preventing or treating inflammation by administering the above novel bacterial capsular oligosaccharide derivative or pharmaceutical composition thereof to an individual.

### Brief Description of Drawings

The accompanying drawings are used to provide an understanding of the technical schemes of the present application, and constitute a part of the specification. They are used to explain the technical schemes of the present application together with the embodiments of the present application, and do not constitute a limitation to the technical schemes of the present application.
FIG. 1 shows the effect of Compound CP-1/CP-2 of the present invention on LPS-induced NO production in RAW264.7 cells; wherein, NC represents blank control; compared with LPS, **** p < 0.0001, n=3;
FIG. 2 shows the effect of Compound CP-1/CP-2 of the present invention on LPS-induced PGE₂ production in RAW264.7 cells; wherein, NC represents blank control; compared with LPS, **** p < 0.0001, n=3;
FIG. 3 shows the effect of Compound CP-1/CP-2 of the present invention on LPS-induced release of IL-1β, IL-6, TNF-α in RAW264.7 cells; wherein, NC represents blank control; compared with LPS, **** p < 0.0001, n=3;
FIG. 4 shows the effect of Compound CP-1/CP-2 of the present invention on LPS-induced expression of iNOS and COX-2 proteins in RAW264.7 cells; Note: NC represents blank control; compared with LPS, * p < 0.05, ** p < 0.001, n=3;

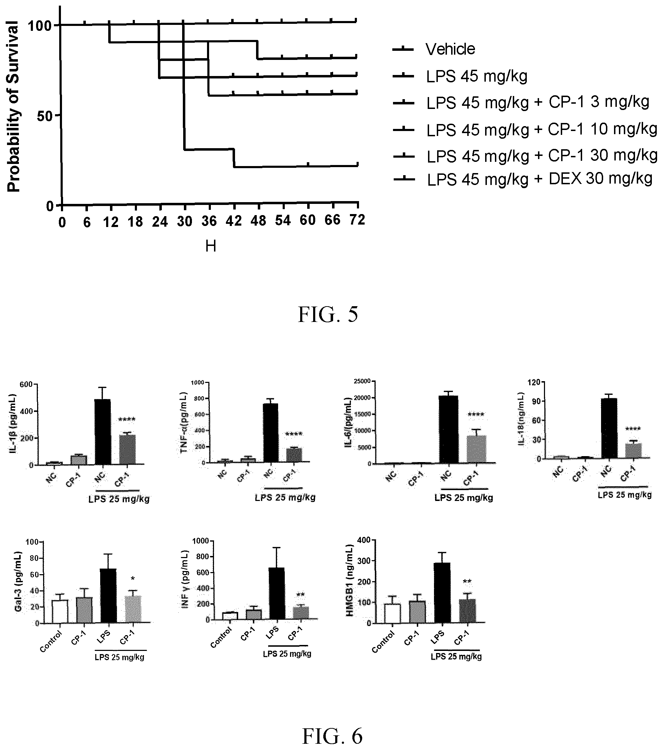

FIG. 5 shows the effect of Compound CP-1 of the present invention on LPS-induced survival rate of a mouse sepsis model;
FIG. 6 shows the inhibitory effect of Compound CP-1 of the present invention on LPS-induced inflammatory factors in serum of mouse sepsis model;

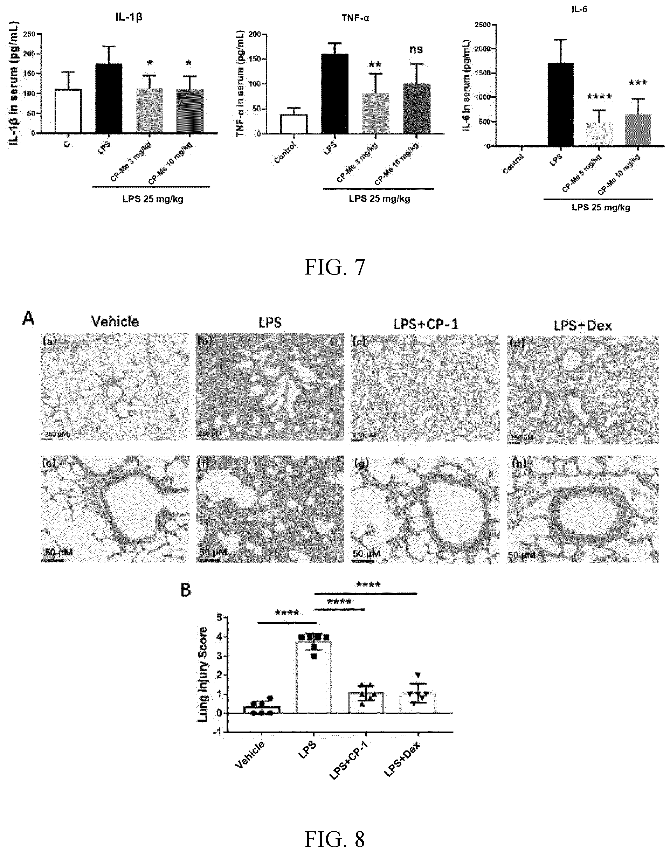

FIG. 7 shows the inhibitory effect of Compound CP-Me of the present invention on LPS-induced inflammatory factors in serum of mouse sepsis model;
FIG. 8 shows that male C57BL/6 mice are divided into control group (a/e), LPS group (bit), CP-1 group (c/g), and dexamethasone group (d/h); after 24 hours of administration of LPS via intraperitoneal injection, histopathology of lung of the mice is studied by HE staining (A); inflammation scores are scored independently by three experimenters (B, n = 6).

### Detailed Description

In an embodiment of the first or second aspect, the present application provides use of a bacterial capsular oligosaccharide derivative or pharmaceutically acceptable salt, solvate, prodrug thereof as an anti-inflammatory drug, or a method of preventing or treating inflammation, the derivative is as shown in Formula I: 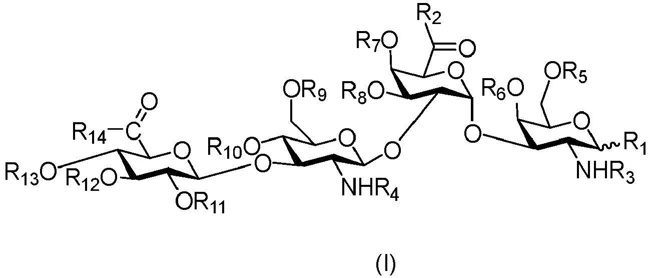
wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy; herein, the substituted C1-C6 alkoxy, substituted C2-C6 alkenyloxy, substituted C2-C6 alkynyloxy, substituted C1-C6 alkylthio, substituted C1-C6 alkynyloxy and substituted aryloxy mean that one or more hydrogen in C1-C6 alkoxy, C2-C6 alkenyloxy, C2-C6 alkynyloxy, C1-C6 alkylthio, C1-C6 alkanoyloxy or aryloxy are substituted by a group selected from hydroxy, C1-C6 alkoxy, halogen, nitro, cyano, acetyl, propionyl and phenyl;
R₂ is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue; the substituted C1-C6 alkoxy and substituted aryloxy mean that one or more hydrogen in C1-C6 alkoxy or the aryloxy is substituted by a group selected from hydroxy, C1-C6 alkoxy, halogen, nitro, cyano and phenyl;
R₃ and R₄ are each independently unsubstituted or substituted C1-C6 alkanoyl; wherein the substituted C1-C6 alkanoyl means that one or more hydrogen in the C1-C6 alkanoyl is substituted by a group selected from hydroxy, C1-C6 alkoxy, halogen, nitro, cyano, acetyl, propionyl and phenyl;
R₅, R₆, R₇, R₈, R₉, R₁₀, R₁₁, R₁₂ and R₁₃ are each independently hydrogen, unsubstituted or substituted C1-C6 alkanoyl, or unsubstituted or substituted C1-C6 alkyl; wherein the substituted C1-C6 alkanoyl or substituted C1-C6 alkyl means that one or more hydrogen in C1-C6 alkanoyl or C1-C6 alkyl is substituted by a group selected from hydroxy, C1-C6 alkoxy, halogen, nitro, cyano, acetyl, propionyl and phenyl; optionally, the phenyl may be substituted by one or more selected from C1-C4 alkoxy and nitro;
R₁₄ is OH, unsubstituted or substituted C1-C6 alkoxy, or unsubstituted or substituted aryloxy; wherein the substituted C1-C6 alkoxy and substituted aryloxy mean that one or more hydrogen in C1-C6 alkoxy or aryloxy is substituted by a group selected from hydroxy, C1-C6 alkoxy, halogen, nitro, cyano and phenyl.

In some embodiments in the first or the second aspect, R₁ in formula (I) is OH, unsubstituted C1-C6 alkoxy, phenyl-substituted C1-C6 alkoxy, or unsubstituted C2-C6 alkenyloxy.

In some embodiments in the first or the second aspect, R₁ in formula (I) is OH, methoxy, ethoxy, n-propoxy, isopropoxy, allyloxy, or benzyloxy.

In some embodiments in the first or the second aspect, R₂ in the formula (I) is OH,-N(H)-R₁₅, N(R₁₆)-R₁₇, unsubstituted C1-C6 alkoxy, or phenyl-substituted C1-C6 alkoxy, where R15 is an amino acid residue excluding proline; R₁₆ and R₁₇ together form a proline residue.

In some embodiments in the first or the second aspect, R₂ in formula (I) is OH, methoxy,-N(H)-R₁₅ or N(R₁₆)-R₁₇, where R₁₅ is glycine residue, alanine residue, valine residue, leucine residue, isoleucine residue, methionine residue, tryptophan residue, serine residue, tyrosine residue, cysteine residue, phenylalanine residue, asparagine residue, glutamine residue, threonine residue, aspartate residue, glutamate residue, lysine residue, arginine residue, or histidine residue; R₁₆ and R₁₇ together form a proline residue.

In some embodiments in the first or the second aspect, R₂ in formula (I) is OH or methoxy.

In some embodiments in the first or the second aspect, R₂ in formula (I) is -N(H)-R₁₅, where R₁₅ is glycine residue, alanine residue, valine residue, leucine residue, isoleucine residue, methionine residue, tryptophan residue, serine residue, tyrosine residue, cysteine residue, phenylalanine residue, asparagine residue, glutamine residue, threonine residue, aspartate residue, glutamate residue, lysine residue, arginine residue or histidine residue.

In some embodiments in the first or the second aspect, R₂ in formula (I) is -N(H)-R₁₅, wherein R₁₅ is a threonine residue.

In some embodiments in the first or the second aspect, the amino acids may be in L configuration or D configuration.

In some embodiments in the first or the second aspect, R₂ in formula (I) is -N(H)-R₁₅, wherein R₁₅ is an L-threonine residue.

In some embodiments in the first or the second aspect, R₃ and R₄ in the formula (I) are each independently unsubstituted or substituted C1-C6 alkanoyl; wherein the substituted C1-C6 alkanoyl means that one or more hydrogen in the C1-C6 alkanoyl is substituted by a group selected from halogen, nitro, cyano, acetyl, propionyl and phenyl.

In some embodiments in the first or the second aspect, R₃ and R₄ in formula (I) are each independently acetyl or trifluoroacetyl.

In some embodiments in the first or the second aspect, R₅, R₆, R₇, R₈, R₉, R₁₀, R₁₁, R₁₂ and R₁₃ in formula (I) are each independently hydrogen, unsubstituted C1-C6 alkanoyl, phenyl-substituted C1-C6 alkanoyl, or substituted phenylmethyl.

In some embodiments in the first or the second aspect, R₅, R₆, R₇, R₈, R₉, R₁₀, R₁₁, R₁₂ and R₁₃ in formula (I) are each independently hydrogen, acetyl, benzyl, or 4-methoxybenzyl.

In some embodiments in the first or the second aspect, R₁₄ in formula (I) is OH, or unsubstituted or substituted C1-C6 alkoxy.

In some embodiments in the first or the second aspect, R₁₄ in formula (I) is OH or methoxy.

In some embodiments in the first or the second aspect, R₁ in formula (I) is OH, methoxy, ethoxy, n-propoxy, isopropoxy, allyloxy, or benzyloxy;
R₂ is OH, methoxy or -N(H)-R₁₅, where R₁₅ is threonine residue;
R₃ and R₄ are each independently acetyl or trifluoroacetyl;
R₅, R₆, R₇, R₈, R₉, R₁₀, R₁₁, R₁₂ and R₁₃ are each independently hydrogen, acetyl, benzyl, or 4-methoxybenzyl; and
R₁₄ is OH or methoxy.

In one embodiment in the first or the second aspect, R₁ in formula (I) is n-propoxy or allyloxy, or benzyloxy;
R₂ is OH or -N(H)-R₁₅, where R₁₅ is threonine residue;
R₃ and R₄ are each independently acetyl;
R₅, R₆, R₇, R₈, R₉, R₁₀, R₁₁, R₁₂ and R₁₃ are each independently hydrogen; and
R₁₄ is OH.

In one embodiment of the first or second aspect, R₁ in formula (I) is OH;
R₂ is OH or -N(H)-R₁₅, where R₁₅ is threonine residue;
R₃ and R₄ are each independently acetyl;
R₅, R₆, R₇, R₈, R₉, R₁₀, R₁₁, R₁₂ and R₁₃ are each independently hydrogen; and
R₁₄ is OH.

In one embodiment in the first or the second aspect, R₁ in formula (I) is methoxy;
R₂ is OH or -N(H)-R₁₅, where R₁₅ is threonine residue;
R₃ and R₄ are each independently acetyl;
R₅, R₆, R₇, R₈, R₉, R₁₀, R₁₁, R₁₂ and R₁₃ are each independently hydrogen; and
R₁₄ is OH.

In one embodiment in the first or the second aspect, R₁ in formula (I) is ethoxy;
R₂ is OH or -N(H)-R₁₅, where R₁₅ is threonine residue;
R₃ and R₄ are each independently acetyl;
R₅, R₆, R₇, R₈, R₉, R₁₀, R₁₁, R₁₂ and R₁₃ are each independently hydrogen; and
R₁₄ is OH.

In one embodiment in the first or the second aspect, R₁ in formula (I) is isopropoxy;
R₂ is OH or -N(H)-R₁₅, where R₁₅ is a threonine residue;
R₃ and R₄ are each independently acetyl;
R₅, R₆, R₇, R₈, R₉, R₁₀, R₁₁, R₁₂ and R₁₃ are each independently hydrogen; and
R₁₄ is OH.

In some embodiments in the first or the second aspect, the present application provides the use of the above bacterial capsular oligosaccharide derivative as anti-inflammatory activity, or a method for the prevention or treatment of inflammation, which can inhibit the production of nitric oxide and prostaglandin E2; and/or inhibit the protein expression of nitric oxide synthase and cyclooxygenase-2; and/or reduce the release of interleukin-1β, interleukin-6 and tumor necrosis factor α.

In some embodiments in the first or the second aspect, the present application provides use of the above bacterial capsular oligosaccharide derivative or method for preventing or treating inflammation, such as treating sepsis; wherein the sepsis includes septicemia.

Sepsis is a life-threatening clinical syndrome, which is characterized by organ dysfunction caused by overreaction of the body to infection; Clinically, it is commonly found in surgery, trauma, and low immunity conditions, where bacteria enter the blood circulation, grow and reproduce, and produce toxins therein, resulting in severe systemic infection, which is manifested as fever, severe toxic blood symptoms, rash and petechia, hepatosplenomegaly and increased white blood cell count and the like.

In some embodiments of the third aspect, the present application provides a method for preparing the above bacterial capsule oligosaccharide derivative, the method including the steps of:
reacting a compound of formula (I-13) with a compound of formula (I-18) to obtain Compound (I-19); reacting a compound of formula (I-13-Me) with a compound of formula (I-18) to obtain Compound (I-19-Me); reacting a compound of formula (I-13-Et) with a compound of formula (I-18) to obtain Compound (I-19-Et); and reacting a compound of formula (I-13-Pr) with a compound of formula (I-18) to obtain Compound (I-19-Pr). 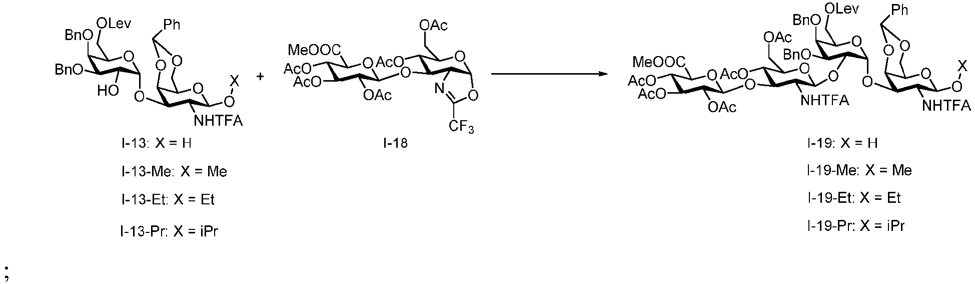 wherein in the compound of formula (I-13), formula (I-13-Me), formula (I-13-Et), formula (I-13-Pr), formula (I-18), formula (I-19), formula (I-19-Me), formula (I-19-Et) or formula (I-19-Pr), Ac is acetyl, Ph is phenyl, Bn is benzyl, Me is methyl, Et is ethyl, Pr is isopropyl, TFA is trifluoroacetyl and Lev is acetylpropionyl.

In some embodiments of the third aspect, the compounds of formula (I-13), formula (I-13-Me), formula (I-13-Et) and formula (I-13-Pr) can be prepared using the following route:

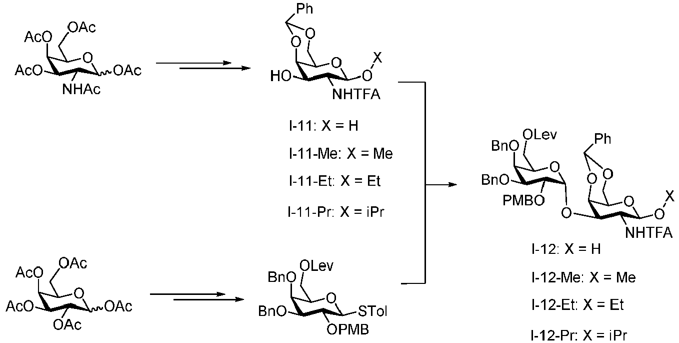

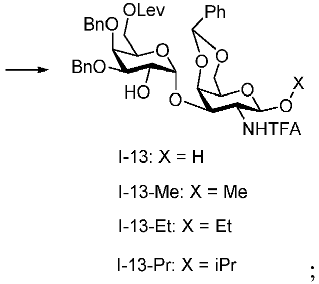

In the above route, the substituent PMB is p-methoxybenzyl and Tol is p-tolyl.

In some embodiments of the third aspect, the compound of formula (I-18) may be prepared with sodium hyaluronate as a raw material by the following route:

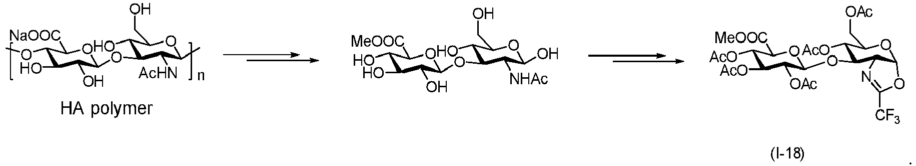

In some embodiments of the third aspect, the method for preparing the bacterial capsular oligosaccharide derivative further comprises selectively treating the Lev group after obtaining the compound of formula (I-19), formula (I-19-Me), formula (I-19-Et) or formula (I-19-Pr), followed by an amidation reaction with the protected amino acid, and lastly completely remove the protecting groups to obtain the compound of formula (I) without the protecting group. Alternatively, the method further comprises completely removing the protecting groups after obtaining the compound of formula (I-19), formula (I-19-Me), formula (I-19-Et) or formula (I-19-Pr), thereby obtaining the compound of formula (I) without the protecting group:

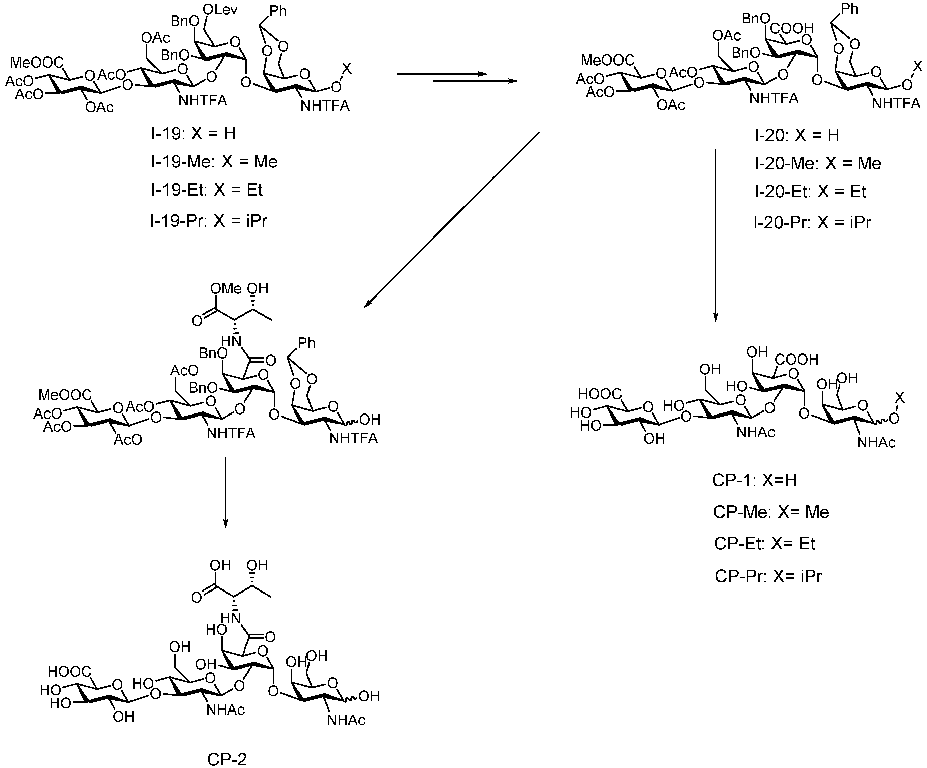

In some embodiments of the fourth aspect, the present application provides a novel bacterial capsular oligosaccharide derivative represented by formula (I'), or pharmaceutically acceptable salts, solvates, prodrugs thereof: 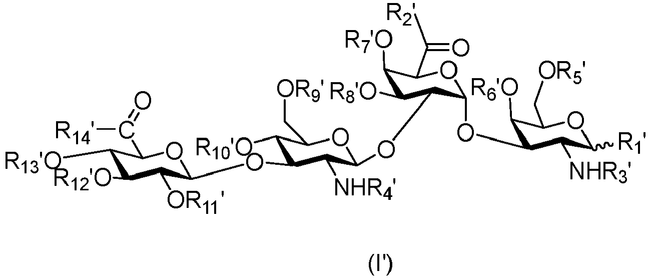
wherein R₁' in formula (I') is hydrogen, unsubstituted or substituted C1-C6 alkoxy, unsubstituted or substituted C2-C6 alkenyloxy, unsubstituted or substituted C2-C6 alkynoxy, unsubstituted or substituted C1-C6 alkylthio, unsubstituted or substituted C1-C6 alkanoyloxy, or unsubstituted or substituted aryloxy; herein, the substituted C1-C6 alkoxy, substituted C2-C6 alkenyloxy, substituted C2-C6 alkynyloxy, substituted C1-C6 alkylthio, substituted C1-C6 alkanoyloxy and substituted aryloxy mean that one or more hydrogen in C1-C6 alkoxy, C2-C6 alkenyloxy, C2-C6 alkynyloxy, C1-C6 alkylthio, C1-C6 alkanoyloxy or aryloxy are substituted by a group selected from hydroxy, C1-C6 alkoxy, halogen, nitro, cyano, acetyl, propionyl, and phenyl;
R₂' is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue; the substituted C1-C6 alkoxy and substituted aryloxy mean that one or more hydrogen in C1-C6 alkoxy or the aryloxy is substituted by a group selected from hydroxy, C1-C6 alkoxy, halogen, nitro, cyano, and phenyl;
R₃' and R₄' are each independently unsubstituted or substituted C1-C6 alkanoyl; wherein the substituted C1-C6 alkanoyl means that one or more hydrogen in the C1-C6 alkanoyl is substituted by a group selected from hydroxy, C1-C6 alkoxy, halogen, nitro, cyano, acetyl, propionyl, and phenyl;
R₅', R₆', R₇', R₈', R₉', R₁₀', R₁₁', R₁₂' and R₁₃' are each independently hydrogen, or substituted or unsubstituted C1-C6 alkanoyl; wherein the substituted C1-C6 alkanoyl means that one or more hydrogen in C1-C6 alkanoyl is substituted by a group selected from hydroxy, C1-C6 alkoxy, halogen, nitro, cyano, acetyl, propionyl, and phenyl;
R₁₄' is OH, unsubstituted or substituted C1-C6 alkoxy, or unsubstituted or substituted aryloxy; wherein the substituted C1-C6 alkoxy and substituted aryloxy mean that one or more hydrogen in C1-C6 alkoxy or aryloxy is substituted by a group selected from hydroxy, C1-C6 alkoxy, halogen, nitro, cyano, and phenyl;
and it is defined that R₁' in formula (I') is not n-propoxy or allyloxy.

In some embodiments of the fourth aspect, R₁' in formula (I') is OH, methoxy, ethoxy, isopropoxy, n-butoxy, isobutoxy, tert-butoxy, or benzyloxy.

In some embodiments of the fourth aspect, R₁' in formula (I') is OH.

In some embodiments of the fourth aspect, R₁' in formula (I') is methoxy.

In some embodiments of the fourth aspect, R₁' in formula (I') is ethoxy.

In some embodiments of the fourth aspect, R₁' in formula (I') is isopropoxy.

In some embodiments of the fourth aspect, R₂' in formula (I') is OH, -N(H)-R₁₅, N(R₁₆)-R₁₇, unsubstituted C1-C6 alkoxy, or phenyl-substituted C1-C6 alkoxy, where R₁₅ is an amino acid residue excluding proline; R₁₆ and R₁₇ together form a proline residue.

In some embodiments of the fourth aspect, R₂' in formula (I') is OH, methoxy, -N(H)-R₁₅ or N(R₁₆)-R₁₇, where R₁₅ is glycine residue, alanine residue, valine residue, leucine residue, isoleucine residue, methionine residue, tryptophan residue, serine residue, tyrosine residue, cysteine residue, phenylalanine residue, asparagine residue, glutamine residue, threonine residue, aspartate residue, glutamate residue, lysine residue, arginine residue or histidine residue; R₁₆ and R₁₇ together form a proline residue.

In some embodiments of the fourth aspect, R₂' in formula (I') is OH or methoxy.

In some embodiments of the fourth aspect, R₂' in formula (I') is -N(H)-R₁₅, where R₁₅ is glycine residue, alanine residue, valine residue, leucine residue, isoleucine residue, methionine residue, tryptophan residue, serine residue, tyrosine residue, cysteine residue, phenylalanine residue, asparagine residue, glutamine residue, threonine residue, aspartate residue, glutamate residue, lysine residue, arginine residue or histidine residue.

In some embodiments of the fourth aspect, R₂' in formula (I') is -N(H)-R₁₅, where R₁₅ is a threonine residue.

In some embodiments of the fourth aspect, the amino acids may be in L configuration or D configuration.

In some embodiments of the fourth aspect, R₂' in formula (I') is -N(H)-R₁₅, where R₁₅ is an L-threonine residue.

In some embodiments of the fourth aspect, R₃' and R₄' in formula (I') are each independently unsubstituted or substituted C1-C6 alkanoyl; wherein the substituted C1-C6 alkanoyl means that one or more hydrogen in the C1-C6 alkanoyl is substituted by a group selected from halogen, nitro, cyano, acetyl, propionyl and phenyl.

In some embodiments of the fourth aspect, R₃' and R₄' in formula (I') are each independently acetyl or trifluoroacetyl.

In some embodiments of the fourth aspect, R₅', R₆', R₇', R₈', R₉', R₁₀', R₁₁', R₁₂' and R₁₃' in formula (I') are each independently hydrogen, unsubstituted C1-C6 alkanoyl, phenyl-substituted C1-C6 alkanoyl, or substituted phenylmethyl.

In some embodiments of the fourth aspect, R₅', R₆', R₇', R₈', R₉', R₁₀', R₁₁', R₁₂' and R₁₃' in formula (I') are each independently hydrogen, acetyl, benzyl, or 4-methoxybenzyl.

In some embodiments of the fourth aspect, R₁₄' in formula (I') is OH, or unsubstituted or substituted C1-C6 alkoxy.

In some embodiments of the fourth aspect, R₁₄' in formula (I') is OH or methoxy.

In some embodiments of the fourth aspect, R₁' in formula (I') is OH, methoxy, ethoxy or isopropoxy;
R₂' is OH, methoxy or -N(H)-R₁₅, wherein R₁₅ is threonine residue;
R₃' and R₄' are each independently acetyl or trifluoroacetyl;
R₅', R₆', R₇', R₈', R₉', R₁₀', R₁₁', R₁₂' and R₁₃' are each independently hydrogen, acetyl, benzyl, or 4-methoxybenzyl; and
R₁₄' is OH or methoxy.

In an embodiment of the fourth aspect, R₁' in formula (I') is OH;
R2 is OH;
R₃' and R₄' are each independently acetyl;
R₅', R₆', R₇', R₈', R₉', R₁₀', R₁₁', R₁₂' and R₁₃' are each independently hydrogen; and
R₁₄' is OH;

That is, formula (I') is Compound CP-1:

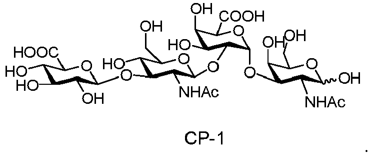

In an embodiment of the fourth aspect, R₁' in formula (I') is OH;
R2' is -N(H)-R₁₅, wherein R₁₅ is a threonine residue;
R₃' and R₄' are each independently acetyl;
R₅', R₆', R₇', R₈', R₉', R₁₀', R₁₁', R₁₂' and R₁₃' are each independently hydrogen; and
R₁₄' is OH;

That is, formula (I') is Compound CP-2:

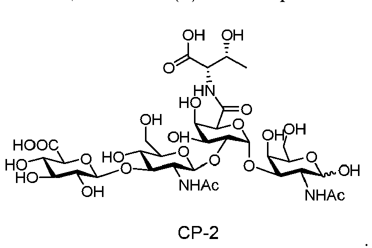

In one embodiment of the fourth aspect, R₁' in formula (I') is methoxy;
R₂' is OH;
R₃' and R₄' are each independently acetyl;
R₅', R₆', R₇', R₈', R₉', R₁₀', R₁₁', R₁₂' and R₁₃' are each independently hydrogen; and
R₁₄' is OH;

That is, formula (I') is Compound CP-Me:

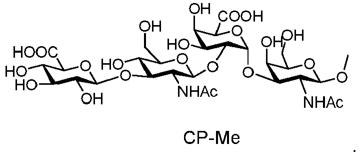

In an embodiment of the fourth aspect, R₁' in the formula (I') is ethoxy;
R₂' is OH;
R₃' and R₄' are each independently acetyl;
R₅', R₆', R₇', R₈', R₉', R₁₀', R₁₁', R₁₂' and R₁₃' are each independently hydrogen; and
R₁₄' is OH;

That is, formula (I') is Compound CP-Et:

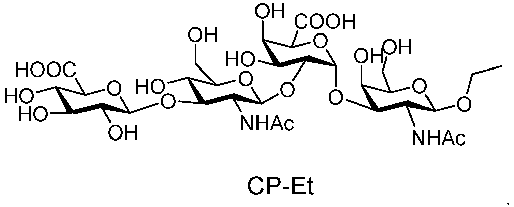

In one embodiment of the fourth aspect, R₁' in the formula (I') is isopropoxy;
R₂' is OH;
R₃' and R₄' are each independently acetyl;
R₅', R₆', R₇', R₈', R₉', R₁₀', R₁₁', R₁₂' and R₁₃' are each independently hydrogen; and
R₁₄' is OH;

That is, formula (I') is Compound CP-Pr:

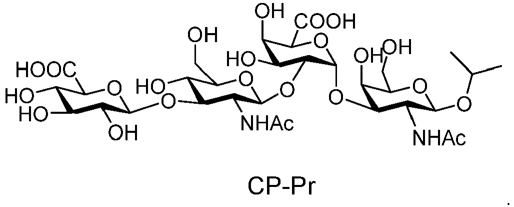

In some embodiments of the fifth aspect, the present application provides a pharmaceutical composition comprising the novel bacterial capsular oligosaccharide derivative described above. The pharmaceutical composition provided in the present application may be in the form of an oral or non-gastrointestinal administration preparation, and the dosage may be 0.1-5000 mg/time/day.

In some embodiments of the first, second, sixth, seventh or eighth aspect, the present application provides the use of the above novel bacterial capsular oligosaccharide derivative or pharmaceutical composition thereof as anti-inflammatory activities, or methods of preventing or treating inflammation, which can inhibit the production of nitric oxide and prostaglandin E2; and/or inhibit the protein expression of nitric oxide synthase and cyclooxygenase-2; and/or reduce the release of interleukin-1β, interleukin-6 and tumor necrosis factor α.

In some embodiments of the first, second, sixth, seventh or eighth aspect, the present application provides the use of the above novel bacterial capsular oligosaccharide derivative or pharmaceutical composition thereof as anti-inflammatory activities, or methods of preventing or treating inflammation, which can inhibit the production of nitric oxide and prostaglandin E2; and/or inhibit the protein expression of nitric oxide synthase and cyclooxygenase-2; and/or reduce the release of interleukin-1β, interleukin-6 and tumor necrosis factor α.

In some embodiments of the first, second, sixth, seventh or eighth aspect, the present application provides that the use of the novel bacterial capsular oligosaccharide derivative or pharmaceutical composition thereof is to treat sepsis or a method of preventing or treating inflammation is to treat sepsis; wherein the sepsis includes septicemia.

Additional features and advantages of the present application will be set forth in the description which follows, and in part will become apparent from the description, or may be learned by practice of the application. Other advantages of the present application can be realized and obtained by embodiments described in the description and the drawings.

### Specific Embodiments

In order to make the purpose, technical schemes, and advantages of the present application more clear, examples of the present application will be described in detail below. It should be noted that the following examples of the present application and the features of the examples may be arbitrarily combined with each other provided that there is no conflict.

### Abbreviations

Ac is acetyl;
PE is petroleum ether;
EtOAc is ethyl acetate;
CDCl3 is deuterated chloroform;
DCM is dichloromethane;
MeOH is methanol;
TLC is thin layer chromatography;
IL-1β is interleukin-1β;
IL-6 is interleukin-6;
TNF-α is tumor necrosis factor-α;
LPS is lipopolysaccharide;
PEG2 is prostaglandin E2;
NO is nitric oxide;
iNOS is human nitric oxide synthase;
COX-2 is cyclooxygenase 2;
Dex is dexamethasone.

### Example 1

### Synthesis of p-tolyl 2,3,4,6-tetra-O-acetyl-1-thio-β-D-galactopyranoside

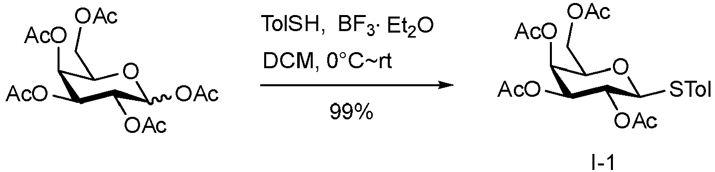

Peracetylated galactose (5.0 g, 12.8 mmol) and p-toluenethiol (1.75 g, 14.1 mmol, 1.1 equiv.) were dissolved in dichloromethane (50.0 mL), cooled to 0 °C, boron trifluoride diethyl ether (4.0 mL, 32.0 mmol, 2.5 equiv.) was added, and the reaction was carried out overnight at room temperature. The reaction solution was diluted with dichloromethane, washed with saturated sodium bicarbonate and saturated salt water in turn, dried with anhydrous sodium sulfate, filtered, concentrated under reduced pressure and purified by column chromatography (petroleum ether/EtOAc 2: 1) to obtain a white solid (5.76 g, 99%). R*_{f}* = 0.22 (petroleum ether/EtOAc 2: 1); ¹H NMR (400 MHz, CDCl₃, TMS) *δ* 7.41 (2H, d, aromatic, J = 8.1 Hz), 7.13 (2H, d, aromatic, J = 7.9 Hz), 5.41 (1H, d, H-4, J = 2.9 Hz), 5.22 (1H, t, H-2, J = 10.0 Hz), 5.04 (1H, dd, H-3, J = 3.3 Hz, J = 10.0 Hz), 4.65 (1H, d, H-1, J = 9.9 Hz), 4.19 (1H, dd, H-6a, J = 6.9 Hz, J = 11.3 Hz), 4.11 (1H, dd, H-6b, J = 6.4 Hz, J = 11.4 Hz), 3.91 (1H, t, H-5, J = 6.9 Hz), 2.35 (3H, s, CH₃ of STol), 2.12 (3H, s, COCH₃), 2.10 (3H, s, COCH₃), 2.04 (3H, s, COCH₃), 1.97 (3H, s, COCH₃); ¹³C NMR (100 MHz, CDCl₃, TMS) *δ* 170.4, 170.2, 170.0, 169.4, 138.5, 133.2, 129.6, 128.6, 87.0, 74.4, 72.0, 67.3, 67.2, 61.6, 21.2, 20.9, 20.7, 20.6, 20.5.

### Synthesis of p-Tolyl 4,6-O-benzylidene-1-thio-β-D-galactopyranoside

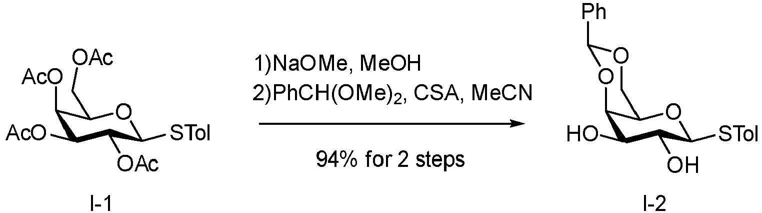

P-tolyl 2,3,4,6-tetra-O-acetyl-1-thio-β-D-galactopyranoside (5.0 g, 11.0 mmol) was dissolved in methanol (50.0 mL), and sodium methoxide was added to adjust the pH of the reaction solution to 9-10, and the reaction was carried out at room temperature for 1 hour. The reaction solution was neutralized by IR-120 cation exchange resin to pH = 7, filtered, the resin was washed with methanol, and the filtrate was concentrated. The concentrated crude product and (+)-camphor sulfonic acid (1.28 g, 5.5 mmol, 0.5 equiv.) were dissolved in anhydrous acetonitrile (70.0 mL), then benzaldehyde dimethyl acetal (2.48 mL, 16.5 mmol, 1.5 equiv.) was added, and the reaction was carried out overnight at room temperature. After the reaction was completed as monitored by TLC, the reaction was quenched by adding proper amount of triethylamine dropwise. The reaction solution was concentrated under reduced pressure, and then purified by column chromatography (DCM/MeOH 30: 1) to obtain a white solid (3.87 g, the yield of two steps was 94%). R*_{f}* = 0.40 (DCM/MeOH 20:1); ¹H NMR (400 MHz, CDCl₃, TMS) *δ* 7.55 (2H, d, aromatic, *J* = 8.1 Hz), 7.40-7.34 (5H, m, aromatic), 7.08 (2H, d, aromatic, *J* = 8.1 Hz), 5.44 (1H, s), 4.39 (1H, d, *J* = 9.1 Hz), 4.31 (1H, dd, *J* = 1.0 Hz, *J* = 12.4 Hz), 4.07 (1H, d, *J* = 1.6 Hz), 3.93 (1H, dd, *J* = 1.4 Hz, *J* = 12.4 Hz), 3.60 (2H, d, *J* = 6.6 Hz), 3.39 (1H, s), 3.09 (1H, d, *J* = 7.6 Hz), 3.07 (1H, s), 2.34 (3H, s, C*H*₃ of STol); ¹³C NMR (100 MHz, CDCl₃, TMS) *δ* 138.3, 137.8, 134.2, 129.7, 129.3, 128.2, 127.0, 126.7, 101.3, 87.0, 75.5, 73.6, 69.9, 69.3, 21.3.

### Synthesis of p-tolyl 3-O-benzyl-4,6-O-benzylidene-1-thio-β-D- galactopyranoside

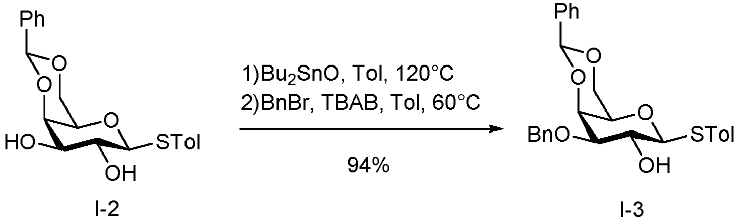

P-tolyl 4,6-O-benzylidene-1-thio-β-D-galactopyranoside (3.50 g, 9.35 mmol) and dibutyl tin oxide (2.79 g, 11.2 mmol, 1.2 equiv.) were dissolved in anhydrous toluene (50.0 mL), reacted at 120°C for 8 hours, cooled to room temperature, and concentrated under reduced pressure to remove solvent, and the resulting intermediate was dried in vacuum for 1 hour. The intermediate and tetrabutylammonium bromide (4.52 g, 14.0 mmol, 1.5 equiv.) were dissolved in anhydrous toluene (30.0 mL), benzyl bromide (1.66 mL, 14.0 mmol, 1.5 equiv.) was added, and the reaction was carried out at 60°C for 12 hours. After the reaction was completed as monitored by TLC, the reaction solution was diluted with ethyl acetate, washed with 1M hydrochloric acid and saturated salt water in turn, dried with anhydrous sodium sulfate, filtered, concentrated under reduced pressure, and purified by column chromatography (Toluene/EtOAc 9: 1) to obtain a white solid (4.08 g, 94%). R*_{f}* = 0.30 (petroleum ether/EtOAc 2: 1); ¹H NMR (400 MHz, CDCl₃, TMS) *δ* 7.57 (2H, d, aromatic, *J* = 8.1 Hz), 7.26-7.41 (10H, m, aromatic), 7.05 (2H, d, aromatic, *J* = 8.1 Hz), 5.41 (1H, s, PhC*H*), 4.71 (2H, d, PhC*H*₂, *J* = 2.0 Hz), 4.46 (1H, d, H-1, *J* = 9.4 Hz), 4.34 (1H, dd, H-6a, *J* = 1.6 Hz, *J* = 12.3 Hz), 4.12 (1H, d, H-4, *J* = 3.3 Hz), 3.96 (1H, dd, H-6b, *J* = 1.7 Hz, *J* = 12.3 Hz), 3.87 (1H, t, H-2, *J* = 9.4 Hz), 3.50 (1H, dd, H-3, *J* = 3.3 Hz, *J* = 9.3 Hz), 3.43 (1H, d, H-5, *J* = 0.9 Hz), 2.45 (1H, s, O*H*), 2.33 (3H, s, C*H*₃ of STol); ¹³C NMR (100 MHz, CDCl₃, TMS) *δ* 138.4, 138.0, 137.9, 134.4, 129.7, 129.0, 128.5, 128.1, 128.0, 126.6, 126.5, 101.2, 87.1, 80.2, 73.3, 71.7, 70.0, 69.4, 67.1, 21.2. ESI-Q-TOF (positive mode) calculated for C₂₇H₃₂NO₅S⁺ [M+NH₄]⁺ m/z 482.2001, found 482.1999.

### Synthesis of p-tolyl 2-O-p-methoxybenzyl-3-O-benzyl-4,6-O-benzylidene-1-thio-β-D-galactopyranoside

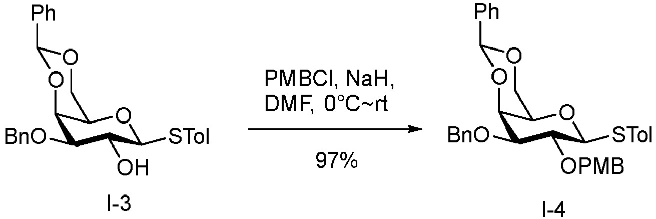

P-tolyl 3-O-benzyl-4,6-O-benzylidene-1-thio-β-D-galactopyranoside (2.60 g, 5.60 mmol) was dissolved in anhydrous N,N-dimethylformamide (50.0 mL), sodium hydride (60%, 448 mg, 11.2 mmol, 2.0 equiv.) was added in batches under ice bath conditions, and p-methoxybenzyl chloride (1.52 mL, 11.2 mmol, 2.0 equiv.) was added, and then moved to room temperature and reacted with stirring for 2 hours. After the reaction was completed as monitored by TLC, the reaction was quenched by adding proper amount of methanol dropwise. The reaction solution was diluted with ethyl acetate, the organic phase was washed with water and saturated salt water in turn, dried with anhydrous sodium sulfate, filtered, concentrated under reduced pressure, and purified by column chromatography (petroleum ether/EtOAc 3: 1) to obtain a white solid (3.18 g, 97%). R*_{f}* = 0.38 (petroleum ether/EtOAc 2: 1); ¹H NMR (400 MHz, CDCl₃, TMS) *δ* 7.61-7.59, 7.51-7.50, 7.35-7.25, 6.98-6.96, 6.86-6.84 (18H, m, aromatic), 5.43 (1H, s, PhC*H*), 4.67 (2H, s, 2PhC*H*₂), 4.63 (2H, s, 2PhC*H*₂), 4.52 (1H, d, H-1, *J* = 9.4 Hz), 4.28 (1H, d, H-6a, *J* = 12.1 Hz), 4.07 (1H, d, H-4, *J* = 2.9 Hz), 3.89 (1H, s, H-6b), 3.82 (1H, t, H-2, *J* = 9.2 Hz), 3.74 (3H, s, OC*H*₃), 3.56 (1H, dd, H-3, *J* = 2.4 Hz, *J* = 8.8 Hz), 3.26 (1H, s, H-5), 2.26 (3H, s, C*H*₃ of STol); ¹³C NMR (100 MHz, CDCl₃, TMS) *δ* 159.4, 138.4, 138.2, 137.7, 133.4, 131.0, 129.9, 129.8, 129.1, 129.0, 128.5, 128.2, 127.9, 126.8, 113.9, 101.3, 86.7, 81.6, 75.3, 75.2, 73.7, 69.8, 69.5, 55.4, 21.3. ESI-Q-TOF (positive mode) calculated for C₃₅H₄₀NO₆S⁺ [M+NH₄]⁺ m/z 602.2576, found 602.2583.

### Synthesis of p-tolyl 2-O-p-methoxybenzyl-3,4-di-O-benzyl-1-thio-β-D-galactopyranoside

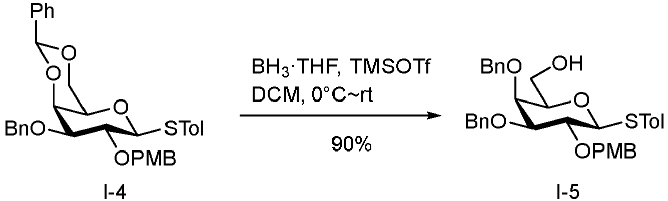

P-tolyl 2-O-p-methoxybenzyl-3-O-benzyl-4,6-O-benzylidene-1-sulfur-β-D-galactopyranoside (2.0 g, 3.42 mmol) was dissolved in anhydrous dichloromethane (30.0 mL) under the protection of argon. 1M borane tetrahydrofuran solution (17.1 mL, 17.1 mmol, 5.0 equiv.) was added under ice bath conditions, stirred for 10 minutes, and then trimethylsilyl trifluoromethylsulfonate (93 µL, 0.51 mmol, 0.15 equiv.) was added dropwise, and the temperature was recovered to room temperature and the reaction was carried out for 2 hours. After the reaction was completed as monitored by TLC, the reaction was quenched by adding proper amount of trimethylamine, the methanol was added dropwise until no hydrogen was released. Then it was concentrated under reduced pressure and purified by column chromatography (petroleum ether/EtOAc 4: 1) to obtain a white solid (1.81 g, 90%). R*_{f}* = 0.26 (petroleum ether/EtOAc 2: 1); ¹H NMR (400 MHz, CDCl₃, TMS) *δ* 7.46-7.44, 7.34-7.29, 7.01-6.99, 6.85-6.83 (18H, m, aromatic), 4.94 (1H, d, *J* = 11.7 Hz), 4.73 (3H, m), 4.66 (1H, d, *J* = 10.0 Hz), 4.61 (1H, d, *J* = 11.5 Hz), 4.56 (1H, d, *J* = 9.4 Hz), 3.89 (1H, t, *J* = 9.4 Hz), 3.83-3.78 (2H, m), 3.76 (3H, s), 3.57-3.50 (2H, m), 3.39 (1H, t, *J* = 6.0 Hz), 2.26 (3H, s); ¹³C NMR (100 MHz, CDCl₃, TMS) *δ* 159.4, 138.5, 138.3, 137.4, 132.1, 130.6, 130.2, 130.1, 129.7, 128.6, 128.4, 128.2, 127.8, 127.6, 113.8, 88.0, 84.3, 78.9, 77.2, 75.3, 74.3, 73.5, 73.0, 62.2, 55.4, 21.2; ESI-Q-TOF (positive mode) calculated for C₃₅H₄₂NO₆S⁺ [M+NH₄]⁺ m/z 604.2733, found 604.2737.

### Synthesis of p-tolyl 2-O-p-methoxybenzyl-3,4-di-O-benzyl-6-acetylpropionyl-1-thio-β-D-galactopyranoside

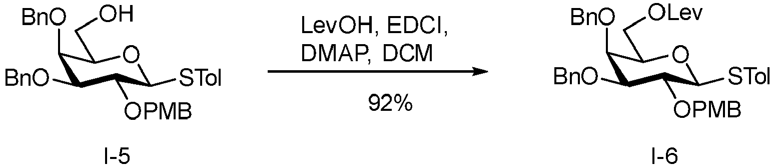

P-tolyl 2-O-p-methoxybenzyl-3,4-di-O-benzyl-1-thio-β-D-galactopyranoside (1.80 g, 3.07 mmol) was dissolved in anhydrous dichloromethane (30.0 mL), levulinic acid (713 mg, 6.14 mmol, 2.0 equiv.), 1-(3-dimethylaminopropyl)-3-ethylcarbodiimide hydrochloride (1.18 g, 6.14 mmol, 2.0 equiv.) and catalytic amount of 4-dimethylaminopyridine were added in turn, and the reaction was carried out for 3 hours at room temperature. After the reaction was completed as monitored by TLC, the reaction solution was diluted with dichloromethane, and the organic phase was washed with saturated sodium bicarbonate solution and saturated salt water in turn, dried with anhydrous sodium sulfate, filtered, concentrated under reduced pressure, and purified by column chromatography (petroleum ether/EtOAc 3: 1) to obtain a white solid (12.3 g, 92%). R*_{f}* = 0.30 (petroleum ether/EtOAc 2: 1); ¹H NMR (400 MHz, CDCl₃, TMS) *δ* 7.47-7.45, 7.38-7.29, 7.02-7.00, 6.86-6.84 (18H, m, aromatic), 4.98 (1H, d, *J* = 11.4 Hz, PhC*H*₂), 4.75 (3H, m, PhC*H*₂), 4.67 (1H, d, *J* = 9.8 Hz, PhC*H*₂), 4.63 (1H, d, *J* = 11.4 Hz, PhC*H*₂), 4.55 (1H, d, *J* = 9.6 Hz, H-1), 4.27 (1H, m, H-6a), 4.15 (1H, m, H-6b), 3.89 (2H, m, H-2, H-4), 3.78 (3H, s, OC*H*₃), 3.59-3.55 (2H, m, H-3, H-5), 2.71-2.68, 2.51-2.47 (4H, m, C*H*₂ of Lev), 2.29 (3H, s, PhC*H*₃), 2.14 (3H, s, C*H*₃CO); ¹³C NMR (100 MHz, CDCl₃, TMS) *δ* 206.5, 159.3, 138.5, 138.3, 137.3, 132.2, 130.6, 130.2, 130.0, 129.6, 128.5, 128.3, 128.1, 127.8, 127.6, 113.8, 88.1, 84.2, 77.0, 75.9, 75.3, 74.3, 73.4, 73.0, 63.4, 55.3, 37.9, 29.8, 27.9, 21.1; HRMS (ESI-MS) calculated for C₄₀H₄₄NaO₈S⁺ [M+Na]⁺ m/z 707.2649, found 707.2635.

### Synthesis of 2-deoxy-2-trifluoroacetamido-1,3,4,6-tetra-O-acetyl-β-D-galactopyranoside

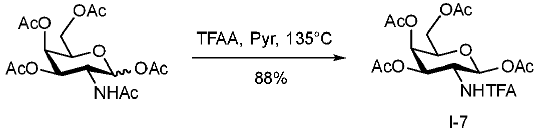

peracetylated galactosamine (5.0 g, 12.8 mmol) was dissolved in anhydrous pyridine (100.0 mL), trifluoroacetic anhydride (9.02 mL, 64.0 mmol, 5.0 equiv.) was added, and the temperature was raised to 135°C, and the reaction was carried out for 1 hour. After the reaction was completed as monitored by TLC, the reaction was quenched by adding proper amount of methanol. The reaction solution was diluted with dichloromethane, the organic phase was washed with 1M hydrochloric acid solution and saturated salt water in turn, dried with anhydrous sodium sulfate, filtered, concentrated under reduced pressure, and purified by column chromatography (DCM/MeOH 60: 1) to obtain a white solid (5.0 g, 88%). R*_{f}* = 0.34 (DCM/MeOH 30: 1); ¹H NMR (400 MHz, CDCl₃, TMS) *δ* 7.19 (1H, d, *J* = 9.4 Hz, N*H*Ac), 5.80 (1H, d, H-1, *J* = 8.9 Hz), 5.42 (1H, d, H-4, *J* = 3.0 Hz), 5.20 (1H, d, H-3, *J* = 3.2 Hz, *J* = 11.3 Hz), 4.50 (1H, m, H-2), 4.21-4.07 (3H, m, H-5, H-6a, H-6b), 2.20 (3H, s, C*H*₃CO), 2.14 (3H, s, C*H*₃CO), 2.06 (3H, s, C*H*₃CO), 2.03 (3H, s, C*H*₃CO); ¹³C NMR (100 MHz, CDCl₃, TMS) *δ* 170.8, 170.7, 170.2, 169.6, 157.7 (q, *J* = 37.4 Hz, *C*OCF₃), 117.0 (q, *J* = 285.1 Hz, CO*C*F₃), 92.3, 72.0, 69.9, 66.2, 61.4, 50.2, 20.6, 20.5, 20.4. ESI-Q-TOF (positive mode) calculated for C₁₆H₂₄F₃N₂O₁₀⁺ [M+NH₄]⁺ m/z 461.1383, found 461.1381.

### Synthesis of p-tolyl 2-deoxy-2-trifluoroacetamido-3,4,6-tri-O-acetyl-1-thio-β-D-galactopyranoside

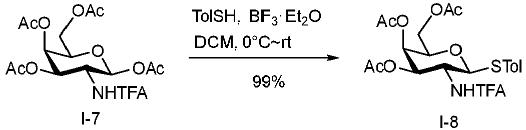

2-deoxy-2-trifluoroacetylamino-1,3,4,6-tetra-O-acetyl-β-D-galactopyranoside (5.0 g, 11.3 mmol) and p-toluenethiol (2.1 g, 17.0 mmol, 1.5 equiv.) were dissolved in anhydrous dichloromethane (100.0 mL), boron trifluoride diethyl ether complex (4.28 mL, 33.9 mmol, 3.0 equiv.) was added dropwise under ice bath, and temperature was recovered to room temperature and the reaction was carried out overnight. After the reaction was completed as monitored by TLC, the reaction was quenched by adding proper amount of trimethylamine. The reaction solution was diluted with dichloromethane, and the organic phase was washed with saturated sodium bicarbonate solution and saturated salt water in turn, dried with anhydrous sodium sulfate, filtered, concentrated under reduced pressure, to obtain a yellow syrup (5.68 g, 99%) with R_{f} = 0.24 (petroleum ether/EtOAc 2: 1), which was directly used for the next reaction.

### Synthesis of benzyl 2-deoxy-2-trifluoroacetamido-3,4,6-tri-O-acetyl-β-D-galactopyranoside

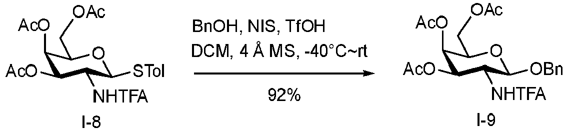

P-tolyl 2-deoxy-2-trifluoroacetamido-3,4,6-tri-O-acetyl-1-thio-β-D-galactopyranoside (5.68 g, 11.2 mmol) and 4Å molecular sieve were dissolved in anhydrous dichloromethane (100.0 mL) under the protection of argon, and benzyl alcohol was added (2.33 mL, 22.4 mmol, 2.0 equiv.), stirred at room temperature for 2 hours, then cooled to -40°C. N-iodosuccinimide (3.53 g, 15.7 mmol, 1.4 equiv.) was added, stirred for 15 minutes, and then trifluoromethyl sulfonic acid was added dropwise (300 µL, 3.36 mmol, 0.3 equiv.) and the reaction was carried out with continuously stirring for 3 hours. After the reaction was completed as monitored by TLC, the reaction was quenched by adding proper amount of trimethylamine. The molecular sieve was filtered out by diatomite, filter cake was washed several times with dichloromethane. Filtrates were combined, concentrated under reduced pressure, and purified by column chromatography (petroleum ether/EtOAc 2: 1) to obtain a white solid (5.06 g, 92%). R*_{f}* = 0.19 (petroleum ether/EtOAc 2: 1); ¹H NMR (400 MHz, CDCl₃, TMS) *δ* 7.35-7.26 (5H, m, aromatic), 7.01 (1H, br s, NHAc), 5.36 (1H, d, *J* = 3.0 Hz), 5.20 (1H, dd, *J* = 11.5 Hz, *J* = 3.3 Hz), 4.89 (1H, d, *J* = 12.1 Hz), 4.63 (1H, d, *J* = 8.4 Hz), 4.61 (1H, d, *J* = 12.3 Hz), 4.29-4.13 (3H, m), 3.93 (1H, t, *J* = 6.8 Hz), 2.15 (3H, s), 2.06 (3H, s), 1.92 (3H, s); ¹³C NMR (100 MHz, CDCl₃, TMS) *δ* 170.7, 170.6, 170.4, 157.5 (q, *J=* 37.1 Hz, *C*OCF₃), 136.4, 128.5, 128.2, 127.9, 115.6 (q, *J* = 286.2 Hz, CO*C*F₃), 99.0, 70.8, 70.7, 69.7, 66.6, 61.7, 51.5, 20.6, 20.5, 20.4; ESI-Q-TOF (positive mode) calculated for C₂₁H₂₈F₃N₂O₉⁺ [M+NH₄]⁺ m/z 509.1747, found 509.1747.

### Synthesis of benzyl 2-deoxy-2-trifluoroacetamido-β-D-galactopyranoside

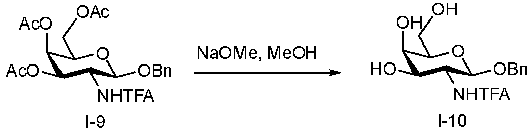

Benzyl 2-deoxy-2-trifluoroacetamido-3,4,6-tri-O-acetyl-β-D-galactopyranoside (2.50 g, 5.09 mmol) was dissolved in methanol (50.0 mL), pH was adjusted to 9-10 by adding proper amount of sodium methoxide, and the reaction was carried out with stirring at room temperature for 2 hours. After the reaction was completed as monitored by TLC, cationic resin was added to the reaction solution and neutralized it to pH = 7, filtered, and filtrate was concentrated under reduced pressure to dryness to obtain a yellow syrup. R*_{f}* = 0.45 (DCM/MeOH 30: 1), which was directly used for the next reaction.

### Synthesis of benzyl 2-deoxy-2-trifluoroacetamido-4,6-O-benzylidene-β-D-galactopyranoside

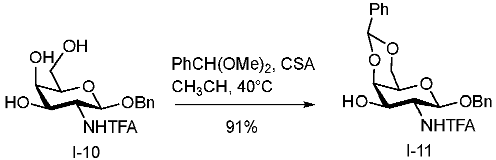

Benzyl 2-deoxy-2-trifluoroacetamido-β-D-galactopyranoside (1.80 g, 4.93 mmol) and (+)-camphor sulfonic acid (572 mg, 2.46 mmol, 0.5 equiv.) were dissolved in anhydrous acetonitrile (50.0 mL) under the protection of argon. Benzaldehyde dimethyl acetal (1.11 mL, 7.40 mmol, 1.5 equiv.) was added, and the temperature was raised to 40°C and the reaction was carried out overnight. After the reaction was completed as monitored by TLC, the reaction was quenched by adding proper amount of triethylamine dropwise. The reaction solution was concentrated under reduced pressure, and purified by column chromatography (petroleum ether/EtOAc 2: 1) to obtain the compound as a white solid (2.03 g, 91%). R*_{f}* = 0.30 (petroleum ether/EtOAc 1: 1); ¹H NMR (400 MHz, DMSO-*d*₆) *δ* 9.26 (1H, d, N*H, J* = 9.0 Hz), 7.52-7.49, 7.42-7.26 (10H, m, aromatic), 5.63 (1H, s, PhC*H*), 5.25 (1H, d, O*H, J* = 6.4 Hz), 4.82 (1H, d, PhC*H*₂, *J* = 12.5 Hz), 4.58 (1H, d, H-1, *J* = 8.3 Hz), 4.55 (1H, d, PhC*H*₂, *J* = 12.3 Hz), 4.17-4.09 (3H, m, H-4, H-6a, H-6b), 3.95 (1H, m, H-2), 3.82 (1H, m, H-3), 3.57 (1H, s, H-5); ¹³C NMR (100 MHz, DMSO-*d*₆) *δ* 157.0 (q, *J* = 35.6 Hz, *C*OCF₃), 138.9, 138.3, 129.2, 128.6, 128.4, 128.0, 127.6, 126.8, 116.5 (q, *J* = 287.0 Hz, CO*C*F₃), 100.6, 100.3, 75.5, 70.4, 69.0, 66.7, 53.2; HRMS (ESI-MS) calculated for C₂₂H₂₂NNaO₆F₃⁺ [M+Na]⁺ m/z 476.1291, found 476.1292.

### Benzyl 2-O-p-methoxybenzyl-3,4-di-O-benzyl-6-acetylpropionyl-α-D-galactopyranosyl-(1→3)-2-deoxy-2-trifluoroacetamido-4,6-O-benzylidene-β-D-galactopyranoside

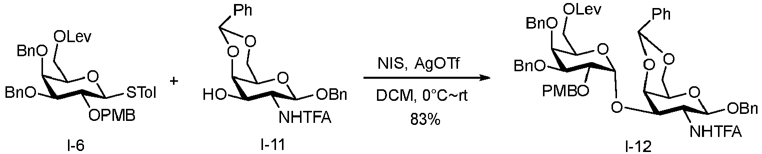

P-tolyl 2-O-p-methoxybenzyl-3,4-di-O-benzyl-6-acetylpropionyl-1-thio-β-D-galactopyranoside (680 mg, 0.99 mmol, 1.5 equiv.) and benzyl 2-deoxy-2-trifluoroacetamido-4,6-O-benzylidene-β-D-galactopyranoside (300 mg, 0.66 mmol) were dissolved in anhydrous dichloromethane/N,N-dimethylformamide (10.0 mL/2. 0 ml) under the protection of argon, and 4 Å molecular sieve (1.0 g) was added, stirred at room temperature for 2 hours, and then cooled to 0°C. N-iodosuccinimide (297 mg, 1.32 mmol, 2.0 equiv.) and silver trifluoromethanesulfonate (51 mg, 0.20 mmol, 0.3 equiv.) were added in turn, reacted with stirring at 0°C for 2 hours, then the temperature was slowly raised to room temperature and the reaction was carried out overnight. After the reaction was completed as monitored by TLC, the reaction was quenched by adding proper amount of triethylamine dropwise. The molecular sieve was removed by diatomite filtration, the filtrate was concentrated under reduced pressure, and purified by column chromatography (DCM/MeOH 100: 1) to obtain compound I-12 (555 mg, 83%) as a white solid. R*_{f}* = 0.28 (DCM/MeOH 30:1); ¹H NMR (600 MHz, CDCl₃, TMS) *δ* 7.55, 7.44, 7.34-7.25, 6.92, 6.63 (25H, m, aromatic, NH), 5.56 (1H, s, PhC*H*), 5.23 (1H, d, H-1^{Gal}, *J* = 3.5 Hz), 4.94 (2H, m, PhC*H*₂), 4.87 (1H, d, H-1^{GalNAc}, *J* = 8.3 Hz), 4.80 (1H, d, PhC*H*₂, *J* = 11.8 Hz), 4.63 (2H, m, PhC*H*₂), 4.54 (1H, d, PhC*H*₂, *J* = 11.5 Hz), 4.45-4.38 (5H, m, H-2^{GalNAc}, H-4^{GalNAc}, H-6a^{GalNAc}, PhC*H*₂), 4.17 (1H, dd, H-3^{GalNA}c, *J* = 3.5 Hz, *J* = 10.9 Hz), 4.16-4.12 (2H, m, H-6a^{Gal}, H-6b^{GalNAc}), 4.07 (1H, dd, H-6b^{Gal}, *J* = 7.8 Hz, *J* = 11.7 Hz), 4.03 (1H, dd, H-2^{Gal}, *J* = 3.5 Hz, *J* = 9.9 Hz), 3.84 (1H, dd, H-3^{Gal}, *J* = 2.7 Hz, *J* = 9.9 Hz), 3.80 (1H, m, H-4^{Gal}), 3.75 (3H, s, OC*H*₃), 3.73 (1H, m, H-5^{Gal}), 3.51 (1H, s, H-5^{GalNAc}), 2.83-2.78, 2.63-2.58, 2.52-2.49, 2.40-2.34 (4H, m, C*H*₂ of Lev), 2.04 (3H, s, C*H*₃CO); ¹³C NMR (150 MHz, CDCl₃, TMS) *δ* 208.9, 172.4, 158.8, 157.1 (q, *J* = 24.2 Hz, *C*OCF₃), 138.6, 138.2, 137.4, 137.3, 130.5, 129.3, 129.1, 128.3, 128.2(2C), 127.9, 127.7(2C), 127.6, 127.5, 126.4, 115.9 (q, *J* = 191.2 Hz, CO*C*F₃), 113.4, 101.2, 98.8, 92.7, 78.1, 75.4, 74.9, 74.5, 73.7, 71.4, 71.3, 70.6, 70.1, 69.4, 66.5, 64.5, 55.2, 52.4, 37.9, 29.7, 27.8; HRMS (ESI-MS) calculated for C₅₅H₅₈NNaO₁₄F₃⁺ [M+Na]⁺ m/z 1036.3702, found 1036.3699.

### Benzyl 3,4-di-O-benzyl-6-acetylpropionyl-α-D-galactopyranosyl-(1→3)-2-deoxy-2-trifluoroacetamido-4,6-O-benzylidene-β-D-galactopyranoside

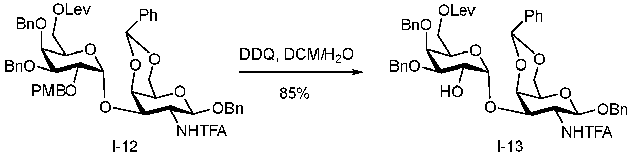

Benzyl 2-O-p-methoxybenzyl-3,4-di-O-benzyl-6-acetylpropionyl-β-D-galactopyranosyl-(1→3)-2-deox y-2-trifluoroacetamido-4,6-O-benzylidene-β-D-galactopyranoside (340 mg, 0.34 mmol) was dissolved in dichloromethane/water (6.0 mL/0. 6 ml), and 2,3-dichloro-5,6-dicyano-p-benzoquinone (154 mg, 0.68 mmol, 2.0 equiv.) was added in batches, and reaction was carried out for 1 hour at room temperature. After the reaction was completed as monitored by TLC, the reaction solution was diluted with dichloromethane, and the organic phase was washed with saturated sodium bicarbonate solution, saturated sodium thiosulfate solution and saturated salt water in turn, dried with anhydrous sodium sulfate, filtered, concentrated under reduced pressure, and purified by column chromatography (DCM/MeOH 60: 1) to obtain a white solid (258 mg, 85%). R*_{f}* = 0.36 (DCM/MeOH 30:1); ¹H NMR (600 MHz, CDCl₃+CD₃OD, TMS) *δ* 7.59, 7.40-7.26 (20H, m, aromatic), 5.64 (1H, s, PhC*H*), 5.14 (1H, d, H-1^{Gal}, *J* = 3.5 Hz), 4.93 (2H, m, PhC*H*₂), 4.83 (1H, d, PhC*H*₂, *J* = 11.8 Hz), 4.76 (1H, d, H-1^{GalNAc}, *J* = 8.6 Hz), 4.62 (2H, m, PhC*H*₂), 4.50 (1H, d, PhC*H*₂, *J* = 11.4 Hz), 4.42 (2H, m, H-4^{GalNAc}, H-6a^{GalNAc}), 4.35 (1H, t, H-2^{GalNAc}, *J* = 10.0 Hz), 4.15 (1H, d, H-3^{GalNAc}, *J* = 12.2 Hz), 4.09 (3H, m, H-6a^{Gal}, H-6b^{GalNAc}), 4.03 (1H, dd, H-6b^{Gal}, *J* = 2.9 Hz, *J* = 10.8 Hz), 3.73 (2H, m, H-3^{Gal},), 3.60 (1H, dd, H-4^{Gal}, *J* = 2.0 Hz, *J* = 10.0 Hz), 3.52 (1H, s, ), 2.82-2.76, 2.69-2.64, 2.52-2.47, 2.43-2.39 (4H, m, C*H*₂ of Lev), 2.07 (3H, s, C*H*₃CO); ¹³C NMR (150 MHz, CDCl₃+CD₃OD, TMS) *δ* 209.5, 172.6, 158.2 (q, *J* = 24.5 Hz, *C*OCF₃), 138.5, 138.2, 137.3, 137.2, 129.2, 128.5, 128.4, 128.3, 127.9, 128.4, 128.3, 127.9(2C), 127.7, 126.4, 116.0 (q, *J* = 190.9 Hz, CO*C*F₃), 101.1, 99.0, 95.2, 79.2, 75.1, 74.8, 73.8, 73.0, 70.6, 70.4, 70.1, 69.3, 68.9, 66.6, 63.9, 51.5, 38.0, 29.8, 27.7; HRMS (ESI-MS) calculated for C₄₇H₅₀NNaO₁₃F₃⁺ [M+Na]⁺ m/z 916.3126, found 916.3132.

### Synthesis of methyl 2,3,4-tri-O-acetyl-β-D-glucopyranuronate-(1→3)-1,4,6-tri-O-acetyl-2-deoxy-2-acetylamino-α,β-D-glucopyranoside

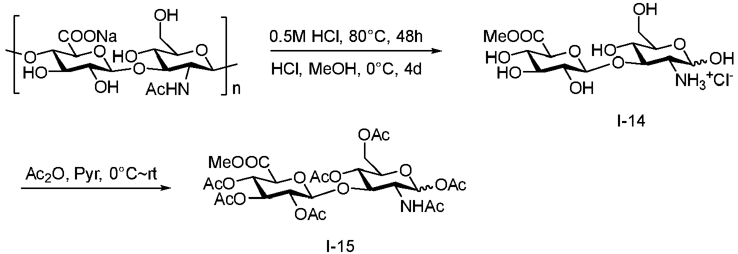

5.0 g hyaluronic acid (Mw=500 kDa) was dissolved in 300.0 mL deionized water, swelled overnight, then concentrated hydrochloric acid (12 M, 13.0 mL, final concentration 0.5 M) was slowly added dropwise, and the reaction was heated at 80°C for two days. After the reaction was completed as monitored by TLC, sodium bicarbonate solid was added to adjust pH to 7.0, the reaction liquid was concentrated to about 20.0 mL under reduced pressure, and slowly dropped into 1000.0 mL ethanol to obtain light brown flocculent precipitate, which was filtered under reduced pressure, and the precipitate was dried in infrared oven to obtain a yellow powdery solid. The crude product was dissolved in methanol solution (0.02 M, 200.0 mL) in hydrochloric acid and reacted at 4°C for 4 days. After the reaction was completed as monitored by TLC, pH was adjusted to 7.0 by adding triethylamine, the solution was concentrated under reduced pressure, with toluene added for 3 times to obtain a brown powdered solid. The brown powdered solid was dissolved in pyridine (100.0 mL), acetic anhydride (50.0 mL) was slowly added dropwise under ice bath conditions, the temperature was naturally raised to room temperature, and reaction was carried out overnight. After the reaction was completed as monitored by TLC, the reaction was quenched by adding proper amount of methanol dropwise udder ice bath conditions. The reaction solution was concentrated under reduced pressure, diluted with dichloromethane, washed with 1 M hydrochloric acid solution, stripped with dichloromethane for three times. The organic phases were combined, and then washed with saturated sodium bicarbonate solution and saturated salt water, dried with anhydrous sodium sulfate, filtered and concentrated under reduced pressure. It was purified by column chromatography (DCM/MeOH 60: 1, 0.1% triethylamine) to obtain a pale yellow solid (2.60 g) (α/β = 2.5/1). R*_{f}* = 0.40 (DCM/MeOH 20: 1). The NMR spectra of the mixture are shown in the appendix, and the proportion of end isomers was confirmed by ¹H NMR. ESI-Q-TOF (positive mode) calculated for C₂₇H₄₁N₂O₁₈⁺ [M+NH₄]⁺ m/z 681.2354, found 681.2360.

### Synthesis of methyl 2,3,4-tri-O-acetyl-β-D-glucopyranuronate-(1→3)-1,4,6-tri-O-acetyl-2-deoxy-2-trifluoroacetamido-α-D-glucopyranoside

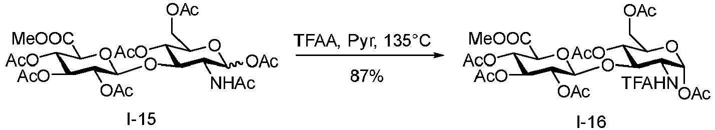

Methyl 2,3,4-tri-O-acetyl-β-D-glucopyranuronate-(1→3)-1,4,6-tri-O-acetyl-2-deoxy-2-acetylamino-α,β-D-glucopyranoside (500 mg, 0.75 mmol) was dissolved in anhydrous pyridine (3.0 mL), trifluoroacetic anhydride (425 µL, 3.01 mmol, 4.0 equiv) was added, and the reaction was carried out under reflux at 135°C for 30 minutes. After the reaction was completed as monitored by TLC, the reaction was quenched by adding proper amount of methanol under ice bath conditions, the reaction solution was concentrated under reduced pressure, and then diluted with dichloromethane, washed with 1 M hydrochloric acid solution, stripped with dichloromethane for three times. The organic phases were combined, and then washed with saturated salt water, dried with anhydrous sodium sulfate, filtered and concentrated under reduced pressure. It was purified by column chromatography (PE/acetone 4: 1) to obtain a brown solid (468 mg, 87%). R*_{f}* = 0.36 (PE/acetone 1: 1). ¹H NMR (400 MHz, CDCl₃) *δ* 6.95 (1H, s, N*H*), 6.08 (1H, d, H-1^{GlcNAc}, J = 3.3 Hz), 5.16-5.03 (3H, m, H-3^{GlcA}, H-5^{GlcA}, H-4^{GlcNAc}), 4.84 (1H, t, H-2^{GlcA}, *J* = 8.0 Hz), 4.66 (1H, d, H-1^{GlcA}, *J* = 7.8 Hz), 4.43 (1H, m, H-2^{GlcNAc}), 4.18 (1H, dd, H-6a^{GlcNAc}, *J* = 3.7 Hz, *J* = 12.5 Hz), 4.11-3.98 (4H, m, H-6b^{GlcNAc}, H-3^{GlcNAc}, H-5^{GlcNAc}, H-4^{GlcA}), 3.70 (3H, s, OC*H*₃), 2.15 (3H, s, C*H*₃CO), 2.09 (3H, s, C*H*₃CO), 2.05 (3H, s, C*H*₃CO), 1.97 (6H, s, 2C*H*₃CO), 1.94 (3H, s, C*H*₃CO); ¹³C NMR (100 MHz, CDCl₃) *δ* 171.0, 170.1, 169.7, 169.6, 169.3, 168.6, 166.9, 157.2 (q, *J* = 39.6 Hz, *C*OCF₃), 115.7 (q, *J* = 286.3 Hz, CO*C*F₃), 100.1, 90.3, 75.5, 72.4, 72.0, 71.0, 70.0, 69.4, 67.5, 61.7, 52.8, 52.2, 20.8, 20.7, 20.5(2C), 20.3, 20.2; ESI-Q-TOF (positive mode) calculated for C₂₇H₃₈F₃N₂O₁₈⁺ [M+NH₄]⁺ m/z 735.2072, found 735.2081.

### Synthesis of methyl 2,3,4-tri-O-acetyl-β-D-glucopyranuronate-(1→3)-4,6-di-O-acetyl -2-deoxy-2-trifluoroacetylamino-α,β-D-glucopyranoside

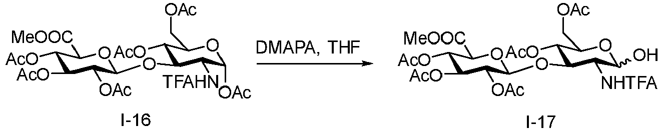

Methyl 2,3,4-tri-O-acetyl-β-D-glucopyranuronate-(1→3)-1,4,6-tri-O-acetyl-2-deoxy -2-trifluoroacetylamino-α-D-glucopyranoside (400 mg, 0.56 mmol) was dissolved in tetrahydrofuran (6.0 mL), and 3-dimethylaminopropylamine (348 µL, 2.79 mmol, 5.0 equiv.) was added dropwise, and the reaction was carried out for 1 h at room temperature. After the reaction was completed as monitored by TLC, the reaction solution was diluted with dichloromethane, washed with 1 M hydrochloric acid solution, stripped with dichloromethane for three times. The organic phases were combined, and then washed with saturated salt water, dried with anhydrous sodium sulfate, filtered and concentrated under reduced pressure. The crude product obtained was directly used for the next reaction without further purification.

### Synthesis of methyl 2,3,4-tri-O-acetyl-β-D-glucopyranuronate-(1→3)-4,6-di-O-acetyl-2-deoxy-2-trifluoroacetylamino-D-glucopyranose [2, 1,-d] 2-oxazoline

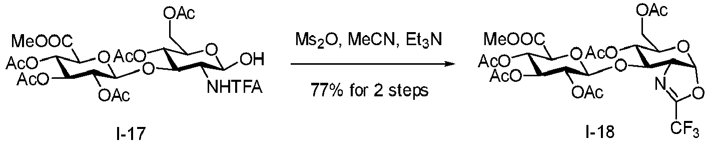

Methyl 2,3,4-tri-O-acetyl-β-D-glucopyranuronate-(1→3)-4,6-di-O-acetyl-2-deoxy-2-trifluoroacetamido -α,β-D-glucopyranoside was dissolved in anhydrous acetonitrile (10.0 mL) under the protection of argon. Mesosulfonic anhydride (293 mg, 1.68 mmol) was added, the reaction was carried out for 25 minutes at room temperature. Then triethylamine (1.55 mL, 11.2 mmol) was added, and the reaction was carried out for 2 hours at room temperature. After the reaction was completed as monitored by TLC, the reaction solution was diluted with dichloromethane, washed with saturated sodium bicarbonate solution, stripped with dichloromethane for three times. The organic phases were combined, and then washed with saturated salt water, dried with anhydrous sodium sulfate, filtered and concentrated under reduced pressure, and purified by column chromatography (PE/acetone 3: 1) to obtain a white solid (284 mg, yield of two-steps was 77%). R*_{f}* = 0.49 (PE/acetone 1: 1). ¹H NMR (400 MHz, CDCl₃) *δ* 6.26 (1H, d, H-1^{GlcNAc}, *J* = 7.6 Hz), 5.27-5.16 (3H, m, H-3^{GlcA}, H-4^{GlcA}, H-4^{GlcNAc}), 4.97 (1H, t, H-2^{GlcA}, *J* = 7.9 Hz), 4.87 (1H, d, H-1^{GlcA}, *J* = 7.9 Hz), 4.26 (1H, d, H-2^{GlcNAc}, *J* = 7.3 Hz), 4.18 (3H, m, H-3^{GlcNAc}, H-6a^{GlcNAc}, H-6b^{GlcNAc}), 4.11 (1H, d, H-5^{GlcA}, *J* = 9.6 Hz), 3.72 (3H, s, OC*H*₃), 3.65 (1H, m, H-5^{GlcNAc}), 2.07 (3H, s, C*H*₃CO), 2.04 (3H, s, C*H*₃CO), 2.02 (3H, s, C*H*₃CO), 2.00 (3H, s, C*H*₃CO), 1.99 (3H, s, C*H*₃CO); ¹³C NMR (100 MHz, CDCl₃) *δ* 170.7, 170.1, 169.8, 169.4, 169.2, 166.9, 156.3 (q, *J* = 40.9 Hz, *C*OCF₃), 116.0 (q, *J* = 273.1 Hz, CO*C*F₃), 102.9, 100.9, 76.7, 72.4, 72.1, 71.2, 69.1, 68.8, 67.1, 64.8, 63.4, 53.0, 20.8, 20.7(2C), 20.6(2C); HRMS (ESI-MS) calculated for C₂₅H₃₀NNaO₁₆F₃⁺ [M+Na]⁺ m/z 680.1409, found 680.1391.

### Synthesis of benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-6-acety lpropionyl-α-D-galactopyranosyl-(1→3)-2-deoxy-2-trifluoroacetylamino-4,6-O-benzyliden e-β-D-galactopyranoside

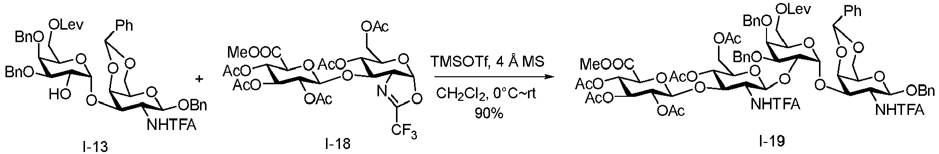

Methyl 2,3,4-tri-O-acetyl-β-D-glucopyranoate-(1→3)-4,6-di-O-acetyl-2-deoxy-2-trifluoro acetamido-D-glucopyranose [2, 1,-d] 2-oxazoline (140 mg, 0.21 mmol, 1.5 equiv.) and benzyl 3,4-di-O-benzyl-6-acetylpropionyl-α-D-galactopyranosyl-(1→3)-2-deoxy-2-trifluoroacetylamin o-4,6-O-benzylidene-β-D-galactopyranoside (127 mg, 0.14 mmol) were dissolved in anhydrous dichloromethane (2.0 mL) under the protection of argon, 4Å molecular sieve (200 mg) was added, and reacted with stirring at room temperature for 2 hours, then cooled to -20°C. Trimethylsilyl trifluoromethylsulfonate (7.6 µL, 0.042 mmol, 0.3 equiv.) was added, and reacted with stirring at -20°C for 2 hours, then the temperature was slowly raised to room temperature and the reaction carried out overnight. After the reaction was completed as monitored by TLC, the reaction was quenched by adding proper amount of triethylamine dropwise. The molecular sieve was removed by diatomite filtration. The filtrate was concentrated under reduced pressure, and purified by column chromatography (PE/acetone 2: 1) to obtain a white solid (195 mg, 90%). R*_{f}* = 0.39 (PE/acetone 1: 1); ¹H NMR (600 MHz, CDCl₃, TMS) *δ* 8.08 (1H, s, NH), 7.88 (1H, d, *N*H*, J* = 9.4 Hz), 7.62, 7.46-7.41, 7.36-7.25, 7.15 (20H, m, aromatic), 5.62 (1H, s, PhC*H*), 5.16 (1H, t, H-4^{GlcA}, *J* = 9.6 Hz), 5.10 (1H, d, H-1^{Gal}, *J* = 3.6 Hz), 5.07 (1H, t, H-3^{GlcA}, *J* = 9.4 Hz), 4.98-4.94 (3H, m, H-2^{GlcA}, H-1^{GlcNAc}, PhC*H*₂), 4.80 (1H, d, PhC*H*₂, *J* = 11.6 Hz), 4.72 (1H, d, H-1^{GalNAc}, *J* = 8.3 Hz), 4.67 (1H, d, PhC*H*₂, *J* = 12.0 Hz), 4.62 (3H, m, H-3^{GlcNAc}, H-4^{GlcNAc}, PhC*H*₂), 4.54 (1H, q, H-2^{GalNAc}, *J* = 9.4 Hz), 4.45 (1H, d, H-4^{GalNAc}, *J* = 3.2 Hz), 4.40-4.36 (3H, m, H-6a^{GalNAc}, H-6a^{GlcNAc}, H-1^{GlcA}), 4.30 (1H, d, PhC*H*₂, *J* = 12.0 Hz), 4.27 (1H, d, PhC*H*₂, *J* = 11.7 Hz), 4.16 (1H, m, H-6b^{GalNAc}), 4.13 (1H, dd, H-6a^{Gal}, *J* = 2.1 Hz, *J* = 11.9 Hz), 4.08 (1H, dd, H-2^{Gal}, *J* = 3.6 Hz, *J* = 10.2 Hz), 3.93 (2H, m, H-5^{GlcA}, H-3^{GalNAc}), 3.88 (1H, dd, H-6b^{GlcNAc}, *J* = 2.1 Hz, *J* = 12.2 Hz), 3.83 (1H, m, H-6b^{Gal}), 3.71 (3H, s, OC*H*₃), 3.70 (1H, dd, H-3^{Gal}, *J* = 2.8 Hz, *J* = 10.5 Hz), 3.63 (1H, d, H-5^{Gal}, J = 8.3 Hz), 3.58 (1H, m, H-5^{GlcNAc}), 3.49 (1H, s, H-5^{GalNAc}), 3.45 (1H, s, H-4^{Gal}), 2.94-2.86 (1H, m, C*H*₂ of Lev), 2.56-2.51 (3H, m, C*H*₂ of Lev, H-2^{GlcNAc}), 2.31-2.28 (1H, m, C*H*₂ of Lev), 2.10 (3H, s, C*H*₃CO), 2.04 (3H, s, C*H*₃CO), 2.00 (12H, m, C*H*₃CO); 13C NMR (150 MHz, CDCl₃, TMS) δ 210.4, 172.3, 170.7, 169.9, 169.8, 169.4(2C), 166.9, 157.9 (q, *J* = 24.5 Hz, *C*OCF₃), 157.7 (q, *J* = 24.3 Hz, *C*OCF₃), 138.1, 137.9, 137.3, 137.2, 130.3, 128.6(2C), 128.3, 128.2, 128.0, 127.9, 127.8, 127.7, 126.4, 116.0 (q, *J* = 191.1 Hz, CO*C*F₃), 115.4 (q, *J* = 191.2 Hz, CO*C*F₃), 101.7, 100.3, 99.1, 98.7, 93.8, 77.6, 76.1, 75.8, 74.7, 74.0, 73.6, 72.6(2C), 72.4, 71.9, 70.9, 70.1, 70.0, 69.9, 69.4, 69.3, 68.6, 66.5, 64.8, 62.3, 58.4, 52.6, 51.3, 37.9, 29.7, 27.7, 20.8, 20.6, 20.4; ESI-Q-TOF (positive mode) calculated for C₇₂H₈₄F₆N₃O₂₉⁺ [M+NH₄]⁺ m/z 1568.5095, found 1568.5099.

### Synthesis of benzyl methyl 2,3,4-tri-O-acetyl-β-D-glucopyranuronate-(1→3)-4,6-di-O -acetyl-2-deoxy-2-trifluoroacetylamino-β-D-glucopyranosyl-(1→2)-3,4-di-O-benzyl-α-D-ga lactopyranosyl-(1→3)-2-deoxy-2-trifluoroacetylamino-4,6-O-benzylidene-β-D-galactopyra noside

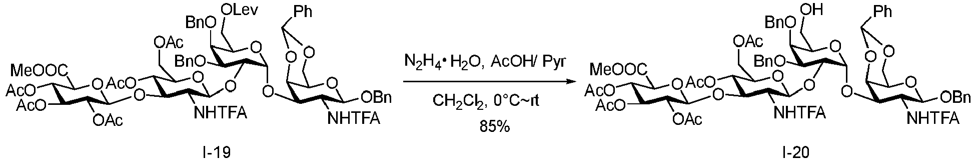

Benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-6-acetylpropionyl-α -D-galactopyranosyl-(1→3)-2-deoxy-2-trifluoroacetylamino-4,6-O-benzylidene-β-D-galactopyr anoside (224 mg, 0.14 mmol) was dissolved in dichloromethane (2.0 mL) and 0.5 M hydrazine acetate was added dropwise under ice bath conditions (0.87 mL, hydrazine hydrate dissolved in a mixed solution of pyridine/acetic acid=3:2), the temperature was slowly raised to room temperature and the reaction was carried out for 1 hour. After the reaction was completed as monitored by TLC, the reaction solution was diluted with dichloromethane, and the organic phase was washed with 1M hydrochloric acid solution, saturated sodium bicarbonate solution and saturated salt water in turn, dried with anhydrous sodium sulfate, concentrated under reduced pressure, and purified by column chromatography (DCM/MeOH=50: 1) to obtain a white solid (178 mg, 85%). R*_{f}* = 0.21 (DCM/MeOH 30: 1); ¹H NMR (600 MHz, CDCl₃, TMS) *δ* 7.56, 7.39, 7.32-7.19, 7.01 (23H, m, aromatic, N*H*, O*H*), 5.61 (1H, s, PhC*H*), 5.14 (1H, t, H-4^{GlcA}, *J* = 9.4 Hz), 5.10 (1H, t, H-3^{GlcA}, *J* = 8.9 Hz), 5.06 (1H, d, H-1^{Gal}, *J* = 3.4 Hz), 4.90 (2H, m, H-2^{GlcA}, PhC*H*₂), 4.81 (1H, d, H-1^{GlcNAc}, *J* = 8.4 Hz), 4.73 (2H, m, H-4^{GlcNAc}, PhC*H*₂), 4.65-4.59 (3H, m, H-1^{GalNAc}, PhC*H*₂), 4.41 (1H, d, H-1^{GlcA}, *J* = 8.0 Hz), 4.39-4.29 (5H, m, H-2^{GalNAc}, H-4^{GalNAc}, H-6a^{GalNAc}, PhC*H*₂), 4.26 (1H, dd, H-6a^{GlcNAc}, *J* = 5.6 Hz, *J* = 12.6 Hz), 4.17 (2H, m, H-3^{GlcNAc}, H-6b^{GalNAc}), 4.06 (1H, m, H-6b^{GlcNAc}), 4.00 (1H, dd, H-2^{Gal}, *J* = 3.5 Hz, *J* = 10.2 Hz), 3.93 (2H, m, H-3^{GalNA}c, H-5^{GlcA}), 3.76 (1H, dd, H-3^{Gal}, *J* = 2.6 Hz, *J* = 10.1 Hz), 3.72 (3H, s, OC*H*₃), 3.70 (1H, m, H-5^{Gal}), 3.61 (2H, m, H-6a^{Gal}, H-5^{GlcNAc}), 3.50 (1H, d, H-4^{Gal}, *J* = 1.4 Hz), 3.43 (2H, m, H-5^{GalNAc}, H-2^{GlcNAc}), 3.32 (1H, m, H-6b^{Gal}), 2.11 (3H, s, C*H*₃CO), 2.00 (9H, m, C*H*₃CO), 1.92 (3H, s, C*H*₃CO); ¹³C NMR (150 MHz, CDCl₃, TMS) δ 171.2, 169.9, 169.6, 169.4, 169.3, 166.8, 157.8 (q, *J* = 24.5 Hz, *C*OCF₃), 157.5 (q, *J* = 24.6 Hz, *C*OCF₃), 138.0, 137.8, 137.7, 137.0, 129.4, 128.6, 128.4(2C), 128.2, 128.0(2C), 127.9(2C), 127.7, 126.3,115.8 (q, *J* = 191.1 Hz, CO*C*F₃), 115.5 (q, *J* = 191.1 Hz, CO*C*F₃), 101.0, 100.6, 100.2, 98.2, 97.5, 77.3, 76.7, 75.8, 74.7, 72.9, 72.4(2C), 72.3, 71.7, 70.9, 69.9, 69.3, 69.1, 68.1, 66.8, 62.5, 62.1, 56.7, 52.8, 52.7, 20.8, 20.6, 20.5, 20.4(2C); ESI-Q-TOF (positive mode) calculated for C₆₇H₇₈F₆N₃O₂₇⁺ [M+NH₄]⁺ m/z 1470.4727, found 1470.4736.

### Synthesis of benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyranuronic acid-(1→3)-2-deoxy-2-trifluoroacetylamino-4,6-O-benzylidene-β-D-galactopyranoside

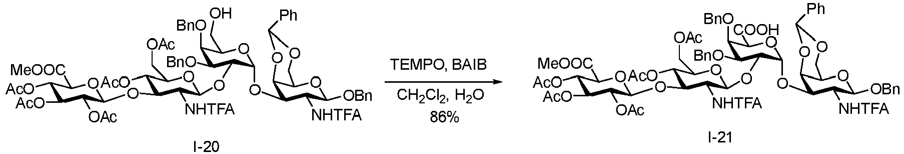

Benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyranosyl-(1→ 3)-2-deoxy-2-trifluoroacetylamino-4,6-O-benzylidene-β-D-galactopyranoside (155 mg, 0.11 mmol) was dissolved in dichloromethane/water (0.8 mL/0. 4 ml), and 2,2,6,6-tetramethylpiperidine-nitrogen-oxide (7 mg, 0.044 mmol, 0.4 equiv.) and diacetoxy iodobenzene (71 mg, 0.22 mmol, 2.0 equiv.) were added in turn under ice bath conditions, and the temperature was naturally raised to room temperature and the reaction was carried out overnight. After the reaction was completed as monitored by TLC, the reaction solution was diluted with dichloromethane, the organic phase was washed with saturated sodium thiosulfate solution, dried with anhydrous sodium sulfate, concentrated under reduced pressure, and purified by column chromatography (DCM/MeOH=60:1, 0.1% CH₃COOH) to obtain a pale yellow solid (135 mg, 86%). R*_{f}* = 0.18 (DCM/MeOH 30:1); ¹H NMR (600 MHz, CDCl₃+CD₃COOD, TMS) *δ* 7.58, 7.39, 7.31-7.24, 7.18, 7.15 (22H, m, aromatic, NH), 5.69 (1H, s, PhC*H*), 5.24 (1H, s, H-1^{GalA}), 5.15 (2H, m, H-3^{GlcA}, H-4^{GlcA}) 4.95 (1H, t, H-2^{GlcA}, *J* = 8.3 Hz), 4.91 (1H, d, PhC*H*₂, *J* = 12.2 Hz), 4.78 (1H, t, H-4^{GlcNAc}, *J* = 9.5 Hz), 4.71-4.64 (5H, m, H-1^{GlcNAc}, H-1^{GalNAc}, 3PhC*H*₂), 4.52 (1H, d, H-1^{GlcA}, *J* = 8.1 Hz), 4.46 (1H, s, H-4^{GalNAc}), 4.36 (2H, m, H-6a^{GalNAc}, PhC*H*₂), 4.30-4.23 (5H, m, H-2^{GalNAc}, H-5^{GalA}, H-6a^{GlcNAc}, H-6b^{GalNAc}, PhC*H*₂), 4.13 (1H, m, H-3^{GlcNAc}), 4.05 (2H, m, H-3^{GalNA}c, H-6b^{GlcNAc}), 3.99 (2H, m, H-2^{GalA}, H-5^{GlcA}), 3.86 (1H, dd, H-3^{GalA}, *J* = 2.2 Hz, *J* = 10.0 Hz), 3.80 (1H, s, H-4^{GalA}), 3.73 (3H, s, OC*H*₃), 3.71-3.60 (2H, m, H-2^{GlcNAc}, H-5^{GlcNAc}), 3.47 (1H, s, H-5^{GalNAc}), 2.13 (3H, s, C*H*₃CO), 2.04 (3H, s, C*H*₃CO), 2.00 (6H, s, 2C*H*₃CO), 1.93 (3H, s, C*H*₃CO); ¹³C NMR (150 MHz, CDCl₃+CD₃COOD, TMS) δ 172.8, 171.6, 170.3, 170.0, 169.9, 169.5, 167.2, 157.7 (q, *J* = 24.7 Hz, 2*C*OCF₃), 137.7, 137.0, 136.9, 129.5, 128.8, 128.5, 128.4, 128.2, 128.1(2C), 128.0, 127.8, 126.5, 115.8 (q, *J* = 191.1 Hz, 2CO*C*F₃), 101.4, 101.2, 100.5, 98.5, 77.7, 75.8, 75.6, 73.8, 72.5(3C), 71.0, 70.8, 70.1, 69.5, 69.1, 68.2, 66.7, 62.3, 56.1, 52.9, 52.5, 20.8, 20.6(2C), 20.4; ESI-Q-TOF (positive mode) calculated for C₆₇H₇₆F₆N₃O₂₈⁺ [M+NH₄]⁺ m/z 1484.4520, found 1484.4539.

### Synthesis of benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-N-L-threonine methyl ester-3,4-di-O-benzyl-α-D-galactopyranuronic acid-(1→3)-2-deoxy-2-trifluoro acetylamino-4,6-O-benzylidene-β-D-galactopyranoside

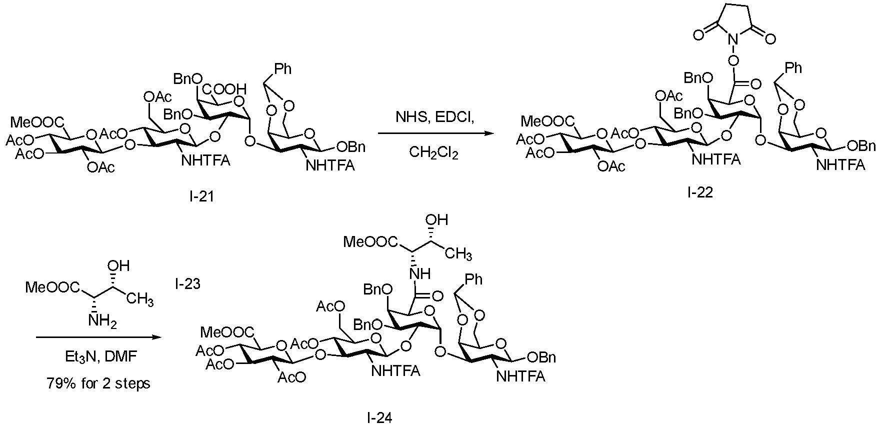

Benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyran uronic acid-(1→3)-2-deoxy-2-trifluoroacetylamino-4,6-O-benzylidene-β-D-galactopyranoside (70.0 mg, 0.048 mmol) was dissolved in anhydrous dichloromethane (0.6 mL), and N-hydroxysuccinimide (11.0 mg, 0.096 mmol, 2.0 equiv.) and 1-(3-dimethylaminopropyl)-3-ethylcarbodiimide hydrochloride (18.4 mg, 0.096 mmol, 2.0 equiv.) were added in turn, and reaction was carried out overnight at room temperature. After the reaction was completed as monitored by TLC, the reaction solution was diluted with dichloromethane, the organic phase was washed with 1M hydrochloric acid solution, saturated sodium bicarbonate solution and saturated salt water in turn, dried with anhydrous sodium sulfate, and concentrated under reduced pressure to obtain a crude product, which was directly used for the next reaction. The crude product and L-threonine methyl ester (40.7 mg, 0.24 mmol, 5.0 equiv.) were dissolved in N,N-dimethylformamide/triethylamine (2.0 mL/0. 1 ml), and the temperature was raised to 40°C and the reaction was carried out overnight. After the reaction was completed as monitored by TLC, the reaction solution was directly concentrated under reduced pressure and purified by column chromatography (DCM/MeOH=60: 1) to obtain a light yellow solid (59.6 mg, yield of two-step 79%). R*_{f}* = 0.26 (DCM/MeOH 30:1); ¹H NMR (600 MHz, CDCl₃, TMS) *δ* 8.00, 7.84, 7.54, 7.39, 7.33-7.17, 7.04 (23H, m, aromatic, NH), 5.68 (1H, s, PhC*H*), 5.23 (1H, d, H-1^{GalA}, *J* = 2.9 Hz), 5.15-5.10 (2H, m, H-3^{GlcA}, H-4^{GlcA}), 4.92 (2H, m, H-2^{GlcA}, PhC*H*₂), 4.79 (2H, m, H-1^{GlcNAc}, H-4^{GlcNAc}), 4.70 (1H, d, PhC*H*₂, *J* = 10.9 Hz), 4.65 (2H, m, PhC*H*₂, H-1^{GalNAc}), 4.59 (1H, d, PhC*H*₂, *J* = 12.3 Hz), 4.50 (1H, d, H-1^{GlcA}, *J* = 8.0 Hz), 4.48 (1H, d, H-4^{GalNAc}, *J* = 2.8 Hz), 4.45 (1H, dd, C*H*, *J* = 2.2 Hz, *J* = 8.9 Hz), 4.41 (1H, d, PhC*H*₂, *J* = 11.0 Hz), 4.37 (2H, m, PhC*H*₂, H-6a^{GalNAc}), 4.32-4.23 (6H, m, H-5^{GalA}, H-6a^{GlcNAc}, H-2^{GalNAc}, H-6b^{GalNAc}, H-3^{GlcNAc}, CH), 4.12 (2H, m, H-4^{GalA}, H-6b^{GlcNAc}), 4.02 (2H, m, H-2^{GalA}, H-3^{GalNAc}), 3.94 (1H, m, H-5^{GlcA}), 3.81 (1H, d, H-3^{GalA}, *J* = 2.2 Hz, *J* = 9.9 Hz), 3.71 (6H, s, 2OC*H*₃), 3.62 (1H, m, H-5^{GlcNAc}), 3.49 (1H, s, H-5^{GalNAc}), 3.44 (1H, m, H-2^{GlcNAc}), 2.12 (3H, s, C*H*₃CO), 2.02 (3H, s, C*H*₃CO), 1.99 (6H, s, 2C*H*₃CO), 1.89 (3H, s, C*H*₃CO), 0.96 (3H, d, C*H*₃, *J* = 6.4 Hz); ¹³C NMR (150 MHz, CDCl₃, TMS) δ 171.4(2C), 170.0, 169.7, 169.6, 169.4, 168.8, 167.0, 162.8, 157.8 (q, *J* = 24.6 Hz, 2*C*OCF₃), 157.5 (q, *J* = 24.6 Hz, 2*C*OCF₃), 138.4, 137.7, 137.5, 137.1, 129.4, 128.6, 128.5, 128.4, 128.1, 128.0, 127.9, 127.8, 127.7, 127.6, 126.3, 115.9 (q, *J* = 191.1 Hz, 2CO*C*F₃),115.7 (q, *J* = 191.1 Hz, 2CO*C*F₃), 101.0, 100.9, 100.2, 98.4, 97.7, 77.5, 76.5, 76.3, 76.2, 75.3, 75.1, 72.9, 72.6, 72.5, 72.4(2C), 72.1, 70.9, 70.1, 69.4, 69.0, 68.0, 67.4, 66.8, 62.1, 57.0, 56.6, 52.8, 52.7, 52.6, 20.7, 20.6, 20.5, 20.4(2C), 19.6; ESI-Q-TOF (positive mode) calculated for C₇₂H₈₅F₆N₄O₃₀⁺ [M+NH₄]⁺ m/z 1599.5153, found 1599.5133.

### Synthesis of benzyl β-D-glucopyranuronic acid-(1→3)-2-deoxy-2-acetylamino-β-D-glucopyranosyl-(1→2)-3,4-di-O-benzyl-α-D-galactopyranoic acid-(1→3)-2-deoxy-2-acetylamino-4,6-O-benzylidene-β-D-galactopyranoside

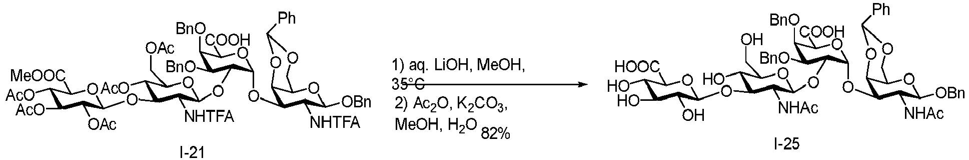

Benzyl methyl 2,3,4-tri-O-acetyl-β-D-glucopyranuronate-(1→3)-4,6-di-O-acetyl-2-deoxy -2-trifluoroacetylamino-β-D-glucopyranosyl-(1→2)-3,4-di-O-benzyl-α-D-galactopyranuronic acid-(1→3)-2-deoxy-2-trifluoroacetylamino-4,6-O-benzylidene-β-D-galactopyranoside (30.0 mg, 0.02 mmol) was dissolved in methanol (1.0 mL), and saturated lithium hydroxide solution (1.0 mL) was added dropwise, and the temperature was raised to 35 °C and the reaction was carried out for 48h. After the reaction was completed as monitored by TLC, the reaction solution was neutralized with IR-120 cation exchange resin to pH=7, filtered, the resin was washed with methanol. The filtrate was concentrated under reduced pressure, and the concentrated crude product was dissolved in a mixed solution of methanol/water (1.0 mL/1.0 mL). Potassium carbonate solid was added to adjust pH=11-12, acetic anhydride (0.3 mL) was added dropwise, and potassium carbonate solid was added to adjust pH=11-12, and the reaction was carried out overnight at room temperature. After the reaction was monitored by TLC to be completed, the reaction solution was neutralized with IR-120 cation exchange resin to pH=7, filtered, the resin was washed with methanol. The filtrate was concentrated under reduced pressure, and purified by Sephadex LH-20 with CH₂Cl₂/MeOH 1:1 as eluent, to obtain a white solid (18.6 mg, two-step yield 82%). R*_{f}* = 0.58 (CHCl₃/MeOH/H₂O=1:1:0.3); ¹H NMR (600 MHz, MeOD-d₆, TMS) *δ* 7.64, 7.40, 7.36-7.30, 7.27-7.19 (20H, m, aromatic), 5.61 (1H, s, PhC*H*), 5.41 (1H, s, H-1^{GalA}), 4.89 (1H, m, PhC*H*₂), 4.77 (3H, m, 2PhC*H*₂, H-4^{GalNAc}), 4.68-4.57 (5H, m, 3PhC*H*₂, H-1^{GlcNAc}, H-1^{GalNAc}), 4.29-4.19 (6H, m, H-4^{GalA}, H-5^{GalA}, H-1^{GlcA}, H-2^{GalNAc}, H-6a^{GalNAc}, H-6b^{GalNAC}), 4.03 (1H, d, H-2^{GalA}, *J* = 7.4 Hz), 3.93-3.81 (5H, m, H-3^{GalA}, H-2^{GlcNAc}, H-6a^{GlcNAc}, H-6b^{GlcNAc}, H-3^{GalNAc}), 3.66 (1H, m, H-3^{GlcNAc}), 3.59 (1H, m, H-5^{GlcA}), 3.54 (1H, s, H-5^{GalNAc}), 3.47 (2H, m, H-4^{GlcA}, H-4^{GlcNAc}), 3.38 (1H, m, H-3^{GlcA}), 3.35 (1H, s, H-5^{GlcNAc}), 3.26 (1H, t, H-2^{GlcA}, *J* = 8.0 Hz); ¹³C NMR (150 MHz, MeOD-d₆, TMS) δ 175.9, 174.9, 174.1, 173.9, 140.2, 139.8, 139.1, 129.7, 129.4, 129.3, 129.1, 129.0, 128.9, 128.6, 128.4, 128.3, 127.8, 105.1, 104.8, 102.0, 101.2, 86.5, 80.7, 77.4, 77.3, 76.1, 75.4, 74.6, 74.2, 73.8, 73.3, 72.9, 71.5, 70.4, 70.1, 68.3, 62.3, 56.6, 52.3, 49.6, 23.3, 23.2; ESI-Q-TOF (negative mode) calculated for C₅₆H₆₅N₂O₂₃⁻[M-H]⁻m/z 1133.3984, found 1133.3983.

### Synthesis of β-D-glucopyranuronic acid-(1→3)-2-deoxy-2-acetylamino-β-D-glucopyranosyl-(1→2)-α-D-galactopyranoic acid-(1→3)-2-deoxy-2-acetylamino-α,β-D-galactopyranoside (Compound CP-1)

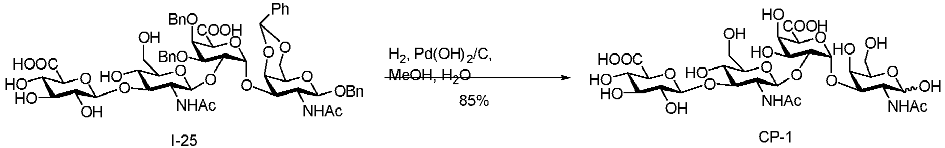

Benzyl β-D-glucopyranuronic acid-(1→3)-2-deoxy-2-acetylamino-β-D-glucopyranosyl-(1 →2)-3,4-di-O-benzyl-α-D-galactopyranoic acid-(1→3)-2-deoxy-2-acetylamino-4,6-O-benzylidene-β-D-galactopyranoside (18.6 mg, 16.4 µ mol) was dissolved in a mixed solution of methanol/water (1.0 mL/1. 0 ml), 10% palladium hydroxide carbon (37.0 mg) was added, and the reaction was carried out at room temperature for 48 hours at 40Pa hydrogen pressure. After the reaction was completed as monitored by TLC, it was filtered, concentrated under reduced pressure, and purified by Sephadex LH-20 with pure water as eluent to obtain a white solid (10.8 mg, 85%). R*_{f}* =0.11 (CHCl₃/MeOH/H₂O=1:1:0.3); ¹H NMR (600 MHz, D₂O) *δ* 5.31 (0.97H, s), 5.19 (0.53H, d, *J* = 3.3 Hz), 4.68 (1.69H, m), 4.49 (1.02H, d, *J* = 7.7 Hz), 4.37 (0.58H, s), 4.31 (0.49H, s), 4.24 (1.52H, m), 4.18 (0.63H, s), 4.11 (0.52H, m), 3.98-3.75 (11.26H, m), 3.70 (0.53H, m), 3.51 (3.46H, m), 3.35 (0.92H, m), 2.02 (3H, s), 1.98 (2.88H, d, *J* = 4.4 Hz); ¹³C NMR (150 MHz, D₂O) *δ* 175.5, 103.9, 103.8, 103.7, 97.8, 97.5, 95.8, 92.0, 83.2, 78.7, 78.6, 78.4, 76.6, 76.2(3C), 76.0, 75.4, 73.5, 72.7, 72.4, 71.7, 70.7, 69.4(2C), 69.1, 69.0, 66.3, 65.4, 61.9, 61.7, 61.6, 61.5, 55.4, 52.8, 49.2, 23.0, 22.9, 22.7; ESI-Q-TOF (positive mode) calculated for C₂₈H₄₂N₂O₂₃²⁻[M-2H]²⁻m/z 387.1095, found 387.1090.

### Synthesis of benzyl β-D-glucopyranuronic acid-(1→3)-2-deoxy-2-acetylamino-β-D-glucopyranosyl-(1→2)-N-L-threonin-3,4-di-O-benzyl-α-D-galactopyranoic acid-(1→3)-2-deoxy-2-acetylamino-β-D-galactopyranoside

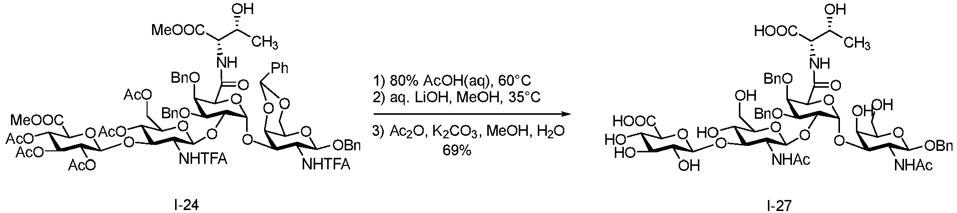

Benzyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-N-L-threonine methyl ester-3,4-di-O-benzyl-α-D-galactopyranoic acid-(1→3)-2-deoxy-2-trifluoroacetylamino-4,6-O-benzylidene-β-D-galactopyranoside (30 mg, 19.0 µmol) was dissolved in 80% aqueous AcOH solution (2.0 mL), heated to 60°C and reacted for 2h. After the temperature was returned to room temperature at the end of the reaction, the solvent was dried with toluene to obtain a crude product, which was then directly dissolved in methanol (1.0 mL). Saturated lithium hydroxide solution (1.0 mL) was added dropwise, and the temperature was raised to 35°C and the reaction was carried out for 48 hours. After the reaction was completed as monitored by TLC, the reaction solution was neutralized with IR-120 cation exchange resin to pH=7, filtered, the resin was washed with methanol, the filtrate was concentrated under reduced pressure, the concentrated crude product was dissolved in the mixed solution of methanol/water (1.0 mL/1. 0 mL). Potassium carbonate solid was added to adjust pH=11-12, acetic anhydride was added dropwise (0.3 mL), and potassium carbonate solid was added to adjust pH=11-12, and the reaction was carried out overnight at room temperature. After the reaction was completed as monitored by TLC, the reaction solution was neutralized with IR-120 cation exchange resin to pH=7, filtered, the resin was washed with methanol, the filtrate was concentrated under reduced pressure, and purified by Sephadex LH-20 with CH₂Cl₂/MeOH 1:1 as eluent, to obtain a white solid (15.1 mg, three-step yield 69%). ¹H NMR (600 MHz, CD₃OD+D₂O) *δ* 7.42-7.25 (15H, m, aromatic), 5.52 (1H, d, H-1^{GalA}, *J* = 3.7 Hz), 4.92-4.64 (6H, m, 5PhC*H*₂, H-1^{GlcNAc}), 4.49 (2H, m, H-1^{GalNAc}, PhC*H*₂), 4.42 (1H, d, H-1^{GlcA}, *J* = 7.8 Hz), 4.37 (1H, d, H-4^{GalNAc}, *J* = 2.8 Hz), 4.32 (1H, m, H-4^{GalA}), 4.29 (1H, s, H-5^{GalA}), 4.21-4.16 (3H, m, C*H*OH, C*H*NH, H-2^{GalNAc}), 4.12 (1H, dd, H-2^{GalA}, *J* = 3.6 Hz, *J* = 10.1 Hz), 3.98 (1H, dd, H-3^{GalA}, *J* = 2.9 Hz, *J* = 10.3 Hz), 3.94-3.77 (7H, m, H-6a^{GalNAc}, H-6b^{GalNAc}, H-3^{GalNAc}, H-2^{GlcNAc}, H-3^{GlcNAc}, H-6a^{GlcNAc}, H-6b^{GlcNAc}), 3.68 (1H, d, H-5^{GlcA}, *J* = 9.6 Hz), 3.65 (1H, t, H-5^{GalNAc}, *J* = 6.7 Hz), 3.53-3.45 (4H, m, H-4^{GlcA}, H-4^{GlcNAc}, H-3^{GlcA}, H-5^{GlcNAc}), 3.32 (1H, m, H-2^{GlcA}), 1.84 (3H, s, C*H*₃CO), 1.75 (3H, s, C*H*₃CO), 0.98 (3H, d, C*H*₃, *J* = 6.2 Hz); ¹³C NMR (150 MHz, CD₃OD+D₂O) *δ* 175.6, 173.7, 173.3, 169.2, 138.2, 137.8, 137.4, 128.4, 128.3, 128.1, 127.8, 127.7, 127.4, 103.3, 103.1, 100.9, 96.9, 83.2, 78.0, 77.9, 76.6, 76.1, 76.0, 75.7, 75.2, 74.8, 74.6, 73.0, 72.9, 71.9, 71.1, 70.8, 69.0, 67.7, 64.4, 60.9(2C), 59.0, 55.0, 50.5, 22.0, 21.8, 19.1; ESI-Q-TOF (negative mode) calculated for C₅₃H₆₇N₃O₂₅²⁻[M-2H]²⁻m/z 572.7037, found 572.7036.

### Example 2

### Synthesis of β-D-glucopyranuronic acid-1→3)-2-deoxy-2-acetylamino-β-D-glucopyranosyl-(1→2)-N-L-threonine-α-D-galactopyranoic acid -(1→3)-2-deoxy-2-acetylamino-α, β-D-galactopyranoside (Compound CP-2)

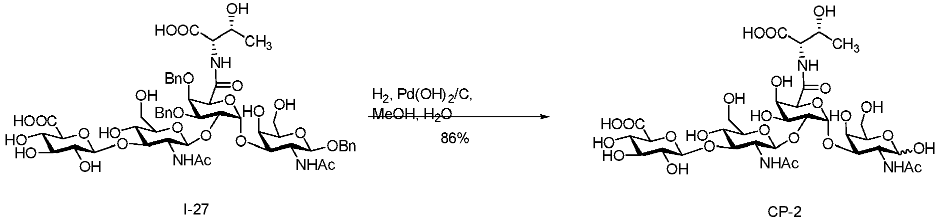

Benzyl β-D-glucopyranuronic acid-(1→3)-2-deoxy-2-acetylamino-β-D-glucopyranosyl-(1→2)-N-L-threonin-3,4-di-O-benzyl-α-D-galactopyranoic acid-(1→3)-2-deoxy-2-acetylamino-β-D-galactopyranoside (19.0 mg, 16.6 µmol) was dissolved in a mixed solution of methanol/water (1.0 mL/1.0 ml), 10% palladium hydroxide carbon (38.0 mg) was added, and the reaction was carried out at room temperature for 48 hours at 40Pa hydrogen pressure. After the reaction was completed as monitored by TLC, it was filtered, concentrated under reduced pressure, and purified by Sephadex LH-20 with pure water as eluent to obtain a white solid (12.5 mg, 86%). ¹H NMR (600 MHz, D₂O) *δ* 5.46 (0.86H, m), 5.18 (0.48H, d, *J* = 3.6 Hz), 4.81 (0.56H, m), 4.66 (0.95H, m), 4.48 (0.98H, d, *J* = 7.9 Hz), 4.39 (0.47H, d, *J* = 2.4 Hz), 4.32-4.23 (5.18H, m), 4.14-4.06 (1.13H, m), 3.99-3.68 (11.95H, m), 3.55-3.47 (4.46H, m), 3.34 (1.26H, m); ¹³C NMR (150 MHz, D₂O) *δ* 175.69, 175.03, 170.93, 170.76, 103.9, 103.8, 103.7, 98.0, 97.7, 96.0, 92.0, 83.1, 79.0, 78.3, 78.2, 76.6, 76.4, 76.2(2C), 76.0, 75.4, 73.5, 72.5, 72.2, 72.1, 70.7, 70.6(2C), 69.4, 69.3, 69.1, 69.0, 68.7(2C), 66.4, 65.5, 61.9, 61.6, 61.5, 60.4, 55.4, 52.6, 49.2, 23.0, 22.8, 22.5, 20.0; ESI-Q-TOF (negative mode) calculated for C₃₂H₄₉N₃O₂₅²⁻[M-2H]²⁻m/z 437.6333, found 437.6323.

### Example 3

### Synthesis of methyl 2-deoxy-2-trifluoroacetamido-3,4,6-tri-O-acetyl-β-D-galactopyranoside

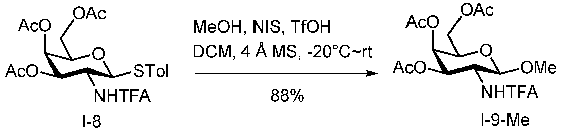

P-tolyl 2-deoxy-2-trifluoroacetamido-3,4,6-tri-O-acetyl-1-thio-β-D- galactopyranoside (101 mg, 0.20 mmol), N-iodosuccinimide (70 mg, 0.31 mmol, 1.5 equiv.) and 4Å molecular sieve were dissolved in anhydrous dichloromethane (1.7 mL) under the protection of argon, and methanol ( 50 µL, 1.24 mmol, 6.0 equiv. ) was added, and stirred at room temperature for 2 hours, then cooled to -20°C. Trifluoromethanesulfonic acid (5.0 µL, 62.5 µmol, 0.30 equiv.) was added dropwise, and the reaction was carried out with continuously stirring for 3 hours, and moved to room temperature and the reaction was carried out overnight. After the reaction was completed as monitored by TLC, the reaction was quenched by adding proper amount of trimethylamine. The molecular sieve was filtered out by diatomite, and the filter cake was washed by dichloromethane several times. The filtrates were combined, concentrated under reduced pressure, and purified by column chromatography (petroleum ether /EtOAc 3:1) to obtain a white solid (73 mg, 88%). R*_{f}* = 0.39 (petroleum ether/EtOAc 1: 1); ¹H NMR (400 MHz, CCl₃, TMS) δ 7.29 (1H, d, *J* = 8.9 Hz, NH), 5.37 (1H, d, *J*=3.2 Hz), 5.28 (1H, dd, *J* = 11.2, 3.4 Hz), 4.57 (1H, d, *J* = 8.4 Hz) 4.25-4.06 (3H, m), 3.98 (1H, t, *J* = 6.5), 3.49 (3H, s), 2.15 (3H, s), 2.03 (3H, s), 1.96 (3H, s); ¹³C NMR (100 MHz, CDCl₃, TMS) δ 170.5, 170.5, 170.2, 157.5, 114.2, 101.2, 70.7, 69.5, 66.5, 61.3, 57.0, 51.8, 20.6, 20.6, 20.3. MS (ESI-MS) calculated for C₁₅H₂₁F₃NO₉⁺ [M+H]⁺ m/z 415.3, found 416.3.

### Synthesis of methyl 2-deoxy-2-trifluoroacetamido-β-D-galactopyranoside

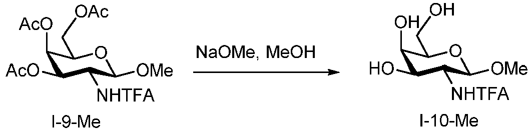

Methyl 2-deoxy-2-trifluoroacetamido-3,4,6-tri-O-acetyl-β-D-galactopyranoside (3.10 g, 7.48 mmol) was dissolved in methanol (50.0 mL), pH was adjusted to 9-10 by adding proper amount of sodium methoxide, and the reaction was carried out with stirring at room temperature for 2 hours. After the reaction was completed as monitored by TLC, cationic resin was added to the reaction solution and neutralized it to pH = 7, filtered, and filtrate was concentrated under reduced pressure to dryness to obtain a yellow syrup, R*_{f}* = 0.75 (DCM/MeOH 5: 1), which was directly used for the next reaction.

### Synthesis of methyl 2-deoxy-2-trifluoroacetamido-4,6-O-benzylidene-β-D-galactopyranoside

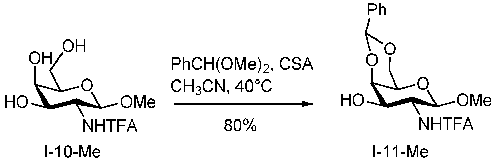

Methyl 2-deoxy-2-trifluoroacetamido-β-D-galactopyranoside (2.16 g, 7.48 mmol) and (+)-camphor sulfonic acid (3.46 g, 14.9 mmol, 2.0 equiv.) were dissolved in anhydrous acetonitrile (60.0 mL) under the protection of argon. Benzaldehyde dimethyl acetal (4.36 mL, 29.0 mmol, 4.0 equiv.) was added and the temperature was raised to 40°C and the reaction was carried out overnight. After the reaction was completed as monitored by TLC, the reaction was quenched by adding proper amount of triethylamine dropwise. The reaction solution was concentrated under reduced pressure, and purified by column chromatography (petroleum ether/EtOAc 2: 1) to obtain a compound as white solid (2.24 g, 80%). R*_{f}* = 0.72 (petroleum ether/acetone 1: 2). MS (ESI-MS) calculated for C₁₆H₁₉F₃NO₆⁺ [M+H]⁺ m/z 378.3, found 378.3.

### Methyl 2-O-p-methoxybenzyl-3,4-di-O-benzyl-6-acetylpropionyl-α-D-galactopyranosyl-(1→3)-2-deoxy-2-trifluoroacetamido-4,6-O-benzylidene-β-D-galactopyr anoside

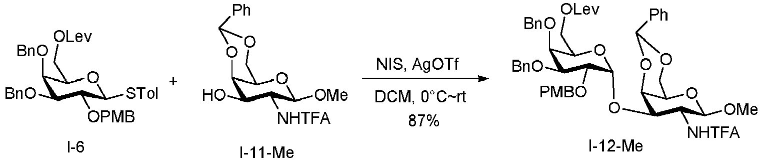

P-tolyl 2-O-p-methoxybenzyl-3,4-di-O-benzyl-6-acetylpropionyl-1-thio-β-D-galactopyranoside (5.80 g, 8.48 mmol, 1.5 equiv.) and methyl 2-deoxy-2-trifluoroacetamido-4,6-O-benzylidene-β-D-galactopyranoside (2.15 g, 5.70 mmol) were dissolved in anhydrous dichloromethane/N,N-dimethylformamide (70.0 mL/14.0 mL) under the protection of argon, and 4Å molecular sieve (8.0 g) was added, stirred at room temperature for 2 hours, and then cooled to 0°C. N-iodosuccinimide (2.50 g, 11.1 mmol, 2.0 equiv. ) and silver trifluoromethanesulfonate (731 mg, 2.85 mmol, 0.5 equiv.) were added in turn, the reaction was carried out with stirring at 0°C for 2 hours, then the temperature was slowly raised to room temperature and the reaction was carried out overnight. After the reaction was completed as monitored by TLC, the reaction was quenched by adding proper amount of triethylamine dropwise. The molecular sieve was removed by diatomite filtration, the filtrate was concentrated under reduced pressure, and purified by column chromatography (petroleum ether/EtOAc 1: 1) to obtain the compound I-12 (4.66 g, 87%) as a white solid. R*_{f}* = 0.58 (DCM/MeOH 25:1); ¹H NMR (400 MHz, CDCl₃, TMS) δ 7.75 (1H, b), 7.61-7.48 (2H, m), 7.39-7.17 (13H, m), 6.95 (2H, d, *J* = 8.5 Hz), 6.62 (2H, d, *J* = 8.6 Hz), 5.54 (1H, s,), 5.20 (1H, d, *J* = 3.6 Hz), 4.94 (1H, d, *J* = 11.4 Hz), 4.85-4.73 (2H, m), 4.65 (1H, d, *J* = 11.8 Hz), 4.54 (1H, d, *J* = 11.4 Hz), 4.46 (2H, s), 4.42 (1H, s), 4.32 (1H, d, *J* = 12.4), 4.20 (3H, q, *J* = 5.3, 4.0 Hz), 4.12-4.02 (4H, m), 3.92-3.87 (1H, m), 3.85-3.81 (1H, m), 3.79-3.74 (1H, m), 3.73(3H, s) 3.50 (3H, s), 2.90-2.79 (1H, m), 2.75-2.38 (3H, m), 2.18 (3H, s); ¹³C NMR (100 MHz, CDCl₃, TMS) δ 208.9, 172.5, 158.9, 157.2 (COCF₃), 138.6, 138.3, 137.6, 130.5, 129.5, 129.0, 128.4, 128.3(2C), 128.2, 127.8, 127.7, 127.5, 126.4, 116.0 (COCF3), 113.5, 101.1, 100.6, 92.8, 78.2, 75.4, 74.7, 74.6, 73.5, 71.4, 71.1, 70.5, 70.1, 69.4, 66.5, 64.2, 56.5, 55.2, 52.5, 38.0, 29.8, 27.8; ESI-Q-TOF (positive mode) calculated for C₄₉H₅₄F₃NO₁₄⁺ [M+NH₄]⁺ m/z 955.3840, found 955.3956.

### Methyl 3,4-di-O-benzyl-6-acetylpropionyl-α-D-galactopyranosyl-(1→3)-2-deoxy -2-trifluoroacetamido-4,6-O-benzylidene-β-D-galactopyranoside

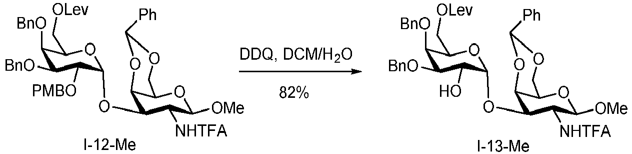

Methyl 2-O-p-methoxybenzyl-3,4-di-O-benzyl-6-acetylpropionyl-β-D-galactopyranosyl-(1→3)-2-deoxy-2-trifluoroacetamido-4,6-O-benzylidene-β-D-galactopyranoside (4.66 g, 4.97 mmol) was dissolved in dichloromethane/water (100 mL/10.0 ml), and 2,3-dichloro-5,6-dicyano-p-benzoquinone (2.17 g, 9.56 mmol, 1.9 equiv.) was added in batches, and the reaction was carried out for 1 hour at room temperature. After the reaction was completed as monitored by TLC, the reaction solution was diluted with dichloromethane, and the organic phase was washed with saturated sodium bicarbonate solution, saturated sodium thiosulfate solution and saturated salt water in turn, dried with anhydrous sodium sulfate, filtered, concentrated under reduced pressure, and purified by column chromatography (DCM/MeOH 40: 1) to obtain a white solid (3.32 mg, 82%). R*_{f}* = 0.79 (petroleum ether/acetone 1: 1); ¹H NMR (400 MHz, CDCl₃, TMS) δ 7.71 (1H, d, *J* = 9.3 Hz), 7.55 (2H, dd, *J* = 7.8, 1.8 Hz), 7.43-7.22 (13H, m), 5.60 (1H, s), 5.19 (1H, d, *J* = 3.9 Hz), 4.94 (1H, d, *J* = 11.4 Hz), 4.86 (1H, d, *J* = 11.8 Hz), 4.67-4.57 (2H, m), 4.50 (1H, d, *J* = 11.5 Hz), 4.47-4.35 (3H, m), 4.20 (1H, dd, *J* = 11.8, 2.7 Hz), 4.15-4.02 (4H, m), 3.83-3.76 (1H, m), 3.72 (1H, b), 3.58 (1H, dd, *J* = 10.0, 2.7 Hz), 3.52-3.46 (4H, m), 2.93 (1H, ddd, *J* = 19.1, 10.1, 3.5 Hz), 2.74-2.55 (2H, m), 2.42 (1H, m), 2.21 (3H, s); ¹³C NMR (100 MHz, CDCl₃, TMS) δ 210.3, 172.2, 157.3(COCF₃), 138.5, 138.2, 137.2, 129.1, 128.4, 128.3, 128.3(2C), 127.8(2C), 127.6, 126.2, 115.9(COCF₃), 101.0(2C), 94.5, 79.6, 75.5, 74.6, 74.0, 73.0, 70.6, 70.3, 69.2, 69.1, 66.5, 64.7, 56.3, 50.8, 38.1, 30.0, 27.7. ESI-Q-TOF (positive mode) calculated for C₄₁H₅₀F₃NO₁₃⁺ [M+NH₄]⁺ m/z 835.3840, found 835.3956.

### Synthesis of methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-6-acety lpropionyl-α-D-galactopyranosyl-(1→3)-2-deoxy-2-trifluoroacetylamino-4,6-O-benzyliden e-β-D-galactopyranoside

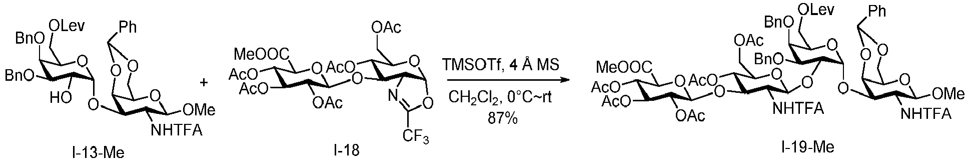

Methyl 2,3,4-tri-O-acetyl-β-d-glucopyranuronate-(1→3)-4,6-di-O-acetyl-2-deoxy-2-trifluoroacetamido-D-glucopyranose [2, 1, -d] 2-oxazoline (1.50 g, 2.28 mmol, 1.4 equiv.) and methyl 3,4-di-O-benzyl-6-acetylpropionyl-α-D-galactopyranosyl-(1→3)-2-deoxy-2-trifluoroacetamido-4,6-O-benzylidene-β-D-galactopyranoside (1.35 g, 1.65 mmol) were dissolved in anhydrous dichloromethane (30.0 mL), and 4Å molecular sieve (3.00 g) was added, stirred at room temperature for 2 hours, and then cooled to -20 °C. Trimethylsilyl trifluoromethylsulfonate (81 µL, 0.47 mmol, 0.3 equiv.) was added, and the reaction was carried out with stirring at -20°C for 2h, and then the temperature was slowly raised to room temperature and the temperature was carried out overnight. After the reaction was completed as monitored by TLC, the reaction was quenched by adding proper amount of triethylamine dropwise, the molecular sieve was removed by diatomite filtration. The filtrate was concentrated under reduced pressure, and purified by column chromatography (petroleum ether/acetone 2: 1) to obtain a white solid (2.11 g, 87%). R*_{f}* = 0.43 (petroleum ether/acetone 1: 1); ¹H NMR (400 MHz, CDCl₃, TMS) δ 8.47 (1H, d, *J* = 6.6 Hz), 7.98 (1H, d, *J* = 9.4 Hz), 7.66-7.55 (2H, m), 7.51-7.39 (3H, m), 7.38-7.23 (8H, m), 7.19-7.06 (2H, m), 5.60 (1H, s), 5.16 (1H, t, *J* = 9.6 Hz), 5.12-5.03 (2H, m), 5.01-4.91 (2H, m), 4.79 (1H, d, *J* = 11.6 Hz), 4.72-4.60 (3H, m), 4.58 (1H, d, *J* = 8.5 Hz), 4.48-4.33 (5H, m), 4.27 (2H, dd, *J* = 11.9, 6.4 Hz), 4.20 (1H, dd, *J* = 11.9, 2.1 Hz), 4.17-4.06 (2H, m), 3.95 (1H, d, *J* = 9.9 Hz), 3.92-3.86 (2H, m), 3.83 (1H, dd, *J* = 8.1, 3.8 Hz), 3.71 (3H, s), 3.67 (1H, d, *J* = 2.7 Hz), 3.65-3.56 (2H, m), 3.51 (3H, s), 3.48 (2H, b), 3.01 (1H, ddd, *J* = 19.0, 11.2, 3.3 Hz), 2.62-2.55 (2H, m), 2.46 (1H, q, *J* = 8.6, 8.1 Hz), 2.33 (1H, ddd, *J* = 17.0, 5.5, 3.3 Hz), 2.22 (3H, s), 2.10 (3H, s), 2.02 (3H, s), 2.02 (3H, s), 2.00 (6H, s); ¹³C NMR (100 MHz, CDCl₃, TMS) δ 210.9, 172.4, 170.8, 169.9(2C), 169.5, 169.4, 167.0, 157.9 (COCF₃), 157.8 (COCF₃), 138.1, 137.9, 137.1, 130.5, 128.7, 128.6, 128.3, 128.2, 128.0, 127.8, 127.8, 126.4, 117.4 (COCF₃), 115.4 (COCF₃), 101.8, 100.9, 100.3, 98.5, 93.5, 77.8, 76.2, 75.8, 74.8, 73.7, 73.5, 72.7, 72.4, 71.9, 70.9, 70.0, 69.8, 69.4, 69.3, 68.7, 66.5, 64.9, 62.4, 58.5, 56.3, 52.6, 51.0, 38.0, 29.9, 27.7, 20.6 (2C), 20.4 (2C); ESI-Q-TOF (positive mode) calculated for C₆₆H₇₆F₆N₂O₂₉⁺ [M+NH₄]⁺ m/z 1492.4782, found 1492.4733.

### Synthesis of methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-gal actopyranosyl-(1→3)-2-deoxy-2-trifluoroacetylamino-4,6-O-benzylidene-β-D-galactopyra noside

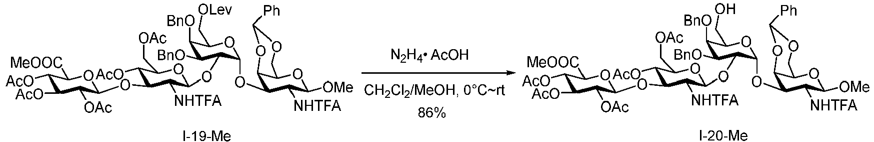

Methyl 2,3,4-tri-O-acetyl-β-D-glucopyranuronate-(1→3)-4,6-di-O-acetyl-2-deoxy-2-trifluoroacetylamino-β-D-glucopyranosu;-(1→2)-3,4-di-O-benzyl-6-acetylpropionyl-α-D-gal actopyranosyl-(1→3)-2-deoxy-2-trifluoroacetylamino-4,6-O-benzylidene-β-D-galactopyranosid e (1.51 g, 1.02 mmol) was dissolved in dichloromethane/methanol (10.0 mL/2.0 mL), hydrazine acetate (182 mg, 2.02 mmol, 2.0 equiv.) was added in ice bath conditions, and the temperature was slowly raised to room temperature and the reaction was carried out overnight. After the reaction was completed as monitored by TLC, the reaction solution was diluted with dichloromethane, and the organic phase was washed with 1M hydrochloric acid solution, saturated sodium bicarbonate solution and saturated salt water in turn, dried with anhydrous sodium sulfate, concentrated under reduced pressure, and purified by column chromatography (DCM/MeOH=50: 1) to obtain a white solid (1.20 g, 86%). R*_{f}* = 0.46 (DCM/MeOH 20: 1); ¹H NMR (400 MHz, CDCl₃, TMS) δ 8.59 (1H, b), 8.16 (1H, d, *J* = 9.7 Hz), 7.58-7.49 (2H, m), 7.45-7.37 (3H, m), 7.35-7.22 (9H, m), 7.19-7.11 (2H, m), 5.50 (1H, s), 5.21-4.99 (3H, m), 4.97-4.86 (2H, m), 4.77 (1H, d, *J* = 11.1 Hz), 4.66 (1H, d, *J* = 12.1 Hz), 4.59-4.46 (2H, m), 4.45-4.25 (5H, m), 4.26-4.14 (2H, m), 4.11 (1H, s), 4.05 (1H, dd, *J* = 10.1, 3.2 Hz), 3.94 (1H, d, *J* = 9.7 Hz), 3.92-3.82 (3H,m), 3.78-3.66 (6H,m), 3.56 (2H, s), 3.52-3.46 (5H, m), 3.24 (1H, s), 2.08 (3H, s), 2.04 (3H, s), 1.99 (6H, s), 1.93 (3H, s); ¹³C NMR (100 MHz, CDCl₃, TMS) δ 171.1, 169.9, 169.8, 169.7, 169.4, 167.0, 158.1 (COCF₃), 157.8 (COCF₃) 138.0, 137.9, 137.4, 130.0, 128.7, 128.6, 128.4, 128.0, 128.0, 126.2, 117.0 (COCF₃), 114.3 (COCF₃), 100.9, 100.7, 100.3(2C), 99.5, 77.9, 74.8, 73.5, 72.5, 72.0, 70.8, 69.4, 68.5, 66.3, 62.4, 57.7, 56.6, 52.7, 20.8, 20.6, 20.5, 20.4, 20.2.

### Synthesis of methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopy ranuronic acid-(1→3)-2-deoxy-2-trifluoroacetylamino-4,6-O-benzylidene-β-D-galactopyranoside

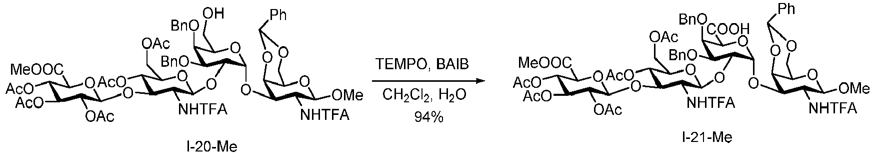

Methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyranosyl-(1→3) -2-deoxy-2-trifluoroacetylamino-4,6-O-benzylidene-β-D-galactopyranoside (950 mg, 0.69 mmol) was dissolved in dichloromethane/water (16.0 mL/8.0 mL). 2,2,6,6-tetramethylpiperidine-nitrogen-oxide (44 mg, 0.28 mmol, 0.4 equiv.) and diacetoxy iodobenzene (440 mg, 1.36 mmol, 2.0 equiv.) were added in turn under ice bath conditions, and the temperature was naturally raised to room temperature and the reaction was carried out overnight. After the reaction was completed as monitored by TLC, the reaction solution was diluted with dichloromethane, the organic phase was washed with saturated sodium thiosulfate solution, dried with anhydrous sodium sulfate, concentrated under reduced pressure, and purified by column chromatography (DCM/MeOH=50: 1) to obtain a light yellow solid (902 mg, 94%). R*_{f}* = 0.18 (DCM/MeOH 30:1); ¹H NMR (400 MHz, DMSO-*d*₆, TMS) δ 7.56 (2H, d, *J* = 7.4 Hz), 7.48-7.09 (15H, m), 5.72 (1H, s), 5.29 (1H, s), 5.19 (1H, t, *J* = 9.3 Hz), 4.92 (1H, t, *J* = 9.7 Hz), 4.82-4.61 (6H, m), 4.61-4.43 (3H, m), 4.41-4.29 (4H, m), 4.20 (1H, d, *J* = 12.1 Hz), 4.16-4.07 (3H, m), 4.00 (2H, d*, J* = 11.7 Hz), 3.90 (5H, s), 3.80-3.70 (1H, m), 3.66 (3H, s), 3.58 (1H, s), 3.38 (3H, s), 2.13-1.92 (12H, m), 1.88 (3H, s); ¹³C NMR (100 MHz, DMSO-*d*₆, TMS) δ 170.6, 169.9, 169.8, 169.7, 169.6, 167.6, 156.9(COCF₃), 156.6(COCF₃) 139.0, 138.7, 138.6, 129.0, 128.7, 128.5, 128.4, 128.1, 128.0, 127.9, 126.4, 117.6 (COCF₃), 114.8 (COCF₃), 101.7, 101.1 (2C), 100.0, 75.6, 75.4, 73.1, 72.0, 71.8, 71.3, 70.6, 69.8, 69.0, 66.5, 62.4, 56.5, 53.1, 21.1, 20.9, 20.7, 20.6, 20.4; ESI-Q-TOF (positive mode) calculated for C₆₁H₆₈F₆N₂O₂₈⁺ [M+NH₄]⁺ m/z 1408.4207, found 1408.4362.

### Synthesis of Methyl β-D-glucopyranuronic acid-(1→3)-2-deoxy-2-acetylamino-β-D-glucopyranosyl-(1→2)-3,4-di-O-benzyl-α-D-galactopyranoic acid-(1→3)-2-deoxy-2-acetylamino-4,6-O-benzylidene-β-D-galactopyranoside

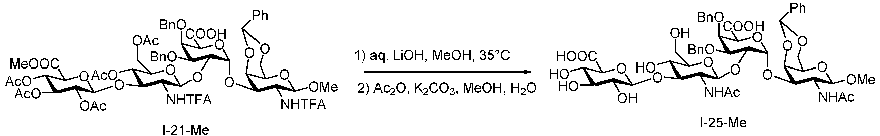

Methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyranuronic acid-(1→3)-2-deoxy-2-trifluoroacetylamino-4,6-O-benzylidene-β-D-galactopyranoside (468mg, 0.34 mmol) was dissolved in methanol (8.0 mL), and saturated lithium hydroxide solution (8.0 mL) was added dropwise, and the temperature was raised to 35°C and the reaction was carried out for 48 h. After the reaction was completed as monitored by TLC, the reaction solution was neutralized with IR-120 cation exchange resin to pH=7, filtered, the resin was washed with methanol. The filtrate was concentrated under reduced pressure, the concentrated crude product was dissolved in a mixed solution of methanol/water (8.0 mL/8. 0 mL), potassium carbonate solid was added to adjust pH=11-12, acetic anhydride (2.4 mL) was added dropwise, and potassium carbonate solid was added to adjust pH=11-12, and the reaction was carried out overnight at room temperature. After the reaction was completed as monitored by TLC, the reaction solution was neutralized with IR-120 cation exchange resin to pH=7, filtered, the resin was washed with methanol, the filtrate was concentrated under reduced pressure, and purified by Sephadex LH-20 with CH₂Cl₂/MeOH 1:1 as eluent, to obtain a white solid (containing salt, directly used for the next step). R*_{f}* = 0.70 (CHCl₃/MeOH/H₂O/acetone=4:3:1:2); ¹H NMR (600 MHz, CD₃OD, TMS) δ 7.67 (2H, d, *J* = 7.7 Hz), 7.47 (2H, t, *J* = 7.6 Hz), 7.40 (1H, t, *J* = 7.5 Hz), 7.36-7.16 (10H, m), 5.84 (1H, s), 5.48 (1H, b), 4.78-4.75 (1H, m), 4.73-4.68 (1H, m), 4.69-4.60 (2H, m), 4.56 (1H, d, *J* = 12.1 Hz), 4.53-4.44 (2H, m), 4.44-4.34 (2H, m), 4.24 (2H, s), 4.10-4.00 (3H, m), 3.96-3.82 (2H, m), 3.72-3.65 (3H, m), 3.64-3.55 (3H, m), 3.53-3.40 (4H, m), 3.31 (4H, s), 1.95 (3H, s), 1.64 (3H, s); ¹³C NMR (100 MHz, CD₃OD, TMS) δ 172.9, 172.8 (2C), 138.6, 137.7, 129.4, 128.7, 128.1, 127.8, 127.7, 127.2, 127.1, 126.9, 126.3, 104.4, 103.7, 101.8, 101.1, 77.3, 76.6, 76.2, 75.4, 74.8, 74.2, 73.5, 72.0 (2C), 71.5, 69.0, 68.9, 66.6, 55.8, 54.5, 50.1, 22.8, 21.8; ESI-Q-TOF (positive mode) calculated for C₅₀H₆₂N₂O₂₃⁺ [M+NH₄]⁺ m/z 1076.4087, found 1076.4073.

### Synthesis of methyl β-D-glucopyranuronic acid-(1→3)-2-deoxy-2-acetylamino-β-D-glucopyranosyl-(1→2)-α-D-galactopyranoic acid-(1→3)-2-deoxy-2-acetylamino-α, β-D-galactopyranoside

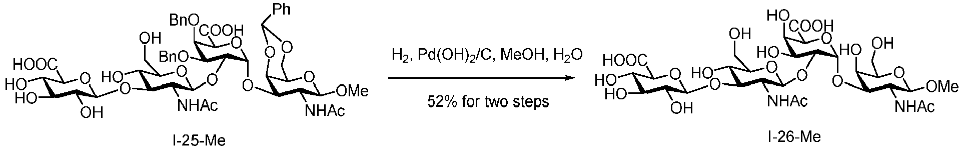

Benzyl β-D-glucopyranuronic acid-(1→3)-2-deoxy-2-acetylamino-β-D-glucopyranosyl -(1→2)-3,4-di-O-benzyl-α-D-galactopyranoic acid-(1→3)-2-deoxy-2-acetylamino-4,6-O-benzylidene-β-D-galactopyranoside (about 0.34 mmol in all of the previous step) was dissolved in a mixed solution of methanol/water (5.0 mL/5. 0 ml), 20% palladium hydroxide carbon (480 mg) was added, and the reaction was carried out at room temperature for 48 hours at 40Pa hydrogen pressure. After the reaction was completed as monitored by TLC, it was filtered, concentrated under reduced pressure, and purified by Sephadex LH-20 with pure water as eluent, to obtain a white solid (95 mg, 52% for both steps). Rf =0.11 (CHCl₃/MeOH/H₂O =1:1:0.3); ¹H NMR (400 MHz, D₂O) δ 5.29 (1H, s), 4.64 (1H, d, *J* = 8.5 Hz), 4.48 (1H, d, *J* = 8.0 Hz), 4.41 (1H, d, *J* = 8.7 Hz), 4.34-4.11 (3H, m), 3.98-3.69 (11H, m), 3.69-3.60 (1H, m), 3.60-3.42 (7H, m), 3.39-3.22 (1H, m), 1.98 (3H, s), 1.94 (3H, s); ¹³C NMR (100 MHz, D₂O) δ 176.2, 103.1, 103.0, 102.2, 96.7, 82.6, 77.8, 75.5, 75.3, 74.7, 72.8, 72.0, 71.7, 71.1, 68.7, 68.4, 64.7, 61.0, 60.8, 57.0, 54.7, 50.7, 22.3, 22.1; ESI-Q-TOF (negative mode) calculated for C₂₉H₄₆N₂O₂₃⁻[M-H⁺]⁻m/z 789.2413, found 789.2446.

### Example 4

### Synthesis of ethyl 2-deoxy-2-trifluoroacetamido-3,4,6-tri-O-acetyl-β-D-galactopyranoside

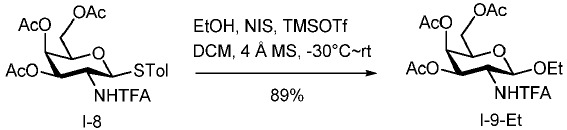

P-tolyl 2-deoxy-2-trifluoroacetamido-3,4,6-tri-O-acetyl-1-thio-β-D-galactopyranoside (2.0 g, 3.94 mmol), N-iodosuccinimide (1.24 g, 5.51 mmol, 1.4 equiv) and 4Å molecular sieve were dissolved in anhydrous dichloromethane (30 mL) under the protection of argon, and ethanol ( 0.69 mL, 11.8 mmol, 3.0 equiv. ) was added, stirred at room temperature for 2 hours, then cooled to -30°C, and trimethylsilyl trifluoromethylsulfonate ( 0.27 mL, 1.55 mmol, 0.40 equiv. ) was added dropwise, and the reaction was carried out with continuously stirring for 3h, and then moved to room temperature and the reaction was carried out overnight. After the reaction was completed as monitored by TLC, the reaction was quenched by adding proper amount of trimethylamine. The molecular sieve was filtered out by diatomite, and the filter cake was washed several times by dichloromethane. Filtrates were combined, concentrated under reduced pressure, and purified by column chromatography (petroleum ether/EtOAc 3: 1) to obtain a white solid (1.51 g, 89%). R*_{f}* = 0.45 (petroleum ether/EtOAc 1: 1); ¹H NMR (400 MHz, CDCl₃, TMS) δ 7.21 (1H, d, *J* = 9.0 Hz), 5.37 (1H, d, *J* = 3.4), 5.33 - 5.18 (2H, m), 4.66 (1H, d, *J* = 8.3 Hz,), 4.23-4.07 (2H, m), 4.00-3.94 (1H, m), 3.94-3.86 (1H, m), 3.62-3.52 (1H, m), 2.14 (3H, s), 2.03 (3H, s), 1.97 (3H, s), 1.18 (3H, t, *J* = 7.1 Hz); ¹³C NMR (100 MHz, CDCl₃, TMS) δ 170.7, 170.6, 170.3, 157.8 (COCF₃), 115.7 (COCF₃), 100.2, 70.7, 69.6, 66.7, 65.6, 61.6, 51.8, 20.6, 20.6, 20.4, 14.8.

### Synthesis of ethyl 2-deoxy-2-trifluoroacetamido-β-D-galactopyranoside

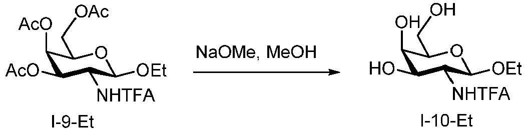

Ethyl 2-deoxy-2-trifluoroacetamido-3,4,6-tri-O-acetyl-β-D-galactopyranoside (1.51 g, 3.52 mmol) was dissolved in methanol (50.0 mL), and proper amount of sodium methoxide was added to adjust pH to 9-10. The reaction was carried out with stirring at room temperature for 2 hours. After the reaction was completed as monitored by TLC, cationic resin was added to the reaction solution, and the solution was neutralized to pH = 7, filtered, and the filtrate was concentrated under reduced pressure to dryness to obtain a yellow syrup, R*_{f}* = 0.57 (DCM/MeOH 5: 1, which was directly used for the next reaction.

### Synthesis of ethyl 2-deoxy-2-trifluoroacetamido-4,6-O-benzylidene-β-D-galactopyranoside

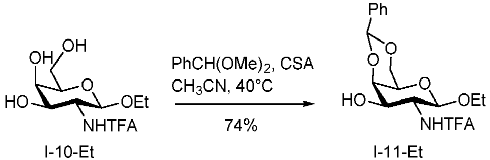

Ethyl 2-deoxy-2-trifluoroacetamido-β-D-galactopyranoside (1.07 g, 3.52 mmol) and (+)-camphor sulfonic acid (1.90 g, 8.18 mmol, 2.3 equiv.) were dissolved in anhydrous acetonitrile (30.0 mL) under the protection of argon. Benzaldehyde dimethyl acetal (2.50 mL, 16.6 mmol, 4.6 equiv.) was added, and the temperature was raised to 40 °C and the reaction was carried out overnight. After the reaction was completed as monitored by TLC, the reaction was quenched by adding proper amount of triethylamine dropwise. The reaction solution was concentrated under reduced pressure, and then purified by column chromatography (petroleum ether/EtOAc = 2: 1) to obtain a white solid (1.01 g, 74%). R*_{f}* = 0.68 (petroleum ether/acetone = 1: 2).

### Ethyl 2-O-p-methoxybenzyl-3,4-di-O-benzyl-6-acetylpropionyl-α-D- galactopyranosyl -(1 → 3)-2-deoxy-2-trifluoroacetamido-4,6-O-benzylidene-β-D- galactopyranoside

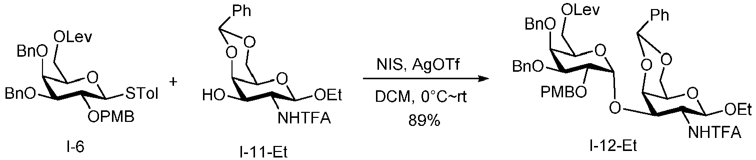

P-tolyl-2-O-p-methoxybenzyl-3,4-di-O-benzyl-6-acetylpropionyl-1-thio-β-D-galactopyran oside (2.21 g, 3.23 mmol, 1.4 equiv.) and ethyl 2-deoxy-2-trifluoroacetamido-4,6-O-benzylidene-β-D-galactopyranoside (0.90 g, 2.30 mmol) were dissolved in anhydrous dichloromethane/ N,N-dimethylformamide (30.0 mL/6.0 mL) under the protection of argon. 4Å molecular sieve (3.6 g) was added, stirred at room temperature for 2 hours and then cooled to 0 °C. N-iodosuccinimide (0.90 g, 4.00 mmol, 1.7 equiv.)and silver trifluoromethanesulfonate (193 mg, 0.75 mmol, 0.3 equiv.) were added in turn, and the reaction was carried out with stirring at 0 °C for 2 hours, then the temperature was slowly raised to room temperature and the reaction was carried out overnight. After the reaction was completed as monitored by TLC, the reaction was quenched by adding proper amount of triethylamine dropwise. The molecular sieve was removed by diatomite filtration, and the filtrate was concentrated under reduced pressure, and purified by column chromatography (petroleum ether/EtOAc 1: 1) to obtain compound I-12 (1.96 g, 89%) as a white solid. R*_{f}* = 0.74 (DCM/MeOH 20:1); ¹H NMR (400 MHz, CDCl₃, TMS) δ 7.58-7.47 (3H, m), 7.28 (13H, m), 6.92 (2H, d, *J* = 8.6 Hz), 6.62 (2H, d, *J* = 8.6 Hz), 5.53 (1H, s), 5.23 (1H, d, *J* = 3.5 Hz), 4.96 (1H, d, *J* = 11.5 Hz), 4.84-4.76 (2H, m), 4.65 (1H, d, *J* = 11.7 Hz), 4.54 (1H, d, *J* = 11.5 Hz), 4.48-4.38 (3H, m), 4.36 - 4.27 (2H, m), 4.22 (1H, dd, *J* = 11.7, 3.2 Hz), 4.15 (1H, dd, *J* = 10.9, 3.4 Hz), 4.11-4.00 (3H, m), 3.94 (1H, dd, *J* = 9.7, 7.1 Hz), 3.87 (1H, dd, *J* = 10.0, 2.7 Hz), 3.85-3.80 (1H, b), 3.79-3.71 (4H, m), 3.61-3.51 (1H, m), 3.46 (1H, s), 2.94-2.82 (1H, m), 2.72-2.51 (2H, m), 2.46-2.37 (1H, m), 2.17 (3H, s), 1.17 (3H, t, *J* = 7.0 Hz); ¹³C NMR (100 MHz, CDCl₃, TMS) δ 209.2, 172.5, 158.9, 157.2 (COCF3), 138.7, 138.3, 137.6, 130.6, 129.3, 129.1, 128.4, 128.3 (2C), 128.2, 127.8, 127.7, 127.5, 126.5,116.0 (COCF3), 113.5, 101.2, 99.6, 92.7, 78.2, 75.6, 74.9, 74.6, 73.7, 71.5, 71.3, 70.5, 70.2, 69.5, 66.5, 64.8, 64.6, 55.3, 52.4, 38.0, 29.8, 27.8, 15.0; ESI-Q-TOF (positive mode) calculated for C₅₀H₅₆F₃NO₁₄⁺ [M+NH₄]⁺ m/z 969.3997, found 969.4011.

### Ethyl 3,4-di-O-benzyl-6-acetylpropionyl-α-D-galactopyranosyl-(1→3)-2-deoxy-2-trifluoroacetamido-4,6-O-benzylidene-β-D-galactopyranoside

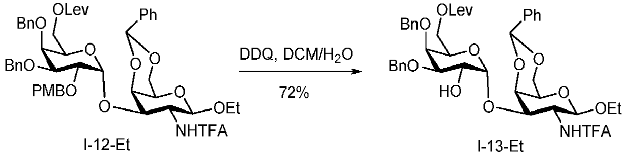

Ethyl 2-O-p-methoxybenzyl-3,4-di-O-benzyl-6-acetylpropionyl-β-D-galactopyranosyl-(1→3)-2-deoxy-2-trifluoroacetamido-4,6-O-benzylene-β-D-galactopyranoside (140 mg, 0.15 mmol) was dissolved in dichloromethane/water (2.90 mL/0. 29 mL), and 2,3-dichloro-5,6-dicyano-p-benzoquinone (44 mg, 0.22 mmol, 1.5 equiv.) was added in batches, and the reaction was carried out for 1 hour at room temperature. After the reaction was completed as monitored by TLC, the reaction solution was diluted with dichloromethane, and the organic phase was washed with saturated sodium bicarbonate solution, saturated sodium thiosulfate solution and saturated salt water in turn, dried with anhydrous sodium sulfate, filtered, concentrated under reduced pressure, and purified by column chromatography (DCM/MeOH 40: 1) to obtain a white solid (88 mg, 72%). R*_{f}* = 0.69 (petroleum ether/acetone 1: 1); ¹H NMR (400 MHz, CDCl₃, TMS) δ 7.67 (1H, d, *J* = 9.4 Hz), 7.58-7.50 (2H, m), 7.44-7.21 (13H, m), 5.58 (1H, s), 5.18 (1H, d, *J* = 3.9 Hz), 4.93 (1H, d, *J* = 11.4 Hz), 4.86 (1H, d, *J* = 11.8 Hz), 4.69-4.58 (2H, m), 4.50 (1H, d, *J* = 11.4 Hz), 4.48-4.31 (3H, m), 4.19 (1H, dd, *J* = 11.8, 2.9 Hz), 4.17-4.00 (4H, m), 3.92 (1H, dd, *J* = 9.7, 7.0 Hz), 3.83-3.77 (1H, m), 3.75-3.70 (1H, m), 3.59 (1H, dd, *J* = 10.0, 2.8 Hz), 3.53 (1H, dd, *J* = 9.7, 7.0 Hz), 3.44 (1H, s), 2.96-2.85 (1H, m), 2.76-2.35 (3H, m), 2.20 (3H, s), 1.17 (3H, t, *J* = 7.0 Hz); ¹³C NMR (100 MHz, CDCl₃, TMS) δ 210.2, 172.2, 157.3(COCF₃), 138.5, 138.2, 137.3, 129.0, 128.4, 128.3 (2C), 127.8, 127.6, 126.2, 115.9 (COCF₃), 100.9, 100.0, 94.5, 79.7, 75.4, 74.6, 73.9, 73.1, 70.6, 70.4, 69.3, 69.1, 66.4, 64.7, 64.6, 51.1, 38.1, 29.9, 27.7, 15.0.

### Synthesis of ethyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-6-ac etylpropionyl-α-D-galactopyranosyl-(1→3)-2-deoxy-2-trifluoroacetylamino-4,6-O-benzyli dene-β-D-galactopyranosyl-(1→3)-2-deoxy-2-trifluoroacetylamino-4,6-O-benzylidene-β-D -galactopyranoside

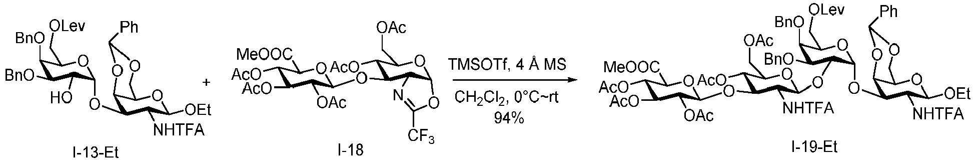

Methyl 2,3,4-tri-O-acetyl-β-D-glucopyranuronate-(1→3)-4,6-di-O-acetyl-2-deoxy-2-trifluoroacetamido-D-glucopyranose [2, 1,-d] 2-oxazoline (400 mg, 0.61 mmol, 1.4 equiv.) and ethyl 3,4-di-O-benzyl-6-acetylpropionyl-α-D-galactopyranosyl-(1→3)-2-deoxy-2- trifluoro acetamido-4,6-O-benzylidene-β-D-galactopyranoside (360 mg, 0.43 mmol) were dissolved in anhydrous dichloromethane (8.0 mL). 4Å molecular sieve (800 mg) was added, and the reaction was carried out with stirring at room temperature for 2h, and then cooled to-20 °C. Trimethylsilyl trifluoromethylsulfonate (21.6 µL, 0.13 mmol, 0.3 equiv.) was added, and the reaction was carried out with stirring at-20 °C for 2h, and then the temperature was slowly raised to room temperature and the reaction was carried out overnight. After the reaction was completed as monitored by TLC, the reaction was quenched by adding proper amount of triethylamine dropwise. The molecular sieve was removed by diatomite filtration, and the filtrate was concentrated under reduced pressure, and purified by column chromatography (petroleum ether/acetone 2: 1) to obtain a white solid (664 mg, 94%). R*_{f}* = 0.45 (petroleum ether/acetone 1: 1); ¹H NMR (400 MHz, CDCl₃, TMS) δ 8.52 (1H, d, *J* = 6.6 Hz), 8.00 (1H, d, *J* = 9.3 Hz), 7.67-7.50 (2H, m), 7.53-7.39 (3H, m), 7.39-7.21 (8H, m), 7.14 (2H, dd, *J* = 7.7, 1.8 Hz), 5.59 (1H, s), 5.16 (1H, t, *J* = 9.6 Hz), 5.11-5.02 (2H, m), 5.00-4.93 (2H, m), 4.79 (1H, d, *J* = 11.6 Hz), 4.74-4.57 (4H, m), 4.44 (1H, d, *J* = 3.6 Hz), 4.43-4.36 (3H, m), 4.34 (1H, d, *J* = 12.0 Hz), 4.27 (2H, dd, *J* = 11.8, 6.1 Hz), 4.20 (1H, d, *J* = 11.9), 4.16-4.08 (2H, m), 3.99-3.92 (2H, m), 3.89 (2H, dt, *J* = 12.3, 2.7 Hz), 3.81 (1H, dd, *J=* 11.8, 8.5 Hz), 3.71 (3H, s), 3.68 (1H, d, *J* = 2.8 Hz), 3.65-3.56 (2H, m), 3.53 (1H, dd, *J* = 9.6, 7.1 Hz), 3.46 (2H, b), 3.07-2.95 (1H, m), 2.69-2.55 (2H, m),2.46 (1H, dd, *J* = 17.2, 8.5 Hz), 2.33 (1H, dt, *J* = 16.9, 4.1 Hz), 2.21 (3H, s), 2.10 (3H, s), 2.02 (3H, s), 2.01 (3H, s), 1.99 (6H, s), 1.20 (3H, t, *J* = 7.0 Hz); ¹³C NMR (100 MHz, CDCl₃, TMS) δ 210.8, 172.4, 170.7, 169.9 (2C), 169.5, 169.4, 167.0, 157.9 (COCF₃), 157.6 (COCF₃), 138.1, 137.9, 137.2, 130.4, 128.7, 128.6, 128.3, 128.2, 128.0, 127.8, 127.8, 126.4, 117.4 (COCF₃), 116.9 (COCF₃), 101.8, 100.3, 99.9, 98.5, 93.5, 77.9, 76.2, 75.8, 74.7, 73.7, 73.4, 72.7, 72.5, 72.4, 71.9, 70.9, 70.1, 69.8, 69.4, 69.3, 68.7, 66.5, 64.8, 62.4, 58.5, 52.6, 51.3, 38.0, 29.8, 27.6, 20.8, 20.6, 20.5, 20.4 (2C), 15.0; ESI-Q-TOF (positive mode) calculated for C₆₇H₇₈F₆N₂O₂₉⁺ [M+NH₄]⁺ m/z 1506.4938, found 1506.4886.

### Synthesis of ethyl methyl 2,3,4-tri-O-acetyl-β-D-glucopyranuronate-(1→3)-4,6-di -O-acetyl-2-deoxy-2-trifluoroacetylamino-β-D-glucopyranosyl-(1→2)-3,4-di-O-benzyl-α-D -galactopyranosyl-(1→3)-2-deoxy-2-trifluoroacetylamino-4,6-O-benzylidene-β-D-galactop yranoside

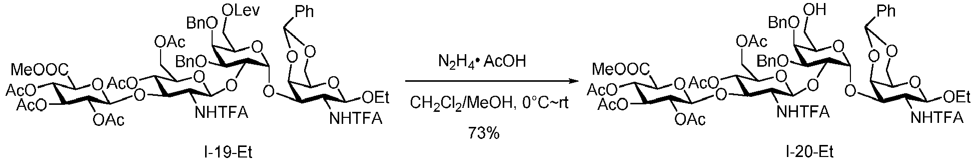

Ethyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-6-acetylpropionyl-α-D-gal actopyranosyl-(1→3)-2-deoxy-2-trifluoroacetylamino-4,6-O-benzylidene-β-D-galactopyranosid e (660 mg, 0.44 mmol) was dissolved in dichloromethane/methanol (5.0 mL/1.0 ml), hydrazine acetate (200 mg, 2.22 mmol, 5.0 equiv.) was added under ice bath conditions, and the temperatue was slowly raised to room temperature and the reaction was carried out overnight. After the reaction was completed as monitored by TLC, the reaction solution was diluted with dichloromethane, and the organic phase was washed with 1M hydrochloric acid solution, saturated sodium bicarbonate solution and saturated salt water in turn, dried with anhydrous sodium sulfate, concentrated under reduced pressure, and purified by column chromatography (DCM/MeOH = 50: 1) to obtain a white solid (450 mg, 73%). R*_{f}* = 0.52 (DCM/MeOH = 20:1); ¹H NMR (400 MHz, CDCl₃, TMS) δ 8.33 (1H, d, *J* = 7.5 Hz), 8.04 (1H, d, *J* = 9.1 Hz), 7.56 (2H, dd, *J* = 7.5, 2.1 Hz), 7.46-7.38 (3H, m), 7.34-7.24 (9H, m), 7.17 (2H, dd, *J* = 7.3, 2.3 Hz), 5.54 (1H, s), 5.23-5.00 (3H, m), 4.93 (1H, t, *J* = 8.5 Hz), 4.88 (1H, d, *J=* 8.3 Hz), 4.75 (1H, d, *J* = 11.3 Hz), 4.66 (1H, s), 4.63 (1H, d, *J* = 4.7 Hz), 4.58 (1H, t, *J* = 9.5 Hz), 4.50-4.29 (5H, m), 4.26-4.14 (3H, m), 4.06 (1H, dd, *J* = 10.2, 3.4 Hz), 4.00 (1H, d, *J* = 11.9 Hz), 3.97-3.85 (4H, m), 3.76 (1H, d, *J* = 2.7 Hz), 3.71 (3H, s), 3.67 (2H, s), 3.62-3.53 (2H, m), 3.50 (1H, d, *J* = 2.8 Hz), 3.45-3.37 (1H, m), 3.36 (1H, s), 3.02-2.91 (1H, m), 2.10 (3H, s), 2.04 (3H, s), 1.99 (6H, d, *J* = 0.9 Hz), 1.96 (3H, s), 1.16 (3H, t, *J* = 7.0 Hz); ¹³C NMR (100 MHz, CDCl₃, TMS) δ 171.1, 170.0, 169.9, 169.6, 169.4, 167.0, 158.2 (COCF₃), 157.8 (COCF₃), 138.0, 137.8, 137.4, 130.0, 128.7, 128.6, 128.4, 128.0, 127.9, 126.3, 117.2 (COCF₃), 114.3 (COCF₃), 101.2, 100.3, 99.6, 99.4, 95.3, 77.7, 76.2, 76.1, 74.8, 73.4, 72.5, 72.4, 72.1, 71.3, 71.0, 70.9, 69.4, 69.3, 68.5, 66.4, 64.7, 62.7, 62.4, 57.5, 52.7, 52.1, 20.9, 20.6, 20.5, 20.4, 20.3, 14.8.

### Synthesis of methyl ethyl 2,3,4-tri-O-acetyl-β-D-glucopyranuronate-(1→3)-4,6-di-O -acetyl-2-deoxy-2-trifluoroacetylamino-β-D-glucopyranosyl-(1→2)-3,4-di-O-benzyl-α-D-ga lactopyranuronic acid-(1→3)-2-deoxy-2-trifluoroacetylamino-4,6-O-benzylidene-β-D-galactopyranoside

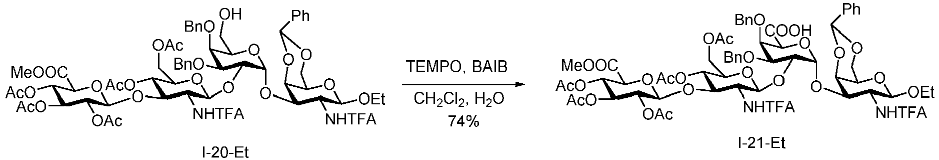

Methyl ethyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyranosyl-(1→ 3)-2-deoxy-2-trifluoroacetylamino-4,6-O-benzylidene-β-D-galactopyranoside (430 mg, 0.31 mmol) was dissolved in dichloromethane/water (8.0 mL/4. 0 ml). 2,2,6,6-tetramethylpiperidine-nitrogen-oxide (20 mg, 0.13 mmol, 0.4 equiv.) and diacetoxy iodobenzene (200 mg, 0.62 mmol, 2.0 equiv.) were added in turn under ice bath conditions, and the temperature was naturally raised to room temperature and the reaction was carried out overnight. After the reaction was completed as monitored by TLC, the reaction solution was diluted with dichloromethane, the organic phase was washed with saturated sodium thiosulfate solution, dried with anhydrous sodium sulfate, concentrated under reduced pressure, and purified by column chromatography (DCM/MeOH = 50: 1) to obtain a light yellow solid (320 mg, 74%). R*_{f}* = 0.19 (DCM/MeOH = 30:1); ¹H NMR (400 MHz, DMSO-*d*₆, TMS) δ7.56 (2H, d, *J* = 7.4 Hz), 7.45-7.10 (15H, m), 5.70 (1H, s), 5.28 (1H, s), 5.19 (1H, t, *J* = 9.3 Hz), 4.91 (1H, t, *J* = 9.7 Hz), 4.83-4.49 (7H, m), 4.49-4.26 (6H, m), 4.25-3.69 (13H, m), 3.65 (3H, s), 3.57 (2H, s), 2.01-1.95 (9H, m), 1.94 (3H, s), 1.88 (3H, s), 1.07 (3H, b); ¹³C NMR (100 MHz, DMSO-*d*₆, TMS) δ 170.6, 169.9, 169.8, 169.7 (2C), 167.6, 157.0 (COCF₃), 156.7 (COCF₃), 139.5, 138.9 (2C), 138.8 (2C), 128.7, 128.4, 128.1, 128.0, 127.9, 126.4, 117.7 (COCF₃), 114.8 (COCF₃), 100.1 (2C), 100.0 (2C), 75.4, 72.0, 71.8, 71.2, 70.7, 69.8, 69.1(2C), 68.0, 66.4, 64.7, 62.4, 53.1, 20.9, 20.8, 20.7, 20.6, 20.5, 15.4; ESI-Q-TOF (positive mode) calculated for C₆₆H₇₆F₆N₂O₂₉⁺ [M+NH₄]⁺ m/z 1422.4363, found 1422.4349.

### Synthesis of ethyl β-D-glucopyranuronic acid-(1→3)-2-deoxy-2-acetylamino -β-D-glucopyranosyl-(1→2)-α-D-galactopyranoic acid (1→3)-2-deoxy-2-acetylamino-α,β-D-galactopyranoside

CP-Et was obtained from I-21-Et in two steps using the same method as in Example 3.

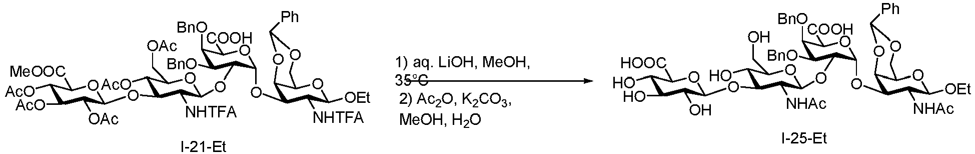

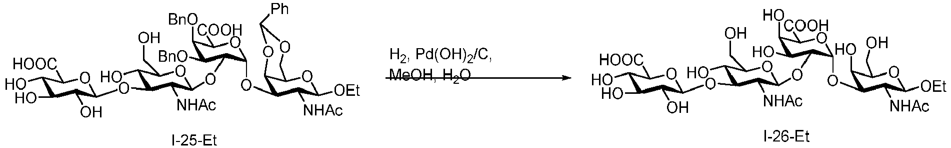

### Example 5

### Synthesis of isopropyl 2-deoxy-2-trifluoroacetamido-3,4,6-tri-O-acetyl-β-D-galactopyranoside

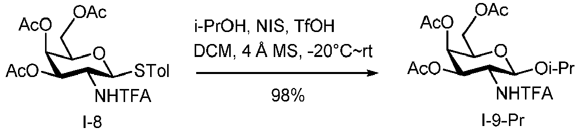

P-tolyl 2-deoxy-2-trifluoroacetamido-3,4,6-tri-O-acetyl-1-thio-β-D-galactopyranoside (2.0 g, mmol), N-iodosuccinimide (1.24 g, mmol, equiv.) and 4Å molecular sieves were dissolved in anhydrous dichloromethane (30 mL) under the protection of argon, stirried at room temperature for 2 hours, and then cooled to -20 °C. Trifluoromethanesulfonic acid (0.11 mL, mmol, equiv.) was added dropwise, and the reaction was carried out with continuously stirring for 3 hours, and moved to room temperature and the temperature was carried out overnight. After the reaction was completed as monitored by TLC, the reaction was quenched by adding proper amount of trimethylamine. The molecular sieve was filtered out by diatomite, and filter cake was washed several times with dichloromethane. Filtrates were combined, concentrated under reduced pressure, and purified by column chromatography (petroleum ether/EtOAc = 3: 1) to obtain a white solid (1.72 g, 98%). R*_{f}* = 0.49 (petroleum ether/EtOAc = 1: 1); ¹H NMR (400 MHz, CDCl₃, TMS) δ 6.60 (1H, d, *J* = 8.7 Hz, NH), 5.32 (1H, d, *J* = 2.1 Hz), 5.28 (1H, dd, *J* = 11.2, 3.4 Hz), 4.69 (1H, d, *J* = 8.3 Hz), 4.16-4.03 (2H, m), 4.02-3.93 (1H, m), 3.92-3.80 (2H, m), 2.09 (3H, s), 1.98 (3H, s), 1.93 (3H, s), 1.18 (d, *J* = 6.1 Hz, 3H), 1.06 (d, *J* = 6.1 Hz, 3H); ¹³C NMR (100 MHz, CDCl₃, TMS) δ 170.61, 170.53, 170.28 ,157.5,117.2, 99.19, 73.18, 70.70, 69.42, 66.58, 61.50, 52.40, 23.24, 21.72, 20.66, 20.47.

### Synthesis of isopropyl 2-deoxy-2-trifluoroacetamido-β-D-galactopyranoside

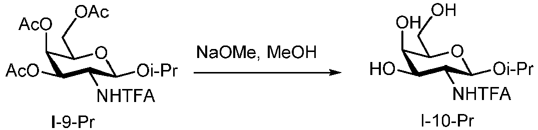

Isopropyl 2-deoxy-2-trifluoroacetamido-3,4,6-tri-O-acetyl-β-D-galactopyranoside (1.71 g, 3.86 mmol) was dissolved in methanol (50.0 mL), and pH was adjusted to 9-10 by adding proper amount of sodium methoxide, and the reaction was carried out with stirring at room temperature for 2 hours. After the reaction was completed as monitored by TLC, cationic resin was added to the reaction solution and the reaction was neutralized to pH = 7, filtered, and the filtrate was concentrated under reduced pressure to dryness to obtain a yellow syrup, R*_{f}* = 0.57 (DCM/MeOH = 5: 1), which was directly used for the next reaction.

### Synthesis of isopropyl-2-deoxy-2-trifluoroacetamido-4,6-O-benzylidene-β-D-galactopyranoside

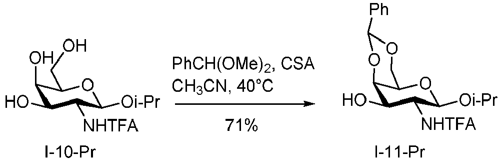

Isopropyl-2-deoxy-2-trifluoroacetamido-β-D-galactopyranoside (1.22 g, 3.86 mmol) and (+)-camphor sulfonic acid (1.60 g, 6.89 mmol, 1.8 equiv.) were dissolved in anhydrous acetonitrile (40.0 mL) under the protection of argon. Benzaldehyde dimethyl acetal (3.87 mL, 25.7 mmol, 6.6 equiv.) was added and heated to 40°C and the reaction was carried out overnight. After the reaction was completed as monitored by TLC, the reaction was quenched by adding proper amount of triethylamine dropwise. The reaction solution was concentrated under reduced pressure, and purified by column chromatography (petroleum ether/EtOAc = 2: 1) to obtain a white solid (1.12 g, 71%). R*_{f}* = 0.55 (petroleum ether/acetone = 1: 2).

### Isopropyl 2-O-p-methoxybenzyl-3,4-di-O-benzyl-6-acetylpropionyl-α-D-galactopyranosyl-(1→3)-2-deoxy-2-trifluoroacetamido-4,6-O-benzylidene-β-D-galactopyr anoside

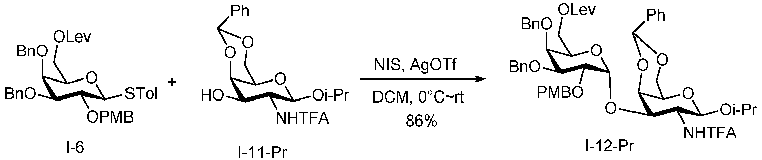

P-tolyl 2-O-p-methoxybenzyl-3,4-di-O-benzyl-6-acetylpropionyl-1-thio-β-D-galactopyranoside (227 mg, 0.33 mmol, 1.3 equiv.) and isopropyl 2-deoxy-2-trifluoroacetamido-4,6-O-benzylidene-β-D-galactopyranoside (103 mg, 0.25 mmol) were dissolved in anhydrous dichloromethane/N,N-dimethylformamide (5.0 mL/1. 0 mL) under the protection of argon, and 4Å molecular sieve (400 mg) was added, stirred for 2h at room temperature, and then cooled to 0 °C. N-iodosuccinimide ( 100 mg, 0.44 mmol, 1.7 equiv. )and silver trifluoromethanesulfonate (30 mg, 0.12 mmol, 0.5 equiv.) were added in turn, and the reaction was carried out with stirring at 0 °C for 2 hours, then the temperature was slowly raised to room temperature and the reaction was carried out overnight. After the reaction was monitored by TLC to be completed, the reaction was quenched by adding proper amount of triethylamine dropwise. The molecular sieve was removed by diatomite filtration, the filtrate was concentrated under reduced pressure, and purified by column chromatography (petroleum ether/EtOAc = 1: 1) to obtain compound I-12 (210 mg, 86%). R*_{f}* = 0.61 (DCM/MeOH = 20: 1); ¹H NMR (400 MHz, CDCl₃, TMS) δ 7.61-7.50 (3H, m), 7.41-7.22 (13H, m), 6.96 (2H, d, *J* = 8.5 Hz), 6.64 (2H, d, *J* = 8.6 Hz), 5.54 (1H, s), 5.21 (1H, d, *J* = 3.6 Hz), 5.00-4.92 (2H, m), 4.78 (1H, d, *J* = 11.7 Hz), 4.66 (1H, d, *J* = 11.8 Hz), 4.55 (1H, d, *J* = 11.4 Hz), 4.47 (2H, dd, *J* = 11.8, 2.3 Hz), 4.42 (1H, d, *J* = 3.4 Hz), 4.35-4.24 (2H, m), 4.19 (1H, dd, *J* = 11.6, 4.0 Hz), 4.13 - 4.03 (4H, m), 3.96 (1H, p, *J* = 6.3 Hz), 3.89 (1H, dd, *J* = 10.0, 2.7 Hz), 3.84 (1H, b), 3.79-3.71 (4H, m), 3.48 (1H, s), 2.91-2.80, 2.75-2.42 (4H, m), 2.19 (3H, s), 1.23 (3H, d, *J* = 6.1 Hz), 1.10 (3H, d, *J=* 6.0 Hz); ¹³C NMR (100 MHz, CDCl₃, TMS) δ 208.5, 172.5, 158.9, 157.3 (COCF₃), 138.6, 138.3, 137.7, 130.4, 129.5, 129.0, 128.4, 128.3 (2C), 128.2, 127.7 (2C), 127.5, 126.4, 115.9 (COCF₃), 113.5, 101.0, 98.2, 92.8, 78.3, 75.2, 74.7, 74.6, 73.4, 72.0, 71.5, 70.9, 70.7, 70.0, 69.5, 66.4, 64.1, 55.2, 53.3, 38.0, 29.8, 27.8, 23.4, 21.8; ESI-Q-TOF (positive mode) calculated for C₅₁H₅₈F₃NO₁₄⁺ [M+NH₄]⁺ m/z 983.3433, found 983.4160.

### Isopropyl 3,4-di-O-benzyl-6-acetylpropionyl-α-D-galactopyranosyl-(1→3)-2-deoxy-2-trifluoroacetamido-4,6-O-benzylidene-β-D-galactopyranoside

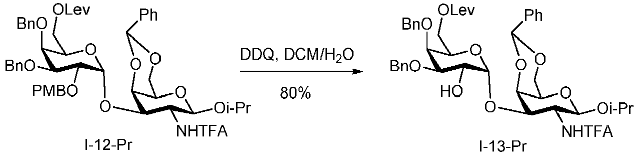

Isopropyl 2-O-p-methoxybenzyl-3,4-di-O-benzyl-6-acetylpropionyl-β-D-galacto pyranosyl-(1→3)-2-deoxy-2-trifluoroacetamido-4,6-O-benzylidene-β-D-galactopyranoside (714 mg, 0.86 mmol) was dissolved in dichloromethane/water (14.6 mL/1. 46 ml), and 2,3-dichloro-5,6-dicyanop-benzoquinone (204 mg, 0.90 mmol, 1.0 equiv) was added in batches, and the reaction was carried out for 1 hour at room temperature. After the reaction was completed as monitored by TLC, the reaction solution was diluted with dichloromethane, and the organic phase was washed with saturated sodium bicarbonate solution, saturated sodium thiosulfate solution and saturated salt water in turn, dried with anhydrous sodium sulfate, filtered, concentrated under reduced pressure, and purified by column chromatography (DCM/MeOH = 40: 1) to obtain a white solid (490 mg, 80%). R*_{f}* = 0.63 (petroleum ether/acetone = 1: 1); ¹H NMR (400 MHz, CDCl₃, TMS) δ 7.64 (1H, d, *J* = 9.3 Hz), 7.60-7.47 (2H, m), 7.42-7.10 (13H, m), 5.59 (1H, s), 5.18 (1H, d, *J* = 3.9 Hz), 4.94 (1H, d, *J* = 11.5 Hz), 4.86 (1H, d, *J* = 11.9 Hz), 4.70 (1H, d, *J* = 8.3 Hz), 4.62 (1H, d, *J* = 11.9 Hz), 4.51 (1H, d, *J* = 11.5 Hz), 4.42-4.24 (3H, m), 4.20-4.02 (6H, m), 3.95 (1H, p, *J* = 6.2 Hz), 3.78 (1H, d, *J* = 7.7 Hz), 3.72 (1H, s), 3.59 (1H, dd, *J* = 10.0, 2.8 Hz), 3.44 (1H, s), 2.92 (1H, ddd, *J* =18.9, 10.0, 3.5 Hz), 2.71-2.55 (2H, m), 2.48-2.40 (1H, m), 2.20 (3H, s), 1.23 (3H, d, *J* = 6.2 Hz), 1.11 (3H, d, *J* = 6.1 Hz); ¹³C NMR (100 MHz, CDCl₃, TMS) δ 210.0, 172.2, 157.3 (COCF₃), 138.5, 138.2, 137.3, 129.0, 128.4, 128.3 (2C), 127.8 (2C), 127.6, 126.2, 116.1 (COCF₃), 100.9, 98.9, 94.6, 79.6, 75.4, 74.6, 73.9, 73.1, 71.7, 70.6, 70.4, 69.4, 69.1, 66.3, 64.6, 51.6, 38.1, 29.9, 27.6, 23.4, 21.8; ESI-Q-TOF (positive mode) calculated for C₄₃H₅₀F₃NO₁₃⁺ [M+NH₄]⁺ m/z 863.3578, found 863.3588.

### Synthesis of isopropyl methyl 2,3,4-tri-O-acetyl-β-D-glucopyranuronate-(1 → 3)-4,6-di-O-acetyl-2-deoxy-2-trifluoroacetylamino-β-D-glucopyranosyl-(1 → 2)-3,4-di-O-benzyl-6-acetylpropionyl-α-D-galactopyranosyl-(1 → 3)-2-deoxy-2-trifluoroacetylamino-4,6-O-benzylidene-β-D-galactopyranoside

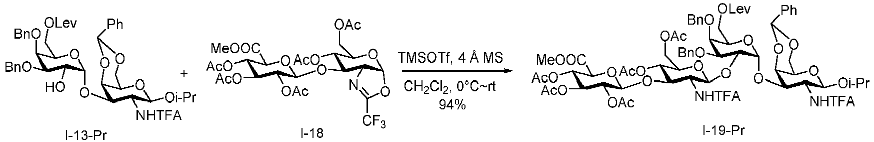

Methyl 2,3,4-tri-O-acetyl-β-D-glucopyranuronate-(1-3)-4,6-di-O-acetyl-2-deoxy-2 -trifluoroacetamido-D-glucopyranose [2, 1,-d] 2-oxazoline (488 mg, 0.74 mmol, 1.4 equiv.) and isopropyl 3,4-di-O-benzyl-6-acetylpropionyl-α-D-galactopyranosyl-(1→3)-2-deoxy-2-trifluoroacetylamino-4,6-O-benzylidene-β-D-galactopyranoside (438 mg, 0.52 mmol) were dissolved in anhydrous dichloromethane (7.0 mL). 4Å molecular sieve (700 mg) was added, stirred at room temperature for 2 hours, and then cooled to-20 °C, and trimethylsilyl trifluoromethylsulfonate (26.5 µL, 0.16 mmol, 0.3 equiv.) was added, and the reaction was carried out with stirring at -20 °C for 2h, and then the temperature was slowly raised to room temperature and the reaction was carried out overnight. After the reaction was completed as monitored by TLC, the reaction was quenched by adding proper amount of triethylamine dropwise. The molecular sieve was removed by diatomite filtration, the filtrate was concentrated under reduced pressure, and purified by column chromatography (petroleum ether/acetone 2: 1) to obtain a white solid (635 mg, 82%). R*_{f}* = 0.49 (petroleum ether/acetone 1: 1); ¹H NMR (400 MHz, CDCl₃, TMS) δ 7.76 (2H, t, *J* = 9.5 Hz), 7.66-7.58 (2H, m), 7.50 - 7.23 (11H, m), 7.19-7.14 (2H, m), 5.59 (1H, s), 5.16 (1H, t, *J* = 9.6 Hz), 5.13-5.02 (2H, m), 4.96 (2H, dd, *J* = 9.4, 8.1 Hz), 4.81 (1H, d, *J* = 11.6 Hz), 4.74-4.51 (4H, m), 4.42 (1H, d, *J* = 3.5 Hz), 4.40-4.24 (6H, m), 4.20-4.10 (2H, m), 4.07 (1H, dd, *J* = 10.2, 3.5 Hz), 4.03-3.78 (5H, m), 3.74 (1H, d, *J* = 2.9 Hz), 3.72 (3H, s), 3.65 (1H, d, *J* = 8.0 Hz), 3.59 (1H, td, *J* = 6.3, 2.9 Hz), 3.45 (2H, b), 3.07-2.92 (1H, m), 2.69-2.55 (3H, m), 2.35 (1H, dt, *J*=16.4, 4.2 Hz), 2.20 (3H, s), 2.10 (3H, s), 2.05 (3H, s), 2.02 (3H, s), 2.00 (6H, s), 1.24 (3H, d, *J* = 6.2 Hz), 1.11 (3H, d, *J* = 6.1 Hz). ¹³C NMR (100 MHz, CDCl₃, TMS) δ 210.2, 172.5, 170.9, 170.1, 169.9, 169.5 (2C), 167.1, 157.9 (COCF₃), 157.8 (COCF₃), 138.3, 138.1, 137.6, 130.3, 128.7, 128.5, 128.2, 128.1, 128.0, 127.9, 126.6, 117.0 (COCF₃), 115.7 (COCF₃), 101.6, 100.4, 99.1, 98.9, 94.2,77.8, 77.4, 76.3, 76.0, 74.9, 73.7, 72.9, 72.7, 72.6, 72.1, 71.8, 71.1, 70.4, 70.0, 69.5, 68.7, 66.5, 64.9, 62.5, 58.5, 52.8, 52.0, 38.1, 29.9, 27.8, 23.6, 21.9, 20.9, 20.7, 20.6; ESI-Q-TOF (positive mode) calculated for C₆₈H₈₀F₆N₂O₂₉⁺ [M+NH₄]⁺ m/z 1520.5095, found 1520.5051.

### Synthesis of isopropyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzy l-α-D-galactopyranosyl-(1→3)-2-deoxy-2-trifluoroacetylamino-4,6-O-benzylidene-β-D-gal actopyranoside

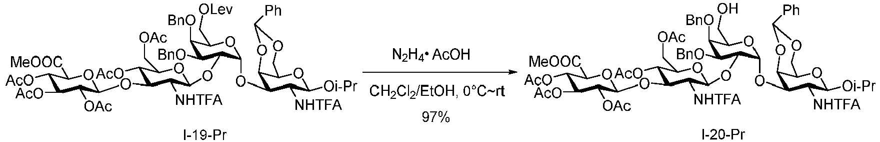

Isopropyl methyl 2,3,4-tri-O-acetyl-β-D-glucopyranuronate-(1→3)-4,6-di-O-acetyl-2 -deoxy-2-trifluoroacetylamino-β-D-glucopyranosyl-(1→2)-3,4-di-O-benzyl-6-acetylpropionyl-alpha-D-galactopyranosyl-(1→3)-2-deoxy-2-trifluoroacetylamino-4,6-O-benzylidene-β-D-gala ctopyranoside (490 mg, 0.33 mmol) was dissolved in dichloromethane/ethanol (17.0 mL/2.0 ml) , and hydrazine acetate (177 mg, 1.96 mmol, 6.0 equiv.) was added under ice bath conditions, and the temperature was slowly raised to room temperature and the reaction was carried out overnight. After the reaction was completed as monitored by TLC, the reaction solution was diluted with dichloromethane, and the organic phase was washed with 1M hydrochloric acid solution, saturated sodium bicarbonate solution and saturated salt water in turn, dried with anhydrous sodium sulfate, concentrated under reduced pressure, and purified by column chromatography (DCM/MeOH = 50: 1) to obtain a white solid (436 mg, 97%). R*_{f}* = 0.44 (DCM/MeOH = 20:1); ¹H NMR (400 MHz, CDCl₃, TMS) δ 7.56 (2H, dd, *J* = 7.3, 2.4 Hz), 7.42-7.35 (3H, m), 7.33-7.18 (12H, m), 7.07 (1H, d, *J* = 8.3 Hz), 5.61 (1H, s), 5.17-5.07 (3H, m), 4.92 (1H, t, *J* = 8.5 Hz), 4.86 (1H, d, *J* = 8.4 Hz), 4.82-4.72 (3H, m), 4.61 (1H, d, *J* = 12.2 Hz), 4.44-4.28 (6H, m), 4.22 (1H, d, *J* = 9.6 Hz), 4.16 (2H, d, J = 12.5 Hz), 4.08-3.99 (3H, m), 3.96 (1H, t, *J* = 6.2 Hz), 3.92 (1H, d, *J* = 9.6 Hz), 3.78 (1H, d, *J* = 2.8 Hz), 3.73 (3H, s), 3.69-3.58 (3H, m), 3.51 (1H, d, *J* = 2.8 Hz), 3.46 (1H, s), 3.45-3.38 (1H, m), 3.34 (1H, dd, *J* = 10.6, 3.7 Hz), 2.12 (3H, s), 2.05 (3H, s), 2.01 (6H, s), 2.00 (3H, s), 1.23 (3H, d, *J* = 6.2 Hz), 1.10 (3H, d, *J* = 6.1 Hz); ¹³C NMR (100 MHz, CDCl₃, TMS) δ 171.1, 170.0, 169.7, 169.4, 169.3, 166.9, 157.9 (COCF₃), 157.8 (COCF₃), 138.1, 137.8, 137.8, 128.6, 128.4 (2C), 128.1, 128.0 (2C), 127.7, 126.3, 117.0 (COCF₃), 115.7 (COCF₃), 101.0, 100.5, 100.2, 98.1, 75.6, 74.7, 72.9, 72.4, 72.2, 71.9, 71.0, 69.3, 68.2, 66.6, 62.4, 56.9, 52.8, 23.3, 21.7, 21.0, 20.6, 20.5, 20.4; ESI-Q-TOF (positive mode) calculated for C₆₃H₇₄F₆N₂O₂₇⁺ [M+NH₄]⁺ m/z 1422.4727, found 1422.4666.

### Synthesis of isopropyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzy l-α-D-galactopyranuronic acid-(1→3)-2-deoxy-2-trifluoroacetylamino-4,6-O-benzylidene-β-D-galactopyranoside

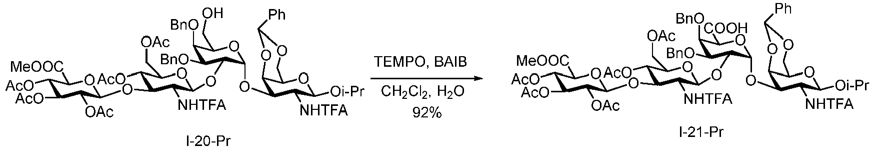

Isopropyl methyl 2,3,4-tri-O-acetyl-β-D-glucopyranuronate-(1→3)-4,6-di-O-acetyl-2-deoxy-2-trifluoroacetylamino-β-D-glucopyranosyl-(1→2)-3,4-di-O-benzyl-α-D-galactopyran osyl-(1→3)-2-deoxy-2-trifluoroacetylamino-4,6-O-benzylidene-β-D-galactopyranoside (413 mg, 0.29 mmol) was dissolved in dichloromethane/water (8.0 mL/4. 0 ml), and 2,2,6,6-tetramethylpiperidine-nitrogen-oxide (23 mg, 0.15 mmol, 0.5 equiv.) and diacetyloxyiodobenzene (189 mg, 0.59 mmol, 2.0 equiv.) were added under ice bath conditions in turn, and the temperature was naturally raised to room temperature and the reaction was carried out overnight. After the reaction was completed as monitored by TLC, the reaction solution was diluted with dichloromethane, the organic phase was washed with saturated sodium thiosulfate solution, dried with anhydrous sodium sulfate, concentrated under reduced pressure, and purified by column chromatography (DCM/MeOH = 50: 1) to obtain a light yellow solid (384 mg, 92%). R*_{f}* = 0.19 (DCM/MeOH = 30:1); ¹H NMR (400 MHz, CDCl₃, TMS) δ 7.62-7.47 (4H, m), 7.42-7.35 (3H, m), 7.31-7.16 (10H, m), 5.62 (1H, s), 5.20 (1H, s), 5.12 (2H, p, *J* = 9.3 Hz), 4.92 (1H, t, *J* = 8.2 Hz), 4.82 (1H, d, *J* = 8.3 Hz), 4.79-4.67 (3H, m), 4.63 (1H, d, *J* = 12.1 Hz), 4.44 (1H, d, *J* = 7.9 Hz), 4.41-4.21 (7H, m), 4.18-4.09 (2H, m), 4.06 (2H, d, *J* = 9.0 Hz), 4.01-3.90 (4H, m), 3.83 (1H, d, *J* = 10.3Hz), 3.71 (3H, s), 3.65-3.56 (1H, m), 3.33 (2H, b), 2.11 (3H, s), 2.07-1.94 (12H, m), 1.21 (3H, d, *J* = 6.1 Hz), 1.07 (3H, d, *J* = 6.0 Hz); ¹³C NMR (100 MHz, CDCl₃, TMS) δ 171.4, 170.4, 170.0, 169.9, 169.6, 169.4, 167.0, 157.9 (COCF₃), 157.5 (COCF₃), 137.9, 137.7, 137.4, 129.7, 128.6, 128.5, 128.3, 128.2, 127.8 (2C), 126.4, 117.1 (COCF₃), 114.3 (COCF₃), 101.1, 100.3, 98.1, 76.4, 75.8, 75.4, 73.1, 72.4 (2C), 72.0, 70.9, 69.4, 69.2, 68.3, 66.4, 62.4, 57.0, 53.1, 52.8, 23.3, 21.7, 20.8, 20.6, 20.5, 20.4(2C); ESI-Q-TOF (positive mode) calculated for C₆₃H₇₂F₆N₂O₂₈⁺ [M+NH₄]⁺ m/z 1436.4520, found 1436.4529.

### Synthesis of isopropyl β-D-glucopyranuronic acid-(1→3)-2-deoxy-2-acetylamino-β-D-glucopyranosyl-(1→2)-α-D-galactopyranoic acid-(1→3)-2-deoxy-2-acetylamino-α, β-D-galactopyranoside

CP-Pr was obtained from I-21-Pr in two steps by the same method as in Example 3.

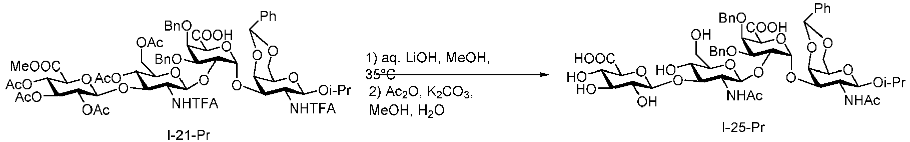

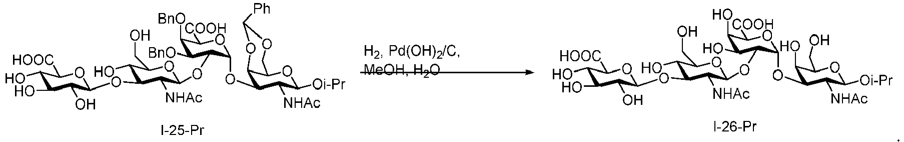

### Test Example 1

### In vitro anti-inflammatory activity test

### Effect of Compound CP-1/CP-2 on LPS induced NO production in RAW 264.7 cells

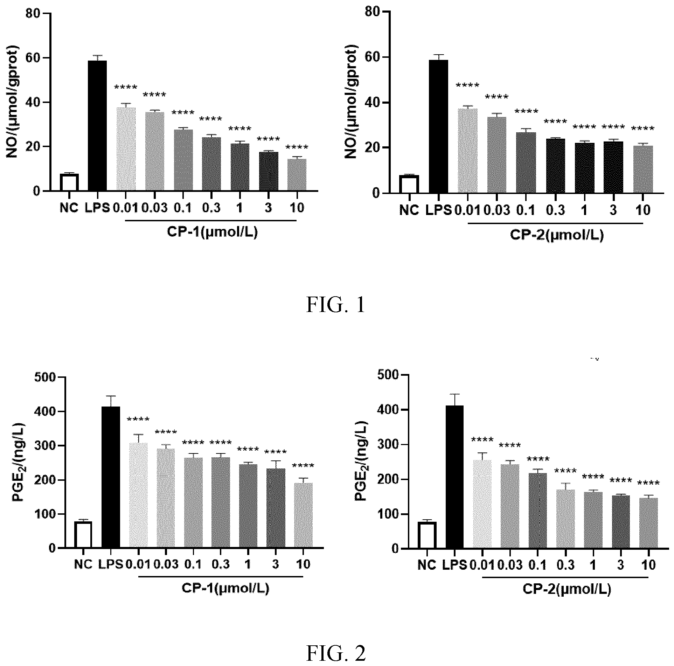

The cells were divided into control group, LPS group, Compound CP-1 and CP-2 groups with different concentrations (the concentrations of the compound being 0.01, 0.03, 0.1, 0.3, 1, 3, 10 µM). Each of the groups was provided with three multiple pores with 100 µL per well. The cells were inoculated into a 96-well cell culture plate at the density of 1 × 10⁵ cells per well and incubated overnight at 37 °C. The next day, the cells were treated by adding different concentrations of compounds for 2 hours, and then lipopolysaccharide (LPS) solution with a concentration of 100 ng/mL was added, and the culture was continued for 24 hours at 37 °C. After the end of culture, nitric oxide level in the cells was detected according to the instructions of the nitric oxide kit. As shown in FIG. 1, compared with the blank control group, the release of nitric oxide can be significantly promoted after being treated with 100 ng/mL of lipopolysaccharide for 24 hours. Compared with LPS model group, both CP-1 and CP-2 can inhibit the release of nitric oxide in a dose-dependent manner, and the difference is statistically significant.

### Effect of Compound CP-1/CP-2 on LPS induced PGE2 production in RAW 264.7 cells

The cells were divided into control group, LPS group, Compound CP-1 and CP-2 groups with different concentrations (the concentrations of the compound being 0.01, 0.03, 0.1, 0.3, 1, 3, 10 µM). Each of the groups was provided with three multiple pores with 100 µL per well. The cells were inoculated into 96-well cell culture plate at the density of 1 × 10⁵ cells per well and incubated overnight at 37 °C. The next day, the cells were treated by adding different concentrations of compounds for 2 hours, and then lipopolysaccharide (LPS) solution with a concentration of 100 ng/mL was added, and the culture was continued for 24 hours at 37 °C. After the end of culture, the cells were centrifuged at 4 °C at 300 g for 5 minutes, the cell supernatant was collected, and the level of prostaglandin E2 in the supernatant was detected according to the instructions of prostaglandin E2 kit. As shown in FIG. 2, compared with the blank control group, the release of prostaglandin E2 can be significantly promoted after being treated with 100 ng/mL of lipopolysaccharide for 24 hours. Compared with LPS model group, both CP-1 and CP-2 can inhibit the release of prostaglandin E2 in a dose-dependent manner, and the difference is statistically significant.

### Effects of Compound CP-1/CP-2 on LPS-induced release of IL-1 β, IL-6 and TNF-α in RAW 264.7 cells

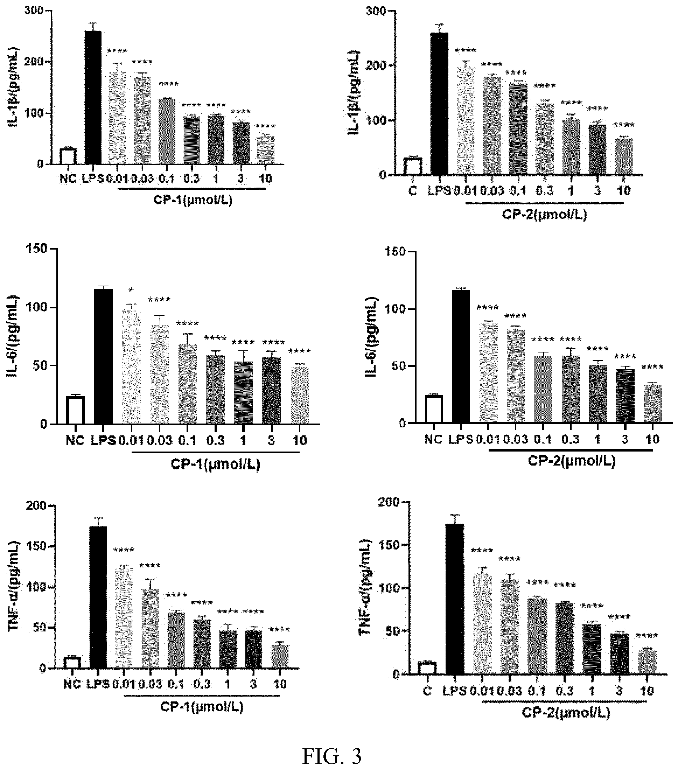

The cells were divided into control group, LPS group, Compound CP-1 and CP-2 groups with different concentrations (the concentrations of the compound being 0.01, 0.03, 0.1, 0.3, 1, 3, 10 µM). Each of groups was provided with three multiple pores with 100 µL per well. The cells were inoculated into 96-well cell culture plate at the density of 1 × 10⁵ cells per well and incubated overnight at 37°C. The next day, the cells were treated by adding different concentrations of compounds for 2 hours, and then lipopolysaccharide (LPS) solution with a concentration of 100 ng/mL was added, and the culture was continued for 24 hours at 37°C. After the end of culture, the cells were centrifuged at 4°C at 300 g for 5 minutes, the cell supernatant was collected, and the concentration of IL-1 β, IL-6, IL-10 and TNF-α in the supernatant was detected according to the instructions of the corresponding Elisa kit. As shown in FIG. 3, compared with the blank control group, the release of IL-1β, IL-6 and TNF-α can be significantly promoted after being treated with 100 ng/mL of lipopolysaccharide for 24 hours. Compared with LPS model group, both CP-1 and CP-2 can inhibit the release of IL-1β, IL-6 and TNF-α in a dose-dependent manner, and the difference is statistically significant.

### Test Example 2

### The effect of Compound CP-1/CP-2 on LPS induced expression of iNOS and COX-2 proteins in RAW 264.7 cells

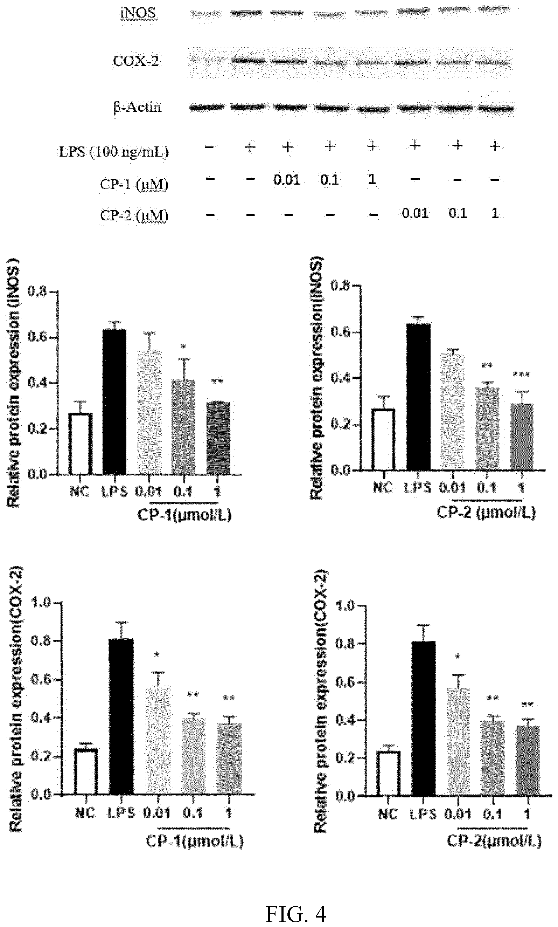

The cells were divided into control group, LPS group, Compound CP-1 and CP-2 groups with different concentrations (the concentrations of the compound being 1, 3, 10, 30, 100 µM). Each of the groups was provided with three multiple pores with 100 µL per well. The cells were inoculated into 96-well cell culture plate at the density of 4 × 10⁵ cells per well and incubated overnight at 37°C. The next day, the cells were treated by adding different concentrations of compounds for 2 hours, and then lipopolysaccharide (LPS) solution with a concentration of 100 ng/mL was added, and the culture was continued for 24 hours at 37°C. After the end of culture, the expression of iNOS and COX-2 proteins in cells was detected by western blot. The cell supernatant was discarded, and washed by adding the precooled PBS for 3 times, RIPA cell lysis solution containing PMSF was added, and placed on ice for 15 minutes to allow the cells to be fully lysed. The cells were centrifuged at 4°C at 300 g for 5 minutes, the cell supernatant was collected, and the concentration of protein was detected according to the instructions of the BCA protein quantitative kit, and then placed in boiled metal bath at 100°C for 5 minutes to denature the protein. After protein was separated by electrophoresis, the membrane was transfered and sealed at room temperature for 1 hour, and was incubated with the primary antibody at 4°C overnight. The membrane was washed with TBST three times the next day, and then placed at room temperature and was incubated with the secondary antibody at room temperature for 1 hour. After the TBST film was washed for three times, the chemiluminescence solution was added and placed it in the chemiluminescence gel imaging system for imaging. The results of western blot are shown in FIG. 4.

Compared with the blank control group, the expression of intracellular iNOS and COX-2 protein can be significantly promoted after treated with 100 ng/mL of lipopolysaccharide for 24 hours. Compared with LPS model group, both CP-1 and CP-2 can inhibit the expression of intracellular iNOS and COX-2 protein in a dose-dependent manner, and the difference is statistically significant.

### Test Example 3 In vivo anti-inflammatory activity test

### Effect of Compound CP-1 on LPS-induced survival rate in sepsis model of mice

A total of 60 male C57BL/6 mice aged 6-8 weeks, weighing 20 ± 2g, were selected. The mice were placed in a feeding room with temperature of 20-24°C and humidity of 50%-60%, and were allowed to drink and eat freely. They were adaptively raised for 7 days before the experiment.

The mice were randomly divided into six groups: control group, model group, CP-1 high dose group (30 mg/kg), CP-1 middle dose group (10 mg/kg), CP-1 low dose group (3 mg/kg), and dexamethasone group, with 10 mice in each group. CP-1 high-dose group was intraperitoneally injected with 200 µL CP-1 solution at the concentration of 30 mg/kg, CP-1 middle-dose group was intraperitoneally injected with equal volume of CP-1 solution at the concentration of 10 mg/kg, CP-1 low-dose group was intraperitoneally injected with equal volume of CP-1 solution at the concentration of 3 mg/kg, dexamethasone group was intraperitoneally injected with equal volume of dexamethasone solution at the concentration of 30 mg/kg, and control group was intraperitoneally injected with equal volume of 0.9% normal saline. Except for the control group, all groups were intraperitoneally injected with 200 µL of LPS solution at 45 mg/kg for modeling. Each group was administered once for a total of three times, 48 hours and 24 hours before modeling, and 30 minutes after modeling. After administration, the state and survival rate of mice were observed and recorded every 6 hours for 72 hours.

The experimental results are shown in the figure. After 72 hours of administration, the survival rate of mice in the model group is 40%, that in the control group of 100%, that in CP-1 high and low dose groups is 70%, that in CP-1 middle dose group is 80%, and that in the positive drug dexamethasone group is 60%.

### Effect of Compound CP-1 on LPS-induced cytokines in serum of sepsis mouse model

A total of 24 male C57BL/6 mice aged 6-8 weeks, weighing 20 ± 2g, were selected. The mice were placed in a feeding room with temperature of 20-24°C and humidity of 50%-60%, and were allowed to drink and eat freely. They were adaptively raised for 7 days before the experiment. Mice were randomly divided into four groups: control group, CP-1 group, LPS group and LPS + CP-1group, with 6 mice in each group. CP-1 group and LPS + CP-1 group were intraperitoneally injected with 200 µL CP-1 solution at the concentration of 10 mg/kg, while the control group and LPS group were intraperitoneally injected with equal volume of 0.9% normal saline. LPS group and LPS + CP-1 group were injected intraperitoneally with 200 µL LPS solution at 25 mg/kg for modeling. Each group was administered once for a total of two times, 24 hours before modeling and 30 minutes after modeling. After 12 hours of administration, the mice were anesthetized and blood was taken from the heart. After the blood stood for 2 hours, it was centrifuged at 5000 rpm for 10 minutes to collect serum. According to the instructions of Elisa kit, the absorbance of each hole was detected by using a multifunctional enzyme-labeled instrument at the wavelength of 450 nm, and the contents of cytokines IL-1β, IL-6, IL-18, TNF-α, Gal-3, INF-γ and HMGB1 were calculated according to the standard curve.

### Effect of Compound CP-Me on LPS-induced cytokines in serum of sepsis mouse model

A total of 24 male C57BL/6 mice aged 6-8 weeks, weighing 20 ± 2g, were selected. The mice were placed in a feeding room with temperature of 20-24°C and humidity of 50%-60%, and were allowed to drink and eat freely. They were adaptively raised for 7 days before the experiment. Mice were randomly divided into four groups: control group, CP-Me group, LPS group and LPS + CP-Me group, with 6 mice in each group. CP-Me 10 mg/kg group and CP-Me 3 mg/kg group were intraperitoneally injected with 200 µL CP-Me solution at the concentrations of 10 mg/kg and 3 mg/kg, while the control group and LPS group were intraperitoneally injected with equal volume of 0.9% normal saline. LPS group was injected intraperitoneally with 200 µL LPS solution at 25 mg/kg for modeling. Each group was administered once for a total of two times, 12 hours before modeling and 30 minutes after modeling. After 12 hours of administration, the mice were anesthetized and blood was taken from the heart. After the blood stood for 2 hours, it was centrifuged at 5000 rpm for 10 minutes to collect serum. According to the instructions of Elisa kit, the absorbance of each hole was detected by using a multifunctional enzyme-labeled instrument at the wavelength of 450 nm, and the contents of cytokines IL-1β, IL-6, and TNF-α were calculated according to the standard curve.

### HE staining and inflammatory index analysis

A total of 60 male C57BL/6 mice aged 6-8 weeks, weighing 20 ± 2g, were selected. The mice were placed in a feeding room with temperature of 20-24°C and humidity of 50%-60%, and were allowed to drink and eat freely. They were adaptively raised for 7 days before the experiment. Mice were randomly divided into six groups: control group, LPS group, CP-1 group (10 mg/kg), dexamethasone group, with 6 mice in each group. CP-1 high-dose group was intraperitoneally injected with 200 µL CP-1 solution at the concentration of 30 mg/kg, CP-1 middle-dose group was intraperitoneally injected with equal volume of CP-1 solution at the concentration of 10 mg/kg, CP-1 low-dose group was intraperitoneally injected with equal volume of CP-1 solution at the concentration of 3 mg/kg, dexamethasone group was intraperitoneally injected with equal volume of dexamethasone solution at the concentration of 30 mg/kg, and control group was intraperitoneally injected with equal volume of 0.9% normal saline. The models were made by intraperitoneal injection of 45 mg/kg LPS solution 200 µL except the control. 24 hours before modeling and 30 minutes after modeling, each was given medicine once, totaling two times. Except for the control group, all groups were intraperitoneally injected with 200 µL of LPS solution at 45 mg/kg for modeling. Each group was administered once for a total of two times, 24 hours before modeling and 30 minutes after modeling.

After 24 hours of administration, the mice were killed by removing the neck, and the left lung was soaked and fixed in formalin solution. After fixation, it was embedded in paraffin and sectioned, and the histomorphologically changes were observed by eosin staining. The scores were increased from 0 to 4 by different tissue changes, such as bleeding, neutrophil infiltration into alveoli, increased membrane transparency, increased tissue debris, and uneven thickening of the intermediate layer. 0 represents no damage, 1 represents less than 25% of the damage, 2 represents 25% to 50% of the damage, 3 represents 50% to 75% of the damage, and 4 represents more than 75% of the damage. The inflammation score is calculated by three different technicians, and the average value obtained by the three technicians is used as the final result.

The results showed that the LPS group had significant alveolar wall thickening, alveolar shrinkage, extensive interstitial inflammatory cell infiltration, and bleeding compared to the blank group; The CP-1 (10 mg/kg) group can significantly alleviate lung injury caused by LPS and maintain the basic morphology of lung tissue; The dexamethasone group can also achieve similar effects, but the morphology is not as good as CP-1, and there may be a higher risk of bleeding early on.

Although the embodiments disclosed in this application are as above, the contents described are only for the sake of understanding the embodiments adopted in this application, and are not intended to limit this application. Any person skilled in the art to which this application belongs may make any modifications and changes in the form and details of implementation without departing from the idea and scope disclosed in this application, but the scope of protection of this application shall still be subject to the scope defined in the appended claims.

## Claims

1. Use of a bacterial capsular oligosaccharide derivative or pharmaceutically acceptable salts, solvates and prodrugs thereof, the derivative is as shown in formula I: 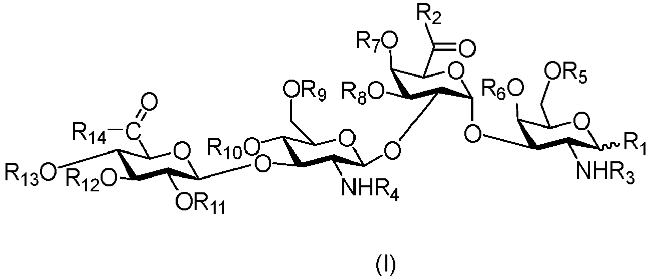
as an anti-inflammatory drug,
wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy;
R₂ is OH, -N(H)-R₁₅, N(R₁₆)-R₁₇, unsubstituted or substituted C1-C6 alkoxy, or unsubstituted or substituted aryloxy, where R₁₅ is an amino acid residue excluding proline; R₁₆ and R₁₇ together form a proline residue;
R₃ and R₄ are each independently unsubstituted or substituted C1-C6 alkanoyl;
R₅, R₆, R₇, R₈, R₉, R₁₀, R₁₁, R₁₂ and R₁₃ are each independently hydrogen, or unsubstituted or substituted C1-C6 alkanoyl, or unsubstituted or substituted C1-C6 alkyl;
R₁₄ is OH, unsubstituted or substituted C1-C6 alkoxy or unsubstituted or substituted aryloxy.

2. The use according to claim 1, wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy; wherein, the substituted C1-C6 alkoxy, substituted C2-C6 alkenyloxy, substituted C2-C6 alkynyloxy, substituted C1-C6 alkylthio, substituted C1-C6 alkynyloxy and substituted aryloxy mean that one or more hydrogen in C1-C6 alkoxy, C2-C6 alkenyloxy, C2-C6 alkynyloxy, C1-C6 alkylthio, C1-C6 alkanoyloxy or aryloxy are substituted by a group selected from hydroxy, C1-C6 alkoxy, halogen, nitro, cyano, acetyl, propionyl and phenyl;
R₂ is OH, -N(H)-R₁₅, N(R₁₆)-R₁₇, unsubstituted or substituted C1-C6 alkoxy, or unsubstituted or substituted aryloxy, wherein R₁₅ is an amino acid residue excluding proline; R₁₆ and R₁₇ together form a proline residue; the substituted C1-C6 alkoxy and substituted aryloxy mean that one or more hydrogen in C1-C6 alkoxy or the aryloxy is substituted by a group selected from hydroxy, C1-C6 alkoxy, halogen, nitro, cyano and phenyl;
R₃ and R₄ are each independently unsubstituted or substituted C1-C6 alkanoyl; wherein the substituted C1-C6 alkanoyl means that one or more hydrogen in the C1-C6 alkanoyl is substituted by a group selected from hydroxy, C1-C6 alkoxy, halogen, nitro, cyano, acetyl, propionyl and phenyl;
R₅, R₆, R₇, R₈, R₉, R₁₀, R₁₁, R₁₂ and R₁₃ are each independently hydrogen, unsubstituted or substituted C1-C6 alkanoyl, or unsubstituted or substituted C1-C6 alkyl; wherein the substituted C1-C6 alkanoyl or substituted C1-C6 alkyl means that one or more hydrogen in C1-C6 alkanoyl or C1-C6 alkyl is substituted by a group selected from hydroxy, C1-C6 alkoxy, halogen, nitro, cyano, acetyl, propionyl and phenyl; optionally, the phenyl may be substituted by one or more selected from the C1-C4 alkoxy and nitro;
R₁₄ is OH, unsubstituted or substituted C1-C6 alkoxy, or unsubstituted or substituted aryloxy; wherein the substituted C1-C6 alkoxy and substituted aryloxy mean that one or more hydrogen in C1-C6 alkoxy or aryloxy is substituted by a group selected from hydroxy, C1-C6 alkoxy, halogen, nitro, cyano and phenyl.

3. The use according to claim 2, wherein R₁ in formula (I) is OH, unsubstituted C1-C6 alkoxy, phenyl-substituted C1-C6 alkoxy, or unsubstituted C2-C6 alkenyloxy; preferably, R₁ is OH, methoxy, ethoxy, n-propoxy, isopropoxy, allyloxy, or benzyloxy.

4. The use according to claim 2, wherein R₂ in formula (I) is OH, -N(H)-R₁₅, N(R₁₆)-R₁₇, unsubstituted C1-C6 alkoxy, or phenyl-substituted C1-C6 alkoxy, wherein R15 is an amino acid residue excluding proline; R₁₆ and R₁₇ together form a proline residue; preferably, R₂ is OH, methoxy, or -N(H)-R₁₅, wherein R₁₅ is threonine residue.

5. The use according to claim 2, wherein R₃ and R₄ in formula (I) are each independently unsubstituted or substituted C1-C6 alkanoyl; wherein the substituted C1-C6 alkanoyl means that one or more hydrogen in the C1-C6 alkanoyl is substituted by a group selected from halogen, nitro, cyano, acetyl, propionyl and phenyl; preferably, R₃ and R₄ are each independently acetyl or trifluoroacetyl.

6. The use according to claim 2, wherein R₅, R₆, R₇, R₈, R₉, R₁₀, R₁₁, R₁₂ and R₁₃ in formula (I) are each independently hydrogen, unsubstituted C1-C6 alkanoyl, phenyl-substituted C1-C6 alkanoyl, or substituted phenylmethyl; preferably, R₅, R₆, R₇, R₈, R₉, R₁₀, R₁₁, R₁₂ and R₁₃ are each independently hydrogen, acetyl, benzyl, or 4-methoxybenzyl.

7. The use according to claim 2, wherein R₁₄ in formula (I) is OH, or unsubstituted or substituted C1-C6 alkoxy; preferably, R₁₄ is OH or methoxy.

8. The use according to any one of claims 1 to 7, wherein anti-inflammatory means inhibiting the production of nitric oxide and prostaglandin E2; and/or inhibiting the protein expression of nitric oxide synthase and cyclooxygenase-2; and/or reducing the release of interleukin-1β, interleukin-6 and tumor necrosis factor α; preferably, treating sepsis.

9. A method of preventing or treating inflammation, the method comprising administering to an individual in need thereof a therapeutically effective amount of a bacterial capsular oligosaccharide derivative or a pharmaceutically acceptable salt, solvate, prodrug thereof, the derivative is as shown in Formula I: 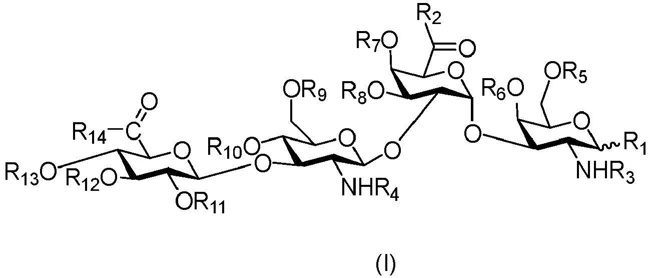
wherein R₁ in formula (I) is OH, unsubstituted or substituted C1-C6 alkoxy, unsubstituted or substituted C2-C6 alkenyloxy, unsubstituted or substituted C2-C6 alkynyloxy, unsubstituted or substituted C1-C6 alkylthio, unsubstituted or substituted C1-C6 alkanoyloxy, or unsubstituted or substituted aryloxy;
R₂ is OH, -N(H)-R₁₅, N(R₁₆)-R₁₇, unsubstituted or substituted C1-C6 alkoxy, or unsubstituted or substituted aryloxy, wherein R₁₅ is an amino acid residue excluding proline; R₁₆ and R₁₇ together form a proline residue;
R₃ and R₄ are each independently unsubstituted or substituted C1-C6 alkanoyl;
R₅, R₆, R₇, R₈, R₉, R₁₀, R₁₁, R₁₂ and R₁₃ are each independently hydrogen, or unsubstituted or substituted C1-C6 alkanoyl, or unsubstituted or substituted C1-C6 alkyl;
R₁₄ is OH, unsubstituted or substituted C1-C6 alkoxy or unsubstituted or substituted aryloxy.

10. The method of Claim 9, wherein preventing or treating inflammation means inhibiting the production of nitric oxide and prostaglandin E2; and/or inhibiting the protein expression of nitric oxide synthase and cyclooxygenase-2; and/or reducing the release of interleukin-1β, interleukin-6 and tumor necrosis factor α; preferably, treating sepsis.

11. The use according to any one of claims 1 to 8, or the method according to any one of claims 9 to 10, wherein the preparation method of the derivative comprises the following steps:
reacting the compound of formula (I-13) with the compound of formula (I-18) to obtain compound (I-19) 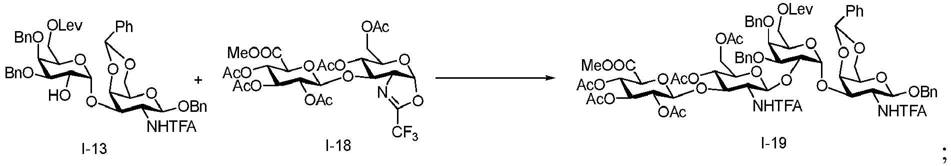
wherein in the compound of formula (I-13), formula (I-18) or formula (I-19), Ac is acetyl, Ph is phenyl, Bn is benzyl, Me is methyl, TFA is trifluoroacetyl, and Lev is acetylpropionyl.

12. A novel bacterial capsular oligosaccharide derivative as shown in formula (I'), or a pharmaceutically acceptable salt, solvate, prodrug thereof: 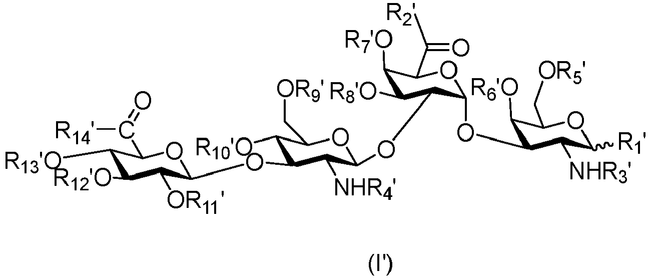
wherein R₁' in formula (I') is hydrogen, unsubstituted or substituted C1-C6 alkoxy, unsubstituted or substituted C2-C6 alkenyloxy, unsubstituted or substituted C2-C6 alkynoxy, unsubstituted or substituted C1-C6 alkylthio, unsubstituted or substituted C1-C6 alkanoyloxy, or unsubstituted or substituted aryloxy; wherein, the substituted C1-C6 alkoxy, substituted C2-C6 alkenyloxy, substituted C2-C6 alkynyloxy, substituted C1-C6 alkylthio, substituted C1-C6 alkynyloxy and substituted aryloxy mean that one or more hydrogen in C1-C6 alkoxy, C2-C6 alkenyloxy, C2-C6 alkynyloxy, C1-C6 alkylthio, C1-C6 alkanoyloxy or aryloxy are substituted by a group selected from hydroxy, C1-C6 alkoxy, halogen, nitro, cyano, acetyl, propionyl and phenyl;
R₂' is OH, -N(H)-R₁₅, N(R₁₆)-R₁₇, unsubstituted or substituted C1-C6 alkoxy, or unsubstituted or substituted aryloxy, wherein R₁₅ is an amino acid residue excluding proline; R₁₆ and R₁₇ together form a proline residue; the substituted C1-C6 alkoxy and substituted aryloxy mean that one or more hydrogen in C1-C6 alkoxy or the aryloxy is substituted by a group selected from hydroxy, C1-C6 alkoxy, halogen, nitro, cyano and phenyl;
R₃' and R₄' are each independently unsubstituted or substituted C1-C6 alkanoyl; wherein, the substituted C1-C6 alkanoyl means that one or more hydrogen in the C1-C6 alkanoyl is substituted with a group selected from hydroxy, C1-C6 alkoxy, halogen, nitro, cyano, acetyl, propionyl and phenyl;
R₅', R₆', R₇', R₈', R₉', R₁₀', R₁₁', R₁₂'and R₁₃' are each independently hydrogen, or substituted or unsubstituted C1-C6 alkanoyl; wherein the substituted C1-C6 alkanoyl means that one or more hydrogen in C1-C6 alkanoyl is substituted by a group selected from hydroxy, C1-C6 alkoxy, halogen, nitro, cyano, acetyl, propionyl and phenyl;
R₁₄' is OH, unsubstituted or substituted C1-C6 alkoxy, or unsubstituted or substituted aryloxy; wherein, the substituted C1-C6 alkoxy and substituted aryloxy mean that one or more hydrogen in C1-C6 alkoxy or aryloxy is substituted by a group selected from hydroxy, C1-C6 alkoxy, halogen, nitro, cyano and phenyl;
and it is defined that R₁' in the formula (I') is not n-propoxy or allyloxy.

13. The derivative according to claim 10, wherein R₁' in formula (I') is OH, methoxy, ethoxy, isopropoxy, n-butoxy, isobutoxy, tert-butoxy, or benzyloxy; preferably R₁' is OH, methoxy, ethoxy, or isopropoxy; and/or
R₂' is OH, -N(H)-R₁₅, N(R₁₆)-R₁₇, unsubstituted C1-C6 alkoxy, or phenyl-substituted C1-C6 alkoxy, wherein R15 is an amino acid residue excluding proline; R₁₆ and R₁₇ together form a proline residue; preferably, R₂' is OH, methoxy or -N(H)-R₁₅, wherein R₁₅ is a threonine residue; and/or
R₃' and R₄' are each independently unsubstituted or substituted C1-C6 alkanoyl; wherein the substituted C1-C6 alkanoyl means that one or more hydrogen in the C1-C6 alkanoyl is substituted by a group selected from halogen, nitro, cyano, acetyl, propionyl and phenyl; preferably, R₃' and R₄' are each independently acetyl or trifluoroacetyl; and/or
R₅', R₆', R₇', R₈', R₉', R₁₀', R₁₁', R₁₂' and R₁₃' in formula (I') are each independently hydrogen, unsubstituted C1-C6 alkanoyl, phenyl-substituted C1-C6 alkanoyl, or substituted phenylmethyl, preferably R₅', R₆', R₇', R₈', R₉', R₁₀', R₁₁', R₁₂' and R₁₃' are each independently hydrogen, acetyl, benzyl, or 4-methoxybenzyl; and/or
R₁₄' is OH, or unsubstituted or substituted C1-C6 alkoxy; preferably, R₁₄' is OH or methoxy.

14. The derivative according to claim 13, wherein formula (I') is Compound CP-1: 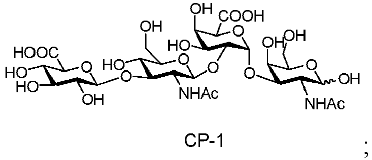 or
formula (I') is Compound CP-Me: 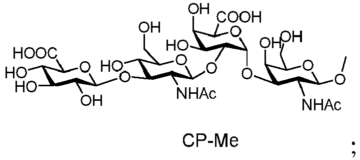 formula (I') is Compound CP-Et: 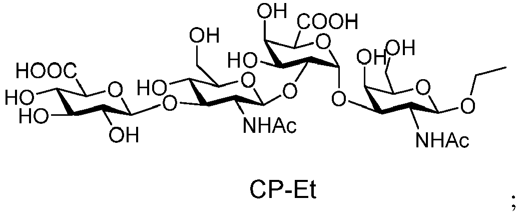 formula (I') is Compound CP-Pr:

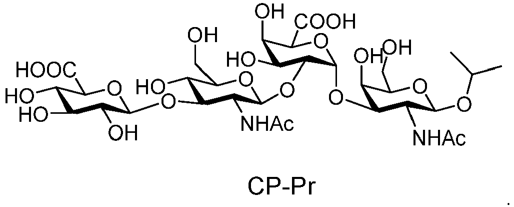

15. The derivative according to claim 13, wherein formula (I') is Compound CP-2:

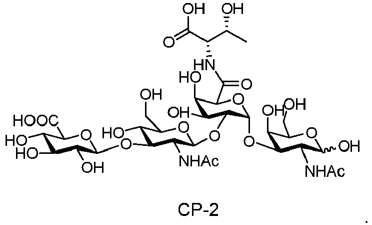

16. A pharmaceutical composition comprising the derivative of any one of claims 13 to 15.

17. The derivative of any one of claims 13 to 15, or the pharmaceutical composition of claim 14, for use as an anti-inflammatory drug.
